(19) Europäisches Patentamt
European Patent Office
Office européen des brevets

(11) EP 4 289 842 A1

(12) EUROPEAN PATENT APPLICATION
published in accordance with Art. 153(4) EPC

(43) Date of publication:
**13.12.2023 Bulletin 2023/50**

(21) Application number: **22749202.2**

(22) Date of filing: **30.01.2022**

(51) International Patent Classification (IPC):
***C07D 417/14*** (2006.01) ***C07D 413/14*** (2006.01)
***C07D 403/14*** (2006.01) ***C07D 487/04*** (2006.01)
***A61K 31/5377*** (2006.01) ***A61K 31/506*** (2006.01)
***A61K 31/4985*** (2006.01) ***A61K 31/4025*** (2006.01)
***A61P 31/20*** (2006.01) ***A61P 31/12*** (2006.01)
***A61P 1/16*** (2006.01)

(52) Cooperative Patent Classification (CPC):
**A61K 31/4025; A61K 31/4985; A61K 31/506; A61K 31/5377; A61P 1/16; A61P 31/12; A61P 31/20; C07D 403/14; C07D 413/14; C07D 417/14; C07D 487/04**

(86) International application number:
**PCT/CN2022/075185**

(87) International publication number:
**WO 2022/166923 (11.08.2022 Gazette 2022/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.02.2021 CN 202110164859**
**02.04.2021 CN 202110360500**
**08.05.2021 CN 202110500219**
**23.06.2021 CN 202110697314**

(71) Applicant: **Hepagene Therapeutics (HK) Limited**
**Wan Chai (HK)**

(72) Inventors:
- **WU, Dongdong**
  **Suzhou, Jiangsu 215000 (CN)**
- **HU, Yonghan**
  **Suzhou, Jiangsu 215000 (CN)**
- **LI, Xin**
  **Suzhou, Jiangsu 215000 (CN)**
- **WU, Yuchuan**
  **Beijing 100176 (CN)**
- **KONG, Desheng**
  **Suzhou, Jiangsu 215000 (CN)**

(74) Representative: **Symbiosis IP Limited**
**The Innovation Centre**
**217 Portobello**
**Sheffield, South Yorkshire S1 4DP (GB)**

# (54) PHENYLDIHYDROPYRIMIDINE COMPOUND AND USE THEREOF

(57) A phenyldihydropyrimidine compound as represented by formula I, a tautomer or a pharmaceutically acceptable salt thereof. The phenyldihydropyrimidine compound has a good anti-HBV activity and can be used for preparing drugs for treating and/or preventing HBV infections.

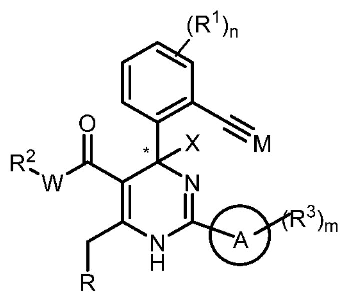

I

EP 4 289 842 A1

## Description

## CROSS REFERENCE TO RELATED APPLICATIONS

This application claims priority to Chinese Patent Application No. 202110164859.6 filed on February 5, 2021, Chinese Patent Application No. 202110360500.6 filed on April 2, 2021, Chinese Patent Application No. 202110500219.8 filed on May 8, 2021, and Chinese Patent Application No. 202110697314.1 filed on June 23, 2021, the entire contents of which are hereby incorporated herein by reference.

## FIELD OF THE INVENTION

The present disclosure relates to a phenyldihydropyrimidine compound and use thereof.

## BACKGROUND OF THE INVENTION

Chronic hepatitis B is a serious threat to human health and is the leading cause of cirrhosis and liver cancer. Chronic hepatitis B is caused when people are infected with the hepatitis B virus. According to the World Health Organization (WHO), there are an estimated 257 million people infected with hepatitis B virus worldwide (hepatitis B surface antigen positive for at least 6 months), and more than 780,000 people die each year from various diseases caused by hepatitis B. China is also a country with a high prevalence of hepatitis B. There are approximately 90 million people living with the hepatitis B virus, 28 million of whom are living with chronic hepatitis B.

Currently, the two main types of drugs used to treat chronic hepatitis B are nucleoside/nucleic acid analogues and interferons, but neither of these drugs can remove the virus from the body to achieve a cure for chronic hepatitis B. Patients need to take medication for a long time or even for life, and even with long-term medication, the chances of liver cancer remain relatively high.

## SUMMARY OF THE INVENTION

The technical problem to be solved by the present disclosure is the defect that the structure of drugs for the treatment and prevention of HBV infection in the prior art is too homogeneous. To this end, the present application provides a phenyldihydropyrimidine compound and use thereof, which has good anti-HBV (hepatitis B virus) activity and can be used for preparing medicines for treating and/or preventing HBV infection.

The present disclosure solves the above-mentioned technical problems through the following technical solutions.

The present disclosure provides a phenyldihydropyrimidine compound as shown in Formula I, a tautomer or a pharmaceutically acceptable salt thereof

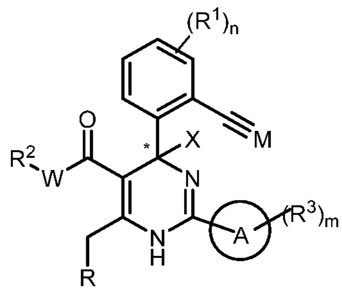

I

where M is CH or N;
X is H or D;
$R^1$ is independently halogen or $C_{1-4}$ alkyl;
n is 0, 1, 2, 3, or 4;
m is 0, 1, 2, 3, or 4;
$R^2$ is methyl or ethyl;
W is O or S;
ring A is a 5- to 6-membered heteroaryl; wherein the 5- to 6-membered heteroaryl comprises 1, 2 or 3 heteroatoms selected from N, O and S;

$R^3$ is independently H, halogen, $C_{1-4}$ alkyl, $C_{1-4}$ haloalkyl, $C_{3-6}$ cycloalkyl, or $-(CH_2)_{n3}-OH$;
n3 is 0, 1, 2, 3, or 4;
Ris

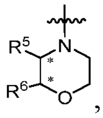, 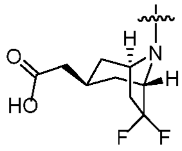, 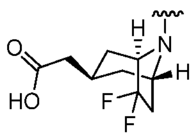, 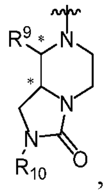, or 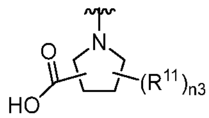;

One of $R^5$, $R^6$ is independently H, -COOH, $-(CH_2)_{n1}COOH$, $-CH=CH-(CH_2)_{n2}COOH$, or

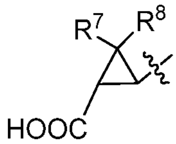,

the other is H or $C_{1-4}$ alkyl;

n1 is 1, 2, 3, or 4; n2 is 0, 1, or 2;
$R^7$, $R^8$ are independently H or halogen;
when $R^9$ is H, $R^{10}$ is phenyl-$C_{1-5}$ alkanediyl-COOH or -$C_{1-5}$ alkanediyl-COOH substituted by one or more $R^{12}$; when there are multiple substituents, they are the same or different;
or when $R^9$ is -COOH, $R^{10}$ is independently $C_{1-4}$ alkyl;
n3 is 0, 1, 2, 3, or 4;
$R^{11}$ is independently halogen or $C_{1-4}$ alkyl; or, when any two $R^{11}$ are joined together with the carbon to which they are attached to form $C_{3-6}$ cycloalkyl;
$R^{12}$ is independently H, halogen, or $C_{1-4}$ alkyl;
a carbon atom with "*" indicates that when it is a chiral carbon atom, it is in S configuration, R configuration or a mixture thereof.

In certain preferred embodiments of the present disclosure, certain groups in said phenyldihydropyrimidine as shown in Formula I, a tautomer or a pharmaceutically acceptable salt thereof are defined below, and the unmentioned groups are as described in any of the embodiments of the present application (hereinafter referred to as, "in a certain embodiment of the present disclosure").

In a certain embodiment of the present disclosure, a tautomer of the phenyldihydropyrimidine compound as shown in Formula I has the following structure:

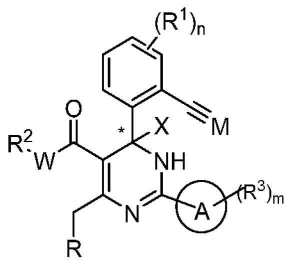

I .

In a certain embodiment of the present disclosure, the phenyldihydropyrimidine compound as shown in Formula I has the following structure:

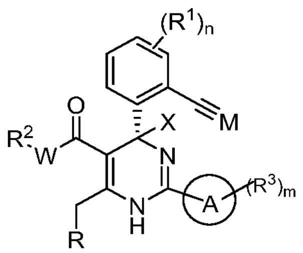

Ia , or

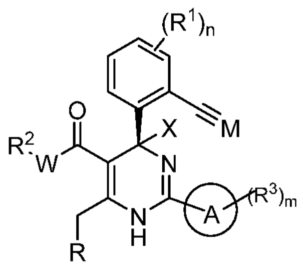

Ib .

In a certain embodiment of the present disclosure, $R^1$ is independently located at an ortho, meta or para position of the dihydropyrimidine ring.

In a certain embodiment of the present disclosure, when $R^1$ is halogen, said halogen is fluorine, chlorine, bromine, or iodine, such as fluorine or chlorine.

In a certain embodiment of the present disclosure, when $R^1$ is $C_{1-4}$ alkyl, said $C_{1-4}$ alkyl is methyl, ethyl, n-propyl, isopropyl, n-butyl, isobutyl, sec-butyl, or tert-butyl; for example methyl.

In a certain scheme of the present disclosure, when ring A is a 5- to 6-membered heteroaryl group, said 5- to 6-membered heteroaryl group is thienyl, thiazolyl, oxazolyl, imidazolyl, thiadiazolyl, or pyridyl; for example,

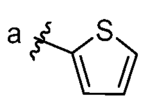
,
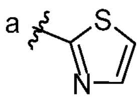
,
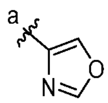
,

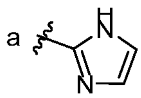
,
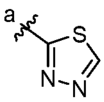
, or
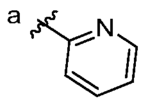
,

end a indicates the position of attachment to the pyrimidine ring; for example,

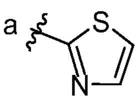
.

In a certain embodiment of the present disclosure, when $R^3$ is independently halogen or $C_{1-4}$ haloalkyl, said halogen and the halogen of said $C_{1-4}$ haloalkyl is fluorine, chlorine, bromine, or iodine, for example fluorine or chlorine.

In a certain embodiment of the present disclosure, when $R^3$ is independently $C_{1-4}$ alkyl or $C_{1-4}$ haloalkyl, said $C_{1-4}$ alkyl and the $C_{1-4}$ alkyl of said $C_{1-4}$ haloalkyl is methyl, ethyl, n-propyl, isopropyl, n-butyl, isobutyl, sec-butyl, or tert-butyl; e.g. methyl.

In a certain embodiment of the present disclosure, when $R^3$ is independently $C_{1-4}$ haloalkyl, the number of halo is 1, 2, 3; for example 3; for example trifluoromethyl.

In a certain embodiment of the present disclosure, when $R^3$ is independently $C_{3-6}$ cycloalkyl, said $C_{3-6}$ cycloalkyl is cyclopropyl, cyclobutyl, cyclopentyl, or cyclohexyl; for example cyclopropyl.

In a certain embodiment of the present disclosure, when one of $R^5$ and $R^6$ is independently $C_{1-4}$ alkyl, said $C_{1-4}$ alkyl is methyl, ethyl, n-propyl, isopropyl, n-butyl, isobutyl, sec-butyl, or tert-butyl; for example methyl.

In a certain embodiment of the present disclosure, when $R^7$ and $R^8$ are independently halogen, said halogen is fluorine, chlorine, bromine, or iodine, for example fluorine or chlorine.

In a certain embodiment of the present disclosure, when $R^{10}$ is phenyl-$C_{1-5}$ alkanediyl-COOH or -$C_{1-5}$ alkanediyl-COOH substituted by one or more $R^{12}$, the $C_{1-5}$ alkanediyl of said phenyl-$C_{1-5}$ alkanediyl-COOH and -$C_{1-5}$ alkanediyl-COOH substituted by one or more $R^{12}$ is methylene, $-CH_2CH_2-$, $-CH(CH_3)-$, $-CH_2CH_2CH_2-$, $-CH(CH_3)CH_2-$, $-CH_2CH(CH_3)-$, $-C(CH_3)_2-$, $-CH_2CH_2CH_2CH_2-$, $-CH(CH_3)CH_2CH_2-$, $-CH_2CH(CH_3)CH_2-$, $-CH_2CH_2CH(CH_3)-$, $-C(CH_3)_2CH_2-$, $-CH_2C(CH_3)_2-$, or $-CH_2C(CH_3)_2-CH_2-$; for example $-CH_2CH_2-$, $-CH_2C(CH_3)_2-$, or $-CH_2C(CH_3)_2-CH_2-$.

In a certain embodiment of the present disclosure, when $R^{10}$ is $C_{1-4}$ alkyl, said $C_{1-4}$ alkyl is methyl, ethyl, n-propyl, isopropyl, n-butyl, isobutyl, sec-butyl, or tert-butyl; for example, tert-butyl.

In a certain embodiment of the present disclosure, when $R^{11}$ is independently halogen, said halogen is fluorine, chlorine, bromine, or iodine, for example, fluorine or chlorine.

In a certain embodiment of the present disclosure, when $R^{11}$ is $C_{1-4}$ alkyl, said $C_{1-4}$ alkyl is methyl, ethyl, n-propyl, isopropyl, n-butyl, isobutyl, sec-butyl, or tert-butyl; for example, methyl.

In a certain embodiment of the present disclosure, when any two $R^{11}$ are joined together with the carbon to which they are attached to form $C_{3-6}$ cycloalkyl, said $C_{3-6}$ cycloalkyl is cyclopropyl, cyclobutyl, cyclopentyl, or cyclohexyl; for example, cyclopropyl .

In a certain embodiment of the present disclosure, when $R^{12}$ is independently halogen, said halogen is fluorine, chlorine, bromine, or iodine, for example, fluorine or chlorine.

In a certain embodiment of the present disclosure, when $R^{12}$ is $C_{1-4}$ alkyl, said $C_{1-4}$ alkyl is methyl, ethyl, n-propyl, isopropyl, n-butyl, isobutyl, sec-butyl, or tert-butyl; for example methyl.

In a certain embodiment of the present disclosure, M is CH.

In a certain embodiment of the present disclosure, $R^1$ is F or methyl.

In a certain embodiment of the present disclosure, n is 1 or 2.

In a certain embodiment of the present disclosure,

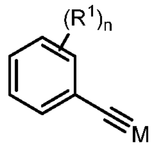 is 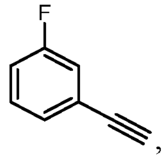, 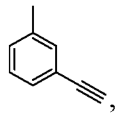,

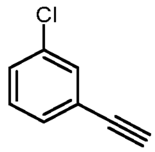, 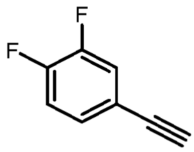, 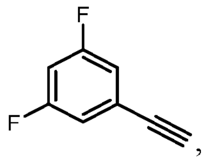, 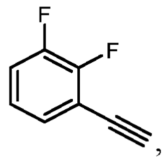, 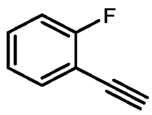, or 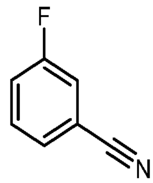.

In a certain embodiment of the present disclosure, W is O.

In a certain embodiment of the present disclosure, $R^2$-W- is ethyl-O-, methyl-O-, methylS-; for example ethyl-O-.

In a certain embodiment of the present disclosure, X is H.

In a certain embodiment of the present disclosure, m is 0, 1, or 2; for example, 0 or 1; such as 0.

In a certain embodiment of the present disclosure, $R^3$ is F, methyl, trifluoromethyl, cyclopropyl, or $-(CH_2)$-OH.

In a certain embodiment of the present disclosure,

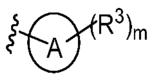

is

a 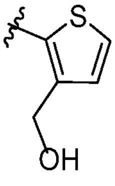, 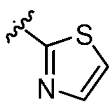,

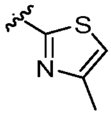, 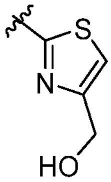, 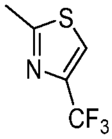, 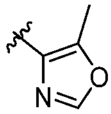, 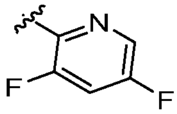;

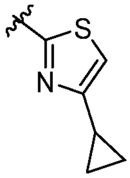 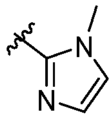 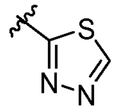

; for example

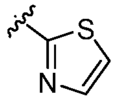.

In a certain embodiment of the present disclosure,

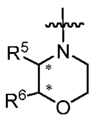 is 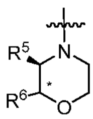 or 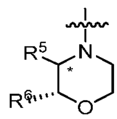; for example,

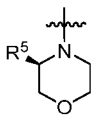 or 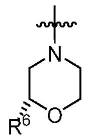.

In a certain embodiment of the present disclosure, n1 is 1 or 2.

In a certain embodiment of the present disclosure, n2 is 0.

In a certain embodiment of the present disclosure, $R^5$ is independently H or -COOH.

In a certain embodiment of the present disclosure, $R^6$ is independently H, methyl, - $(CH_2)_{n1}COOH$, --$CH=CH-(CH_2)_{n2}COOH$, or,

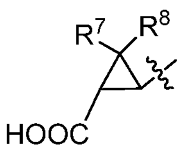,

for example, H or $-CH=CH-(CH_2)_{n2}COOH$.

In a certain embodiment of the present disclosure, $R^5$ is independently -COOH, $R^6$ is independently H or methyl; or $R^5$ is independently H $R^6$ is independently H $-(CH_2)_{n1}COOH$, - $CH=CH-(CH_2)_{n2}COOH$, or

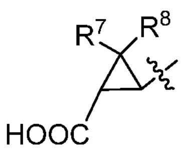,

For example, $R^5$ is independently -COOH, $R^6$ is independently H; or $R^5$ is independently H, $R^6$ is independently H or $-CH=CH-(CH_2)_{n2}COOH$.

In a certain embodiment of the present disclosure, $R^7$, $R^8$ are independently H or F. In a certain embodiment of the present disclosure,

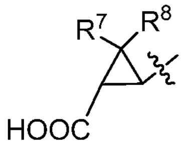 is 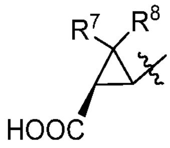

or

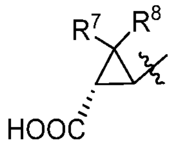.

In a certain embodiment of the present disclosure, $R^5$, $R^6$ are independently H, methyl, - COOH, $-(CH_2)_2COOH$, -CH=CH-COOH,

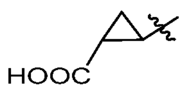, or 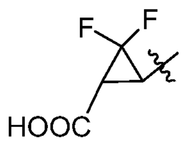.

In a certain embodiment of the present disclosure,

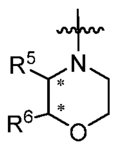 is 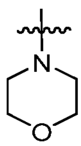, 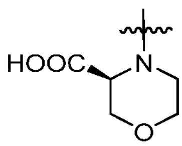,

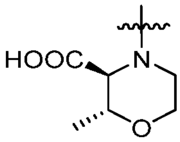, 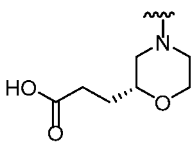, 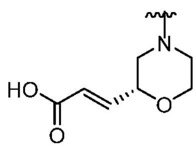, 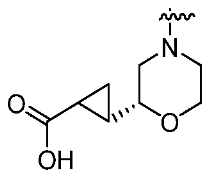, or 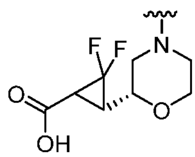;

For example,

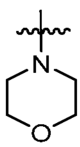, 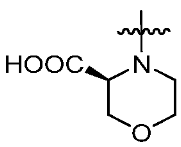, 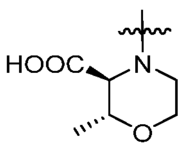, 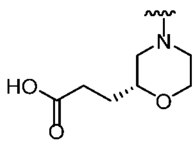, or 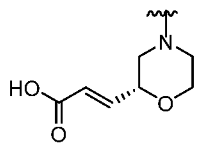.

In a certain embodiment of the present disclosure, when $R^{10}$ is phenyl-$C_{1-5}$ alkanediyl-COOH substituted by one or more $R^{12}$, it may be

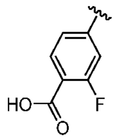 , or 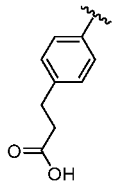 .

In a certain embodiment of the present disclosure, when $R^{10}$ is $-C_{1-5}$ alkanediyl-COOH, it may be $-CH_2C(CH_3)_2-COOH$ or $-CH_2C(CH_3)_2-CH_2-COOH$.

In a certain embodiment of the present disclosure,

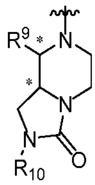 is 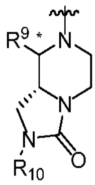 , 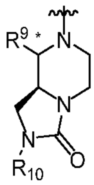 , 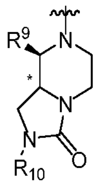 ,

or

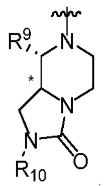

; for example

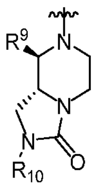 or 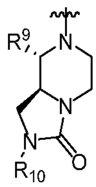 ;

for example

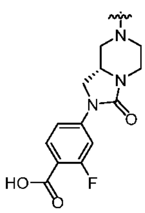 , 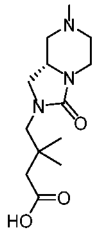 , 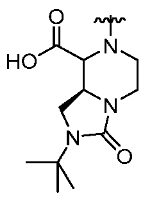 ,

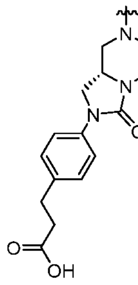 , or 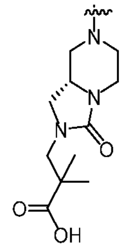 .

In a certain embodiment of the present disclosure, n3 is 0, 1, or 2; for example, 2.

In a certain embodiment of the present disclosure, $R^{11}$ is F; alternatively, two $R^{11}$ are joined together with the carbon they attached to form

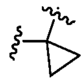

(spiro-linked cyclopropyl).

In a certain embodiment of the present disclosure,

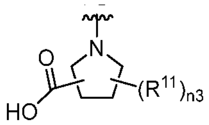 is 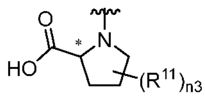 ;

for example

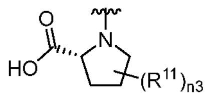 or 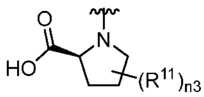

; or for example;

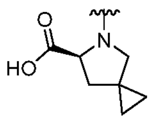 or 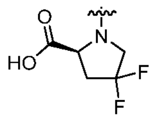 .

In a certain embodiment of the present disclosure, R is

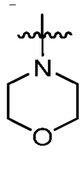 , 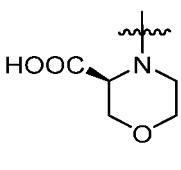 ,

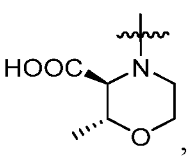 , 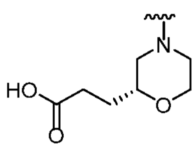 , 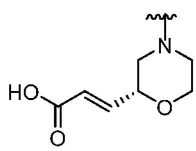 , 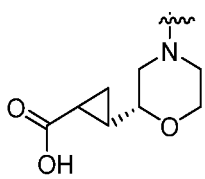 , 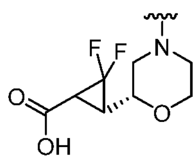 ,

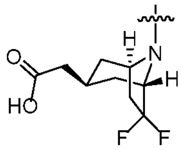, 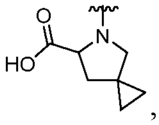, 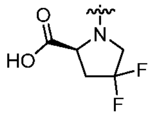, 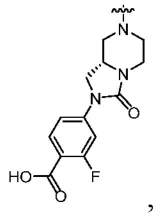, 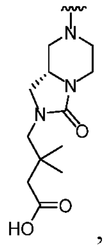, 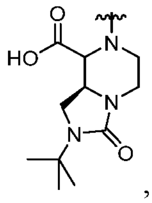, 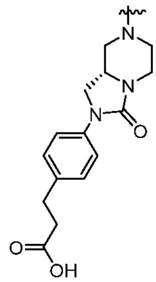,

or

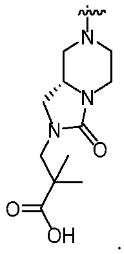.

In a certain embodiment of the present disclosure,

M is CH or N;
X is H or D;
$R^1$ is independently halogen;
n is 1;
$R^2$ is ethyl or methyl;
W is O or S;

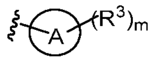 is 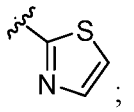;

Ris

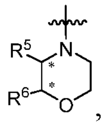, 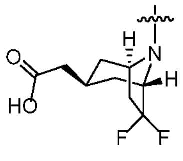, or 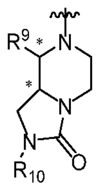;

$R^5$ is independently H, and $R^6$ is independently $-CH=CH-(CH_2)_{n2}COOH$;
$R^9$ is H, $R^{10}$ is $-C_{1-5}$ alkanediyl-COOH;
a carbon atom with "*" indicates that when it is a chiral carbon atom, it is in S configuration, R configuration or a mixture thereof.
In a certain embodiment of the present disclosure, M is CH or N;
XisH;
$R^1$ is independently F;
n is 1;
$R^2$ is ethyl;
WisO;

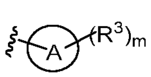 is 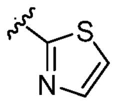;

Ris

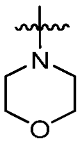, 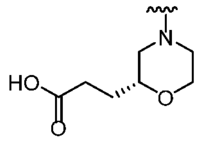, 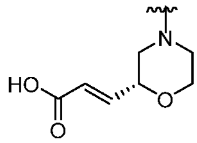, or 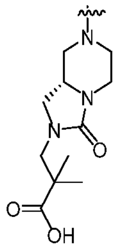;

a carbon atom with "*" indicates that when it is a chiral carbon atom, it is in S configuration, R configuration or a mixture thereof.

In a certain embodiment of the present disclosure, the phenyldihydropyrimidine compound as shown in Formula I is any of the following structures:

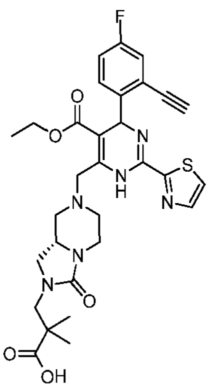

SZ-023089RAC

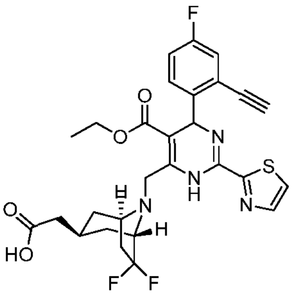

SZ-023091RAC

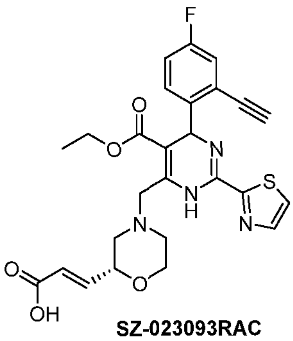

SZ-023093RAC

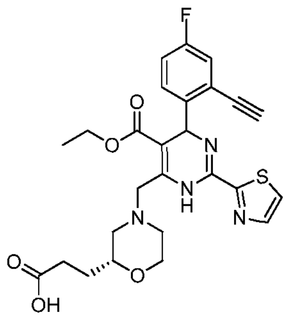

SZ-023095RAC

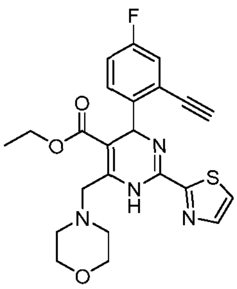

SZ-023115RAC

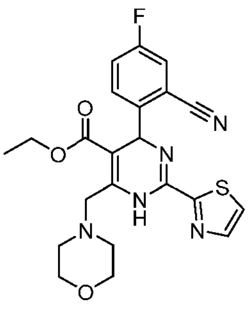

SZ-023117RAC

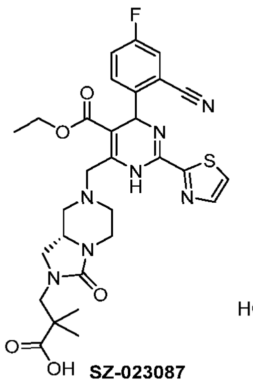

SZ-023087

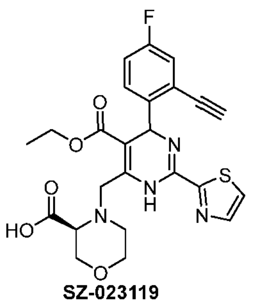

SZ-023119

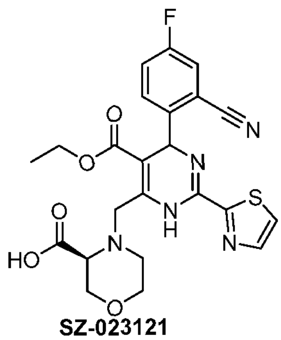

**SZ-023121**

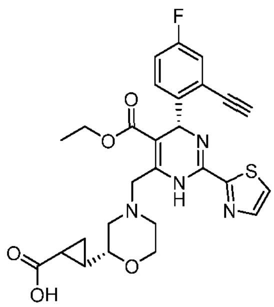

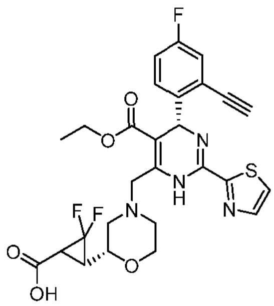

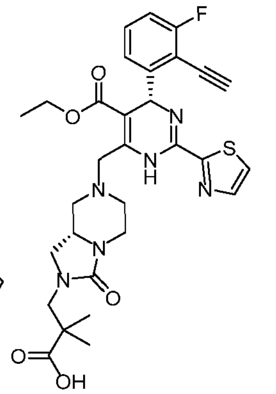

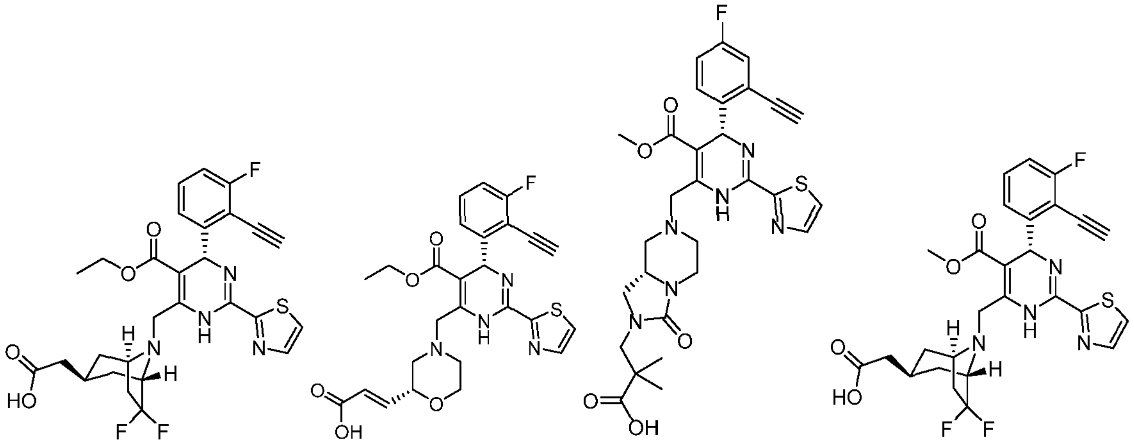

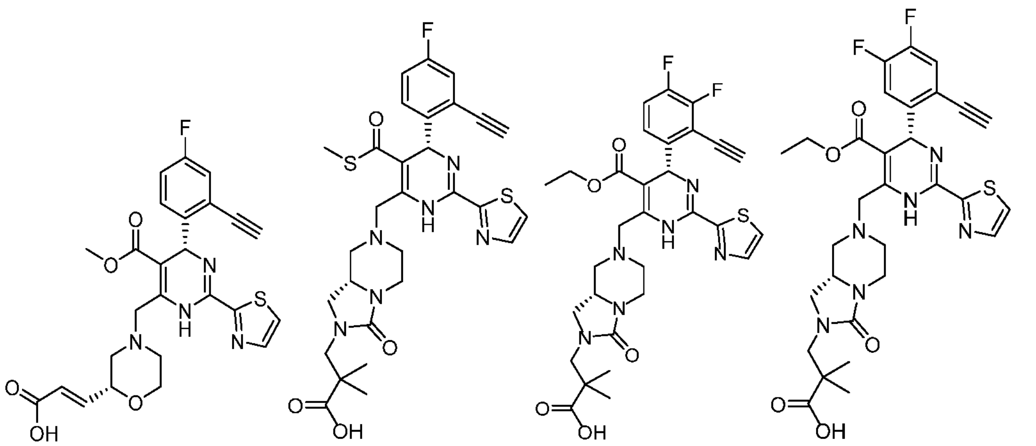

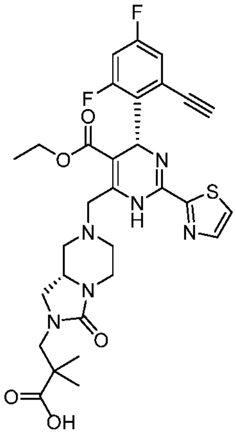 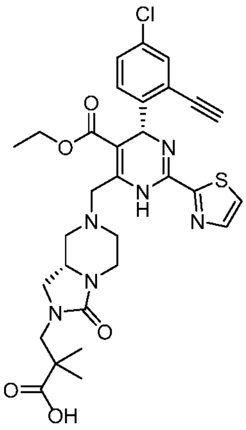 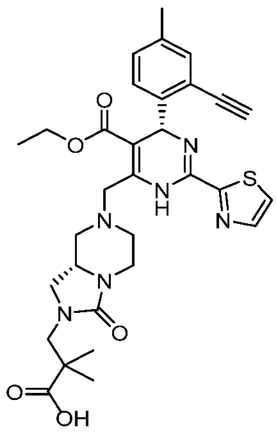 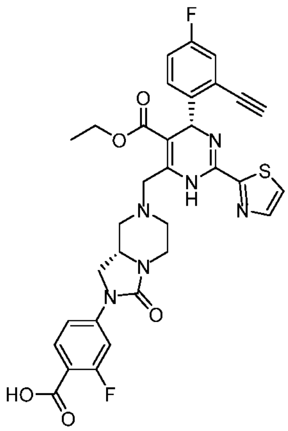

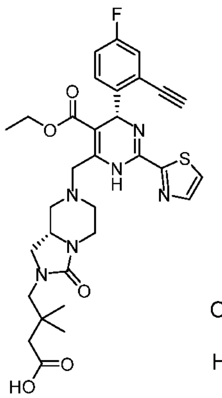 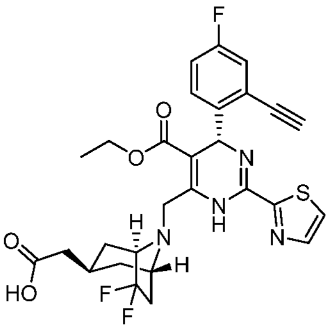 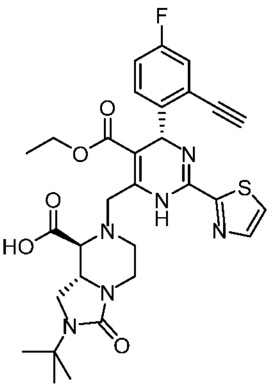 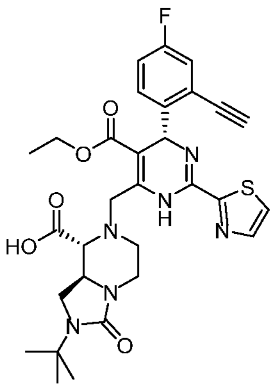

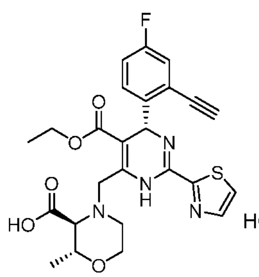 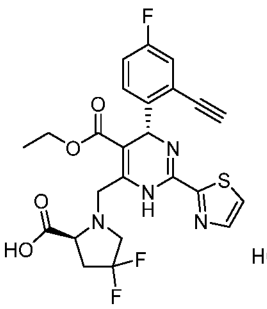 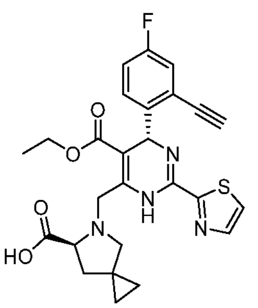 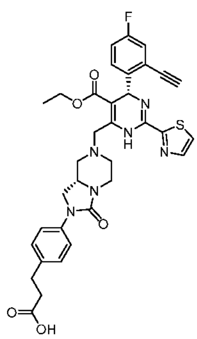 .

In a certain embodiment of the present disclosure, the phenyldihydropyrimidine compound as shown in Formula I is any of the following structures:

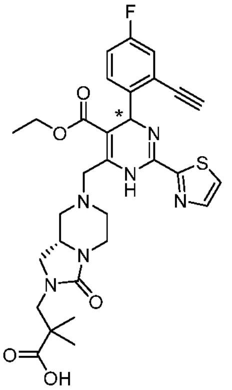 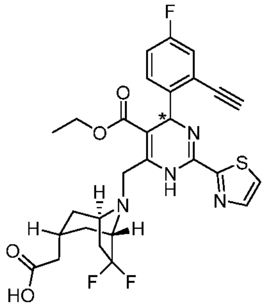 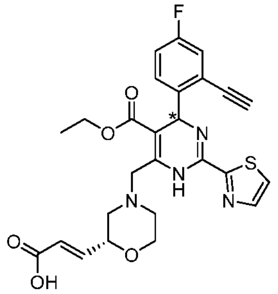 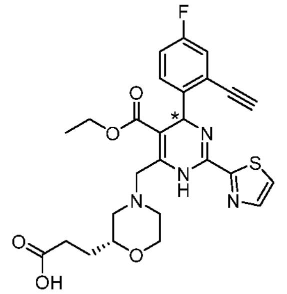

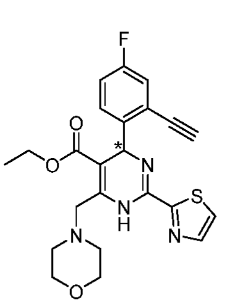
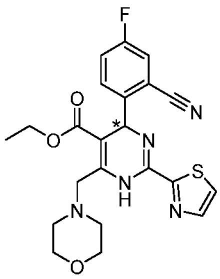
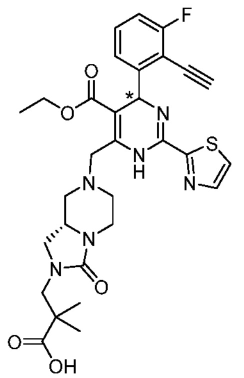
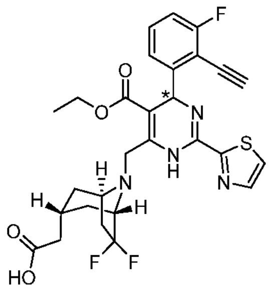
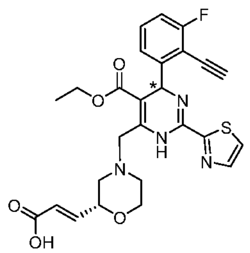
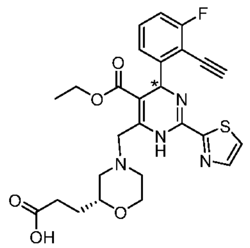
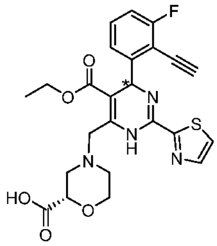
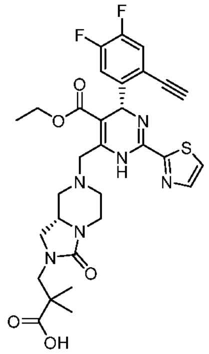
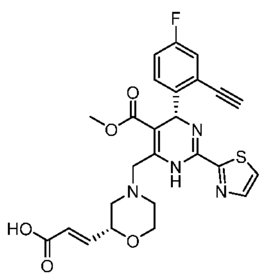
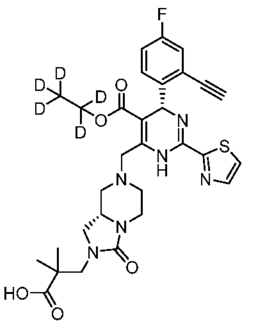
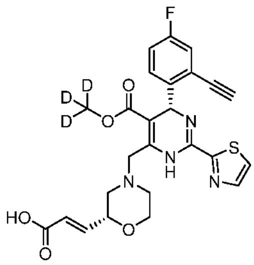
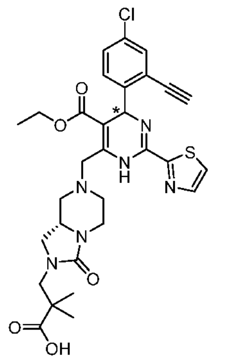
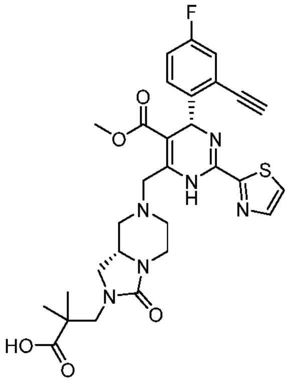
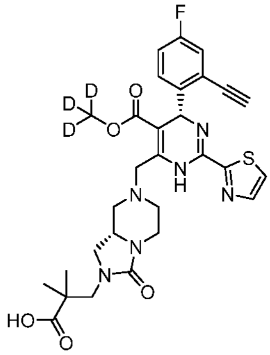
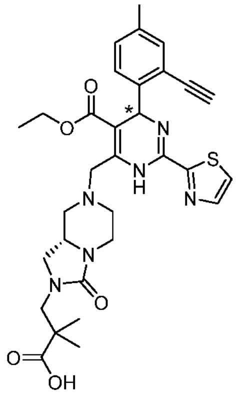
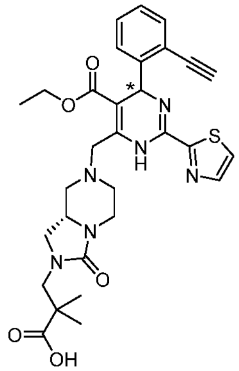

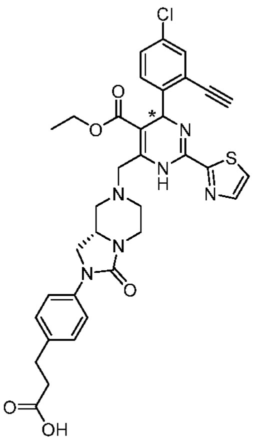
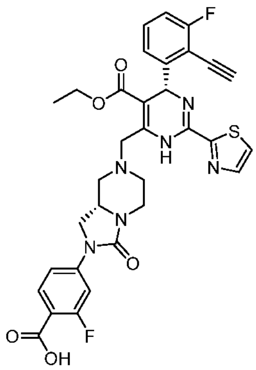
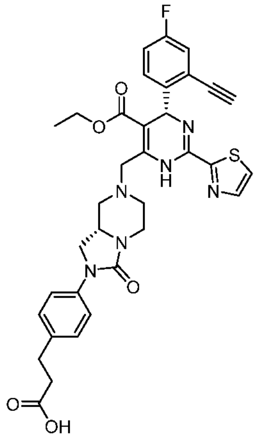
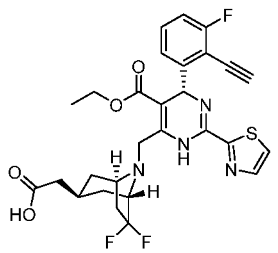
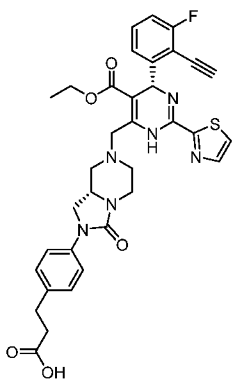
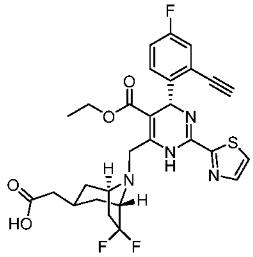
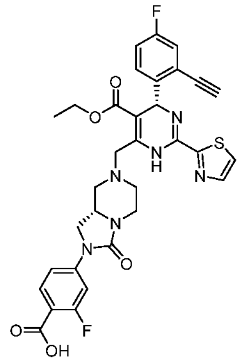
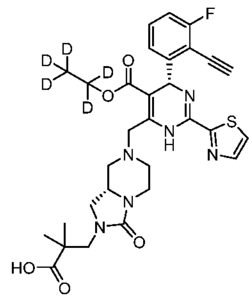
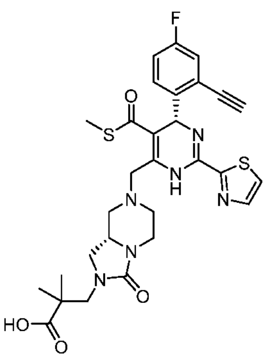
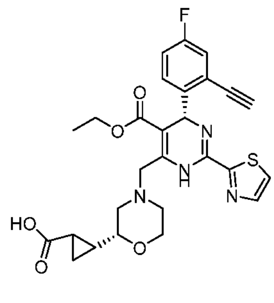
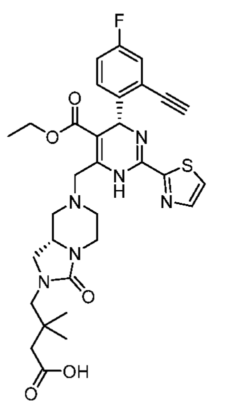
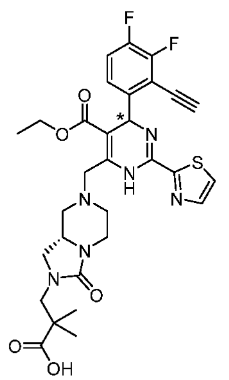
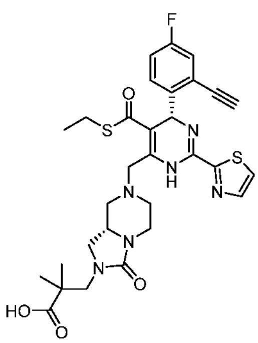

a carbon atom with "*" indicates that when it is a chiral carbon atom, it is in S configuration, R configuration or a mixture thereof.

Accordingly, throughout the present specification, those skilled in the art may select the groups and their substituents in said phenyldihydropyrimidine compound as shown in Formula I, a tautomer or a pharmaceutically acceptable salt thereof to provide a stable phenyldihydropyrimidine compound as shown in Formula I, a tautomer or a pharmaceutically acceptable salt thereof, including but not limited to the compounds described in embodiments of the present disclosure.

The phenyldihydropyrimidine compound as shown in Formula I, a tautomer or a pharmaceutically acceptable salt thereof as described herein may be synthesized by methods comprising steps and conditions similar to those of methods well known in the chemical field, with reference to the steps and conditions of similar reactions in the field, in particular according to the description herein. Starting materials are usually from commercial sources such as Aldrich or can be readily prepared using methods known to those skilled in the art (available through SciFinder, Reaxys online database).

In the present disclosure, the phenyldihydropyrimidine compound as shown in Formula I can also be obtained by peripheral modification of the phenyldihydropyrimidine compound as shown in Formula I, which has already been prepared, to obtain other heterocyclic compounds as shown in Formula I, using conventional methods in the art.

Generally, the compounds of the present disclosure can be prepared by the methods described herein, unless further specified, wherein the substituents are defined as shown in Formula I. The following reaction schemes and examples are used to further exemplify the present disclosure.

The necessary raw materials or reagents for the preparation of compounds such as those in Formula I are commercially available or can be prepared by synthetic methods known in the art. The compounds of the present disclosure can be prepared as free bases or as salts of acids as described in the experimental section below. The term pharmaceutically acceptable salt refers to a salt that is pharmaceutically acceptable as defined herein and has all the pharmacological activity of the parent compound. Pharmaceutically acceptable salts can be prepared by adding the corresponding acid to a suitable organic solvent of an organic base and treating it according to conventional methods.

Examples of salt formation include: salts formed with inorganic acids such as hydrochloric acid, hydrobromic acid, sulphuric acid, nitric acid, phosphoric acid; and salts formed with organic acids such as acetic acid, benzenesulphonic acid, benzoic acid, camphorsulphonic acid, citric acid, ethanesulphonic acid, fumaric acid, glucoheptulonic acid, glutamic acid, ethanoic acid, hydroxynaphthoic acid, 2-hydroxyethanesulphonic acid, lactic acid, maleic acid, malic acid, malonic acid, mandelic acid, methanesulphonic acid, 2-naphthalenesulphonic acid, propionic acid, salicylic acid, succinic acid, tartaric acid, p-toluenesulphonic acid or trimethylacetic acid.

The phenyldihydropyrimidine compound as shown in Formula I may have one or more chiral carbon atoms and can therefore be isolated to give optically pure isomers, such as pure enantiomers, or racemates, or mixed isomers. Pure single isomers can be obtained by separation methods in the art, such as chiral crystallization into salts, or chiral preparative column separations.

Chemicals used in the synthetic route described in the present patent, including solvents, reagents, catalysts and protecting groups, deprotective groups, protecting groups including tert-butoxycarbonyl (Boc). The above methods may additionally include steps before or after the steps specifically described herein, where suitable protecting groups may be added or removed to obtain the target compound. Alternatively, the various synthesis steps can be performed alternately or in sequence to obtain the final target product.

The present disclosure provides a pharmaceutical composition comprising a phenyldihydropyrimidine compound as shown in Formula I as described above, a tautomer or a pharmaceutically acceptable salt thereof, and, (one or more) pharmaceutical adjuvants (e.g. excipients or carriers). By way of example, such pharmaceutical composition may comprise one or more additional phenyldihydropyrimidine compounds as shown in Formula I, tautomers or pharmaceutically acceptable salts thereof. In said pharmaceutical composition, an amount of said phenyldihydropyrimidine compounds as shown in Formula I, a tautomer or a pharmaceutically acceptable salt thereof may be in therapeutically effective amounts.

The present disclosure also provides use of the phenyldihydropyrimidine compound as shown in Formula I as described above, a tautomer or a pharmaceutically acceptable salt thereof in preparation of HBV inhibitors. In the use, said HBV inhibitor can be used *in vivo;* it can also be used *in vitro,* mainly as an experimental use, for example: as a standard or control sample to provide a comparison, or in a kit according to the conventional methods in the field to provide a rapid test for the inhibitory effect of HBV

The present disclosure also provides use of the phenyldihydropyrimidine compound as shown in Formula I as described above, a tautomer or a pharmaceutically acceptable salt thereof in preparation of a medicament; the medicament is a medicament for prevention and/or treatment of HBV infection.

Another aspect of the present disclosure relates to a method of preventing and/or treating HBV infection comprising administering to a patient a therapeutically effective amount of the phenyldihydropyrimidine compound as shown in Formula I, a tautomer or a pharmaceutically acceptable salt thereof, or a pharmaceutical composition comprising thereof.

Another aspect of the present disclosure relates to a pharmaceutical product for the treatment and/or prevention

of HBV infection comprising a phenyldihydropyrimidine compound as described in formula I, a tautomer or a pharmaceutically acceptable salt thereof, or a pharmaceutical composition comprising thereof.

The compounds, pharmaceutical compositions of the present disclosure can be administered topically or systemically, for example, for enteral administration, such as rectal or oral administration, or for parenteral administration to mammals (meaning in particular humans). Exemplary combinations for rectal administration include suppositories, which may comprise, for example, suitable non-irritating excipients such as cocoa butter, synthetic glycerides or polyethylene glycols, which are solid at room temperature but melt and/or dissolve in the rectal lumen to release the drug. The compounds of the present disclosure may also be administered parenterally, for example, by inhalation, injection or infusion, such as by intravenous, intra-arterial, intraosseous, intramuscular, intracerebral, extraventricular, intra-synovial, intrastromal, intrathecal, intra-lesional, intracranial, intra-tumour, intradermal and subcutaneous injection or input.

The compounds, pharmaceutical compositions described herein can be used for topical or systemic administration, for example for enteral administration, such as rectal or oral administration, or for parenteral administration to mammals (meaning in particular humans), and comprise a therapeutically effective amount of the compound according to The present disclosure, a tautomer or a pharmaceutically acceptable salt thereof as the active ingredient, together with a pharmaceutically acceptable excipient, such as a pharmaceutically acceptable carrier. The therapeutically effective amounts of the active ingredients are as defined in the context and depend on the species, body weight, age, individual condition, individual pharmacokinetic parameters of the mammal, disease to be treated and mode of administration, for example for enteral administration, such as oral administration, the compounds of the present disclosure can be formulated in a wide range of dosage forms.

The effective amounts of the compounds, pharmaceutical compositions or drugs described in the present disclosure can be readily determined by conventional experiments, as can the most effective and convenient routes of administration and the most appropriate formulations.

Said pharmaceutical excipients may be those widely used in the field of pharmaceutical production. Excipients are primarily used to provide a safe, stable and functional pharmaceutical composition and may also provide methods to enable the active ingredient to dissolve at a desired rate after the subject has received administration or to facilitate effective absorption of the active ingredient after the subject has received administration of the composition. Said pharmaceutical excipients may be inert fillers or provide some function such as stabilizing the overall pH of the composition or preventing degradation of the active ingredients of the composition. Said pharmaceutical excipients may include one or more of the following excipients: binders, suspension aids, emulsifiers, diluents, fillers, granulation agents, gelling agents, disintegrants, lubricants, anti-adhesive agents, flow aids, wetting agents, gelling agents, absorption retardants, dissolution inhibitors, enhancers, adsorbents, buffers, chelating agents, preservatives, coloring agents, flavoring agents and sweeteners.

Substances that may be used as pharmaceutically acceptable excipients include, but are not limited to, ion exchangers, aluminum, aluminum stearate, lecithin, serum proteins such as human serum proteins, buffering substances such as phosphates, glycine, sorbic acid, potassium sorbate, mixtures of partial glycerides of saturated vegetable fatty acids, water, salts or electrolytes such as ichthyosperm sulfate, disodium hydrogen phosphate, potassium hydrogen phosphate, sodium chloride, zinc salts, colloidal silica, magnesium trisilicate, polyvinylpyrrolidone, polyacrylates, waxes, polyethylene-polyoxypropylene-blocking polymers, lanolin, saccharides such as lactose, glucose, and sucrose; and starch such as cornstarch and potato starch; cellulose and its derivatives such as sodium carboxymethyl cellulose, ethyl cellulose and cellulose acetate; gum powder; malt; gelatin; talc; excipients such as cocoa butter and suppository waxes; and oils such as peanut, cottonseed, safflower, sesame, olive, corn and soybean oils; diols such as propylene glycol and polyethylene glycol; esters such as ethyl oleate and ethyl laurate; agar; buffers such as magnesium hydroxide and aluminum hydroxide; alginic acid; pyrogen-free water; isotonic salts; and Ringer's solution; ethanol, phosphate buffer solution, and other non-toxic suitable lubricants such as sodium lauryl sulphate and magnesium stearate, coloring agents, releasing agents, coating coatings, sweeteners, flavourings and fragrances, preservatives and antioxidants.

The pharmaceutical compositions of the present disclosure may be prepared according to the disclosure using any method known to those skilled in the art. For example, conventional mixing, dissolution, granulation, emulsification, milling, encapsulation, embedding or lyophilization processes.

Pharmaceutical dosage forms of the compounds of the present disclosure may be provided in the form of immediate release, controlled release, sustained release or target drug release systems. For example, commonly used dosage forms include solutions and suspensions, (micro)emulsions, ointments, gels and patches, liposomes, tablets, sugar-coated pills, soft-shell or hard-shell capsules, suppositories, ovules, implants, amorphous or crystalline powders, aerosols and lyophilized formulations. Depending on the route of administration used, special devices may be required to administer or give the drug, such as syringes and needles, inhalers, pumps, injection pens, applicators, or special flasks. Pharmaceutical dosage forms often consist of a drug, an excipient and a container/sealing system. One or more excipients (also known as inactive ingredients) may be added to the compounds of the present disclosure to improve or facilitate drug manufacture, stability, administration, and safety, and may provide methods of obtaining a desired drug release profile. Thus, the type of excipient added to the drug may depend on various factors, such as the physical and

chemical properties of the drug, the route of administration and the preparation step. Pharmaceutical excipients exist in the art and include those listed in various pharmacopoeias (see U.S. Pharmacopeia (USP), Japanese Pharmacopoeia (JP), European Pharmacopoeia (EP), and British pharmacopoeia (BP); publications of Center for Drug Evaluation and Research (CEDR) of the U.S. Food and Drug Administration (www.fda.gov), e.g. Inactive Ingredient Guide (1996); Hand book of Pharmaceutical Additives, 2002, by Ash and Ash, Synapse Information Resources, Inc. Endicott NY; etc.).

Pharmaceutical dosage forms of the compounds of the present disclosure may be manufactured by any of the methods known in the art, for example, by conventional mixing, sieving, dissolving, melting, granulating, manufacturing sugar-coated pills, tableting, suspending, extruding, spray-drying, milling, emulsifying, (nano/micron scale) encapsulation, encapsulation, or lyophilization processes. As described above, the compositions of the present disclosure may include one or more physiologically acceptable inactive ingredients that will facilitate the processing of the active molecule into a formulation for pharmaceutical use.

Said pharmaceutical compositions and dosage forms may comprise one or more compounds of The present disclosure or one or more pharmaceutically acceptable salts thereof as the active component. The pharmaceutically acceptable carrier may be solid or liquid. Formulations in solid form include powders, tablets, pills, ingots, capsules, suppositories and dispersible granules. The solid carrier may also be one or more substances that act as diluents, flavouring agents, solubilizing agents, lubricants, suspending agents, binders, preservatives, tablet disintegrants or encapsulating materials. In powders, the carrier is usually a finely ground solid which is a mixture with a finely ground active component. In tablets, the active component is usually mixed in a suitable ratio with a carrier having the necessary binding capacity and compacted to the desired shape and size. Suitable carriers include, but are not limited to, magnesium carbonate, magnesium stearate, talc, saccharide, lactose, pectin, dextrin, starch, gelatin, methylcellulose, sodium carboxymethylcellulose, low melting waxes, cocoa butter and the like. The formulation of the active compound may include an encapsulating material as a carrier, providing capsules in which the active component with or without a carrier is surrounded by the carrier to which it is bound.

Other forms suitable for oral administration include liquid form preparations, including emulsions, syrups, elixirs, aqueous solutions, aqueous suspensions, or solid form preparations intended to be converted to liquid form preparations shortly before use. The emulsion may be prepared in a solution, for example in a propylene glycol aqueous solution, or may contain an emulsifier such as lecithin, dehydrated sorbitan monooleate, or gum arabic. Aqueous solutions can be prepared by dissolving the active component in water and adding suitable colorants, flavorings, stabilizers and thickeners. Aqueous suspensions can be prepared by dispersing fine particles of the active ingredient in water with binders such as natural or synthetic gums, resins, methyl cellulose, carboxymethyl cellulose and other commonly used suspending agents. Solid formulations include solutions, suspensions and emulsions, and may contain, in addition to the active component, coloring agents, flavorings, stabilizers, buffers, artificial and natural sweeteners, dispersing agents, thickeners, solubilizers, and the like.

For parenteral administration, the pharmaceutical compositions of The present disclosure may be in the form of sterile injectable or infusible injectable formulations, e.g., as sterile aqueous or oily suspensions. The suspension may be formulated according to techniques known in the art using a suitable dispersant or wetting agent (e.g., Tween 80) and suspending agent. The sterile injectable or infusible formulation may also be a sterile injectable or infusible solution or suspension in a non-toxic parenterally acceptable diluent or solvent. For example, the pharmaceutical composition may be a solution in 1,3-butanediol. Other examples of acceptable media and solvents that may be used in the pharmaceutical compositions of the present disclosure include, but are not limited to, mannitol, water, Ringer's solution, and isotonic sodium chloride solution. In addition, sterile non-volatile oils are commonly used as solvents or suspension media. Any mild non-volatile oil, including synthetic mono- or diglycerides of glycerol, may be used for this purpose. Fatty acids such as oleic acid and its glycerolipid derivatives can be used in the preparation of injectables, as well as natural pharmaceutically acceptable oils such as olive oil or castor oil, in particular their polyoxyethylated forms. These oil solutions or suspensions may also contain long chain alcohol diluents or dispersants. Solutions for parenteral use may also include suitable stabilisers and, if desired, buffering substances. Suitable stabilisers include antioxidants such as sodium bisulphate, alone or in combination, sodium sulphite or ascorbic acid, citric acid, and salts thereof and EDTA sodium salt. The parenteral solution may also comprise preservatives such as benzalkonium chloride, p-hydroxybenzoic acid or propyl p-hydroxybenzoate and chlorobutanol.

The therapeutically effective dose may first be estimated using various methods well known in the art. The initial dose for use in animal studies may be based on the effective concentration established in the cell culture assay. Dosage ranges suitable for human individuals can be determined, for example, using data obtained from animal studies and cell culture assays. In some embodiments, the compounds of the present disclosure can be prepared as a medicament for oral administration.

An effective amount or a therapeutically effective amount or dose of an agent (e.g., a compound of the present disclosure) refers to the amount of the agent or compound that causes an improvement in the symptoms or prolongation of survival of an individual. The toxicity and therapeutic efficacy of said molecules can be determined in cell cultures or experimental animals by standard pharmaceutical procedures, e.g., by measuring the $LD_{50}$ (the dose that is lethal to

50% of the population) and the $ED_{50}$ (the dose that is therapeutically effective for 50% of the population). The dose ratio of the toxic effect to the therapeutic effect is the therapeutic index, which can be expressed as $LD_{50}/ED_{50}$. Agents showing a high therapeutic index are preferred.

An effective amount or therapeutically effective amount is the amount of a compound or pharmaceutical composition that will trigger a biological or medical response in a tissue, system, animal or human being that is being explored by a researcher, veterinarian, physician or other clinician. The dose is preferably in the range of circulating concentrations that include an $ED_{50}$ of minimal or no toxicity. The dose may vary within this range, depending on the dosage form used and/or the route of administration used. The correct formulation, route of administration, dosage, and dosing interval should be selected according to methods known in the field, taking into account the particularities of the individual condition.

Dosages and intervals may be individually adjusted to provide plasma levels of the active fraction sufficient to obtain the desired effect; i.e., the minimum effective concentration (MEC). The MEC will vary from compound to compound, but can be estimated, for example, from *in vitro* (*in vitro*) data and animal experiments. The dose necessary to obtain MEC will depend on individual characteristics and route of administration. In the case of local administration or selective uptake, the effective local concentration of the drug may be independent of the plasma concentration.

The amount of agent or composition administered may depend on a variety of factors, including the sex, age and weight of the individual being treated, the severity of the ailment, the mode of administration and the judgement of the prescribing physician.

Where desired, the composition of the present disclosure may be provided in packages or dispensing devices containing one or more unit dosage forms (containing the active ingredient). By way of example, said packages or devices may comprise metal or plastic foil (e.g., styrofoam packages) or glass and rubber stoppers, such as in vials. Said packaging or dispensing device may be accompanied by dosing instructions. Compositions comprising the compounds of The present disclosure formulated in a compatible pharmaceutical carrier may also be prepared, placed in suitable containers and labelled for the treatment of specified conditions.

Unless otherwise specified, all technical and scientific terms used herein have the standard meanings in the field to which the subject matter belongs. Where more than one definition of a term exists, the definition herein shall prevail.

Definitions of groups

Unless otherwise indicated, the following definitions used herein should be applied. For the purposes of the present disclosure, the chemical elements are consistent with the CAS edition of the Periodic Table of Elements, and the Handbook of Chemistry and Physics, 75th edition, 1994. In addition, general principles of organic chemistry can be found in "Organic Chemistry", Thomas Sorrell, University Science Books, Sausalito: 1999, and "March's Advanced Organic Chemistry "by Michael B. Smith and Jerry March, John Wiley & Sons, New York: 2007, the entire contents of which are incorporated herein by reference.

In this specification, groups and their substituents may be selected by those skilled in the art to provide stable structural portions and compounds. When a substituent is described by a conventional chemical formula written from left to right, the substituent likewise includes the chemically equivalent substituent obtained when the structural formula is written from right to left.

Certain chemical groups defined herein are preceded by simplified symbols to indicate the total number of carbon atoms present in the group. For example, $C_1$-$C_6$ alkyl means an alkyl group having a total of 1, 2, 3, 4, 5, or 6 carbon atoms as defined below. The total number of carbon atoms in the simplified symbols does not include carbon that may be present in substituents of said groups.

Herein, the range of values defined in the substituent such as 0 to 4, 1-4, 1 to 3, etc. indicates an integer in that range, e.g., 1-6 is 1, 2, 3, 4, 5, and 6.

In addition to the foregoing, when used in the specification and claims of this application, the following terms have the meanings indicated below unless otherwise specifically indicated.

The term "comprise" is open-ended, i.e., includes what is specified in the present disclosure, but does not exclude other aspects.

The term "substituted" means the substitution of any one or more hydrogen atoms on a given atom by a substituent, including heavy hydrogen and hydrogen variants, provided that the valence state of the given atom is normal and the substituted compound is stable.

In general, the term "substituted" indicates that one or more hydrogen atoms in the given structure are replaced by specific substituents. Further, when the group is substituted with more than one said substituent, said substituents are independent of each other, i.e., said more than one substituent may be different from each other or identical. Unless otherwise indicated, a substituent group may be substituted at various substitutable positions of the substituted group. When more than one position in the given structural formula can be substituted by one or more substituents selected from specific groups, then the substituents may be substituted identically or differently at various positions.

In various parts of this specification, the substituents of the disclosed compounds of the present disclosure are disclosed according to the type or range of groups. In particular, it is noted that the present disclosure includes separate sub-combinations of each of the individual members of these moiety classes and ranges. For example, the terms "$C_1$-$C_6$ alkyl" or "$C_{1-6}$ alkyl" refer, in particular, to the independently disclosed methyl, ethyl, $C_3$ alkyl, $C_4$ alkyl, $C_5$ alkyl, and $C_6$ alkyl groups; and the term "$C_{1-4}$ alkyl" refers, in particular, to the independently disclosed methyl, ethyl, $C_3$ alkyl (i.e., propyl, including ortho-propyl and iso-propyl), and $C_4$ alkyl (i.e., butyl, including n-butyl, iso-butyl, sec-butyl, and tert-butyl) groups.

The term "halogen" is selected from F, Cl, Br, or I, in particular F or Cl.

In this application, the term "alkyl", as a group or as part of another group, refers to a saturated aliphatic hydrocarbon group, which is a straight or branched group comprising from 1 to 12 carbon atoms, preferably a straight or branched alkyl group comprising from 1 to 6 carbon atoms. The general formula is $C_nH_{2n+1}$. The term "$C_1$-$C_6$ alkyl", "$C_{1-6}$ alkyl" means that the alkyl portion contains 1, 2, 3, 4, 5 or 6 carbon atoms. In one embodiment, said "alkyl" means $C_1$-$C_6$ alkyl. In one embodiment, said "alkyl" means $C_1$-$C_4$ alkyl.

Lower alkyl groups comprising 1 to 6 carbon atoms, non-limiting embodiments of which include methyl, ethyl, n-propyl, isopropyl, n-butyl, isobutyl, tert-butyl, sec-butyl, n-amyl, 1,1-dimethylpropyl, 1,2-dimethylpropyl, 2,2-dimethylpropyl, 1-ethylpropyl, 2-methylbutyl, 3-methylbutyl, hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and others.

The term "cycloalkyl" refers to a saturated monocyclic or polycyclic carbocyclic substituent consisting solely of a carbon atom and a hydrogen atom, which may be connected to the rest of the molecule by a single bond via any suitable carbon atom; when polycyclic, it may be a parallel, bridged, or spiro-conjugated (i.e., where both hydrogens on the same carbon atom have been substituted with alkanediyl group) ring system. In one embodiment, a typical monocyclic cycloalkyl group is, for example, cyclopropyl, cyclobutyl, cyclopentyl, or cyclohexyl.

For the purposes of this application, the term "heteroaryl", as part of a moiety or other group, refers to a group ("4- to 16-membered heteroaryl") of a 4- to 16-membered monocyclic or dicyclic 4n+2 aromatic ring system (e.g., having 6 or 10 shared p-electrons in the cyclic array) having carbon atoms and 1-3 heteroatoms (each of which is independently selected from nitrogen, oxygen, and sulphur) provided in the aromatic ring system. In heteroaryl groups comprising one or more nitrogen atoms, the point of attachment may be either a carbon or a nitrogen atom, as far as the valency allows.

In some embodiments, said heteroaryl is a 4- to 6-membered heteroaryl with a heteroatom selected from one or more of N, O, and S, with a number of heteroatoms from 1 to 3, more preferably a 5- to 6-membered heteroaryl.

Exemplary 5-membered heteroaryl groups include, but are not limited to: pyrrolyl, furanyl, thienyl, imidazolyl, pyrazolyl, oxazolyl, isoxazolyl, thiazolyl, isothiazolyl, triazolyl, oxadiazolyl, thiadiazolyl, furazanyl, oxatriazolyl, or tetrazolyl. Exemplary 6-membered heteroaryl groups include, but are not limited to: pyridinyl, pyrazinyl, pyridazinyl, pyrimidinyl, triazinyl, or tetrazinyl.

The terms "moiety", "structural moiety", "chemical moiety", "group", "chemical group" used herein refer to a specific fragment or functional group in a molecule. A chemical moiety is usually considered to be a chemical entity embedded in or attached to a molecule.

When no indication is given in the enumerated substituents as to the atom through which they are attached to a compound included, but not specifically mentioned, in the general formula of the chemical structure, such substituents may be bonded through any of their atomic phases. Combinations of substituents and/or variants thereof are permissible only if such combinations result in stable compounds.

In various parts of the present disclosure, linking substituents are described. When the structure clearly requires a linking group, the Markush variables listed for that group are to be understood as linking groups. For example, if the structure requires a linking group and the definition of a Markush group for that variable lists "alkyl" or "aryl", it should be understood that the "alkyl" or "aryl" represents a linking alkanediyl group or araldiyl group, respectively.

In some specific structures, when an alkyl group is clearly represented as a linking group, the alkyl group represents a linking alkanediyl group, e.g., the $C_1$-$C_6$ alkyl group in the group "halo-$C_1$-$C_6$ alkyl" is to be understood as a $C_1$-$C_6$ alkanediyl group.

The term "alkanediyl" denotes a saturated divalent hydrocarbon group obtained by removing two hydrogen atoms from a saturated straight or branched chain hydrocarbon group. Examples of alkanediyl groups include methylene ($-CH_2-$), ethylene {including $-CH_2CH_2-$ or $-CH(CH_3)-$}, isopropylene {including $CH(CH_3)CH_2-$ or $-C(CH_3)_2-$}, and the like.

Unless otherwise specified, all technical and scientific terms used herein have the standard meanings in the field to which the subject matter belongs. Where more than one definition of a term exists, the definition herein shall prevail.

It should be understood that the singular forms used in the present disclosure, such as "a", include plural references unless otherwise specified. Furthermore, the terms "comprise" and "include" is open-ended and not closed-ended, i.e., it includes what is specified in the present disclosure, but does not exclude other aspects.

Unless otherwise indicated, the present disclosure employs conventional methods of mass spectrometry and elemental analysis, and the steps and conditions may be referred to conventional operating steps and conditions in the

field.

Unless otherwise indicated, the present disclosure employs standard nomenclature and standard laboratory steps and techniques in analytical chemistry, organic synthetic chemistry, and optics. In some cases, standard techniques are used for chemical synthesis, chemical analysis, and luminescent device performance testing.

Furthermore, it is to be noted that, unless otherwise expressly stated, the description "...independently" used in the present disclosure is to be broadly construed to mean that the various individuals described are independent of each other and may be independently of the same or different specific moieties. In more detail, the description "...independently" can mean either that the specific options expressed between the same symbols in different groups do not affect each other, or that the specific options expressed between the same symbols in the same groups do not affect each other.

It will be understood by those skilled in the art that, according to the conventions used in the art, the " 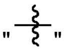 " used in the structural formulae describing the groups in the present application means that the corresponding group is connected to other fragments, groups, in the compound by means of this site.

"Pharmacologically acceptable" refers to conditions that can be used to prepare pharmaceutical compositions, are generally safe and non-toxic, are not biologically or otherwise undesirable, and include conditions that are acceptable for veterinary use and for human pharmaceutical use.

The term "excipient" refers to a pharmaceutically acceptable chemical substance, such as a reagent known to those of ordinary skill in the art of pharmacy, used to assist in the administration of medicinal products. It is a compound that can be used in the preparation of a pharmaceutical component, is generally safe, non-toxic, and is biologically or otherwise undesirable, and includes excipients that are acceptable for veterinary and human pharmaceuticals. Typical excipients include binders, surfactants, diluents, disintegrants and lubricants.

The term "effective therapeutic amount" means the amount of compound used which, when given to a subject to treat a disease state, is sufficient to achieve such treatment of the disease state. The "effective therapeutic amount" will vary depending on the compound, the disease state being treated, the severity of the disease being treated, the age and relative health of the subject, the route and mode of administration, and the judgement of the treating medical or veterinary practitioner.

As used in this application, "treat" or " treatment" means obtaining a beneficial or desired result, including a clinical result. Beneficial or desired clinical outcomes include, but are not limited to, reduction or amelioration of one or more symptoms or conditions, reduction in the extent of the disease, stabilization of the disease state (e.g., no deterioration), prevention of disease transmission, delay or slowing of the progression of the disease, improvement or remission of the disease state, and partial or complete improvement, whether detectable or non-detectable. The term can also refer to prolonged survival compared to the expected survival corresponding to no treatment.

The term mammal refers to a human or any mammal, such as a primate, farm animal, pet animal, or laboratory animal. Examples of these animals are monkeys, cows, sheep, horses, pigs, dogs, cats, rabbits, mice and rats. Mammals are preferred to humans.

On the basis of not violating the common knowledge in the field, the above preferred conditions can be arbitrarily combined to obtain the preferred embodimetns of the present disclosure.

The reagents and raw materials used in the present disclosure are commercially available.

The positive progressive effect of the present disclosure lies in the fact that the phenyldihydropyrimidine compounds provided in the present application have good anti-HBV (Hepatitis B virus) activity and can be used in the preparation of drugs for the treatment and/or prevention of HBV infection.

## Detailed Description of the Invention

The present invention is further described below by way of examples, but does not thereby limit the present invention to the scope of the described examples. Experimental methods for which specific conditions are not indicated in the following embodiments were selected in accordance with conventional methods and conditions, or in accordance with the trade descriptions.

### Example 1

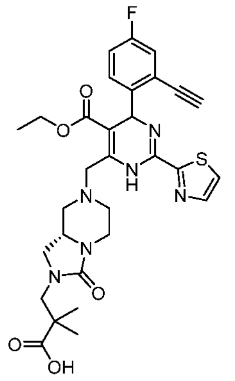

SZ-023089RAC

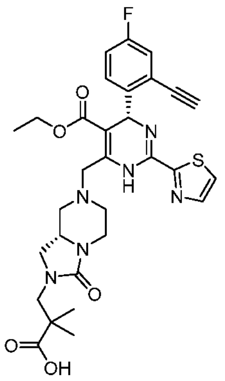

或

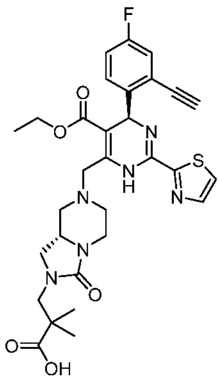

SZ-023089

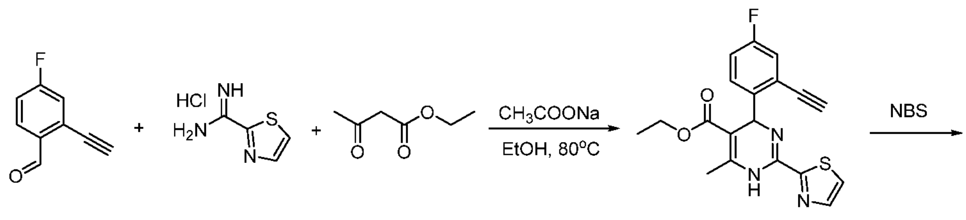


023119A2

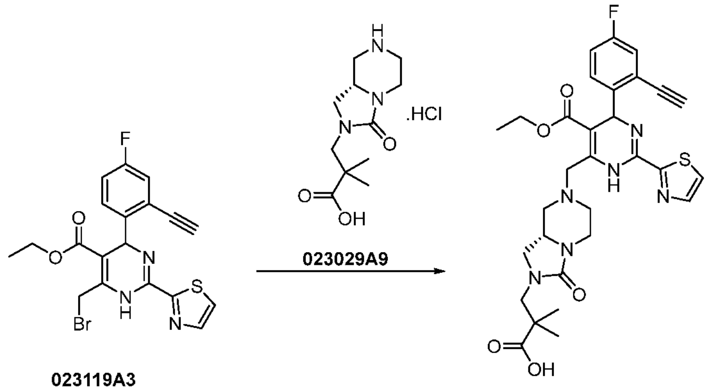


023119A3

SZ-023089RAC

**Step 1: Synthesis of 023119A2**

To a solution of compound 2-alkynyl-4-fluorobenzaldehyde (5.0 g, 33.78 mmol) in ethanolic solution (60 mL), thiazolemethanimidium hydrochloride (5.5 g, 33.78 mmol), ethyl acetoacetate (6.8 g, 33.78 mmol), and sodium acetate (3.0 g, 37.16 mmol) were added at room temperature, and t he reaction solution was stirred and reacted overnight at 80°C. The reaction solution was concentrated to remove the solvent, and the resulting residue was purified by silica gel column chromatography (EA/PE = 33% to 100%) to obtain a black solid (8.0 g, 64%), which was then pulped with ether for 2 h. The filter cake was collected after filtration, and dried to obtain the pure yellow solid **023119A2** (4.0 g, 32.1% yield). LCMS $(M+H)^+$ m/z calcd 370.1, found 370.1.

**023119A2RAC** was isolated by chiral HPLC purification (chiral analytical method: SFC: IE (CHIRALPAK® 250mmL*4.6mm Particle Size: 5um) $-CO_2$-ACN-65-35; $CO_2$ Flow Rate: 1.95 (g/min); Co-Solvent Flow Rate: 1.05 (ml/min); T: 40°C; wavelength: 254 nm; elution time: 7 min) to obtain two isomers **023119A2P1** (1.4 g) RT = 2.45 min and **023119A2P2** (1.4 g) RT = 3.68 min. LCMS $(M+H)^+$ m/z calcd. 370.1, found 370.1.

**023119A2P1** $^1$H NMR (DMSO-d6, 400 MHz): δ 9.84 (brs, 1H), 7.97 (d, $J$ = 2.8 Hz, 1H), 7.89 (d, $J$ = 2.4 Hz, 1H), 7.37-7.29 (m, 2H), 7.22 (dt, $J_1$ = 8.8 Hz, $J_2$ = 2.8 Hz, 1H), 6.04 (s, 1H), 4.41 (s, 1H), 3.92 (q, $J$ = 7.2 Hz, 2H), 2.44 (s, 3H), 1.04 (t, $J$ = 7.2 Hz, 3H).

**Step2: Synthesis of 023119A3**

To a solution of compound **023119A2** (700 mg, 1.90 mmol) in acetonitrile (15 mL) was added N-bromosuccinimide (337.7 mg, 1.90 mmol) at room temperature and reacted for 10 min at 10°C. The reaction solution was concentrated and the residue was purified by silica gel column chromatography (EA/PE = 1:10) to give the pale yellow colloidal substance **023119A3** (400 mg, 47.1% yield). LCMS $(M+H)^+$ m/z calcd. 448.0, found 448.0.

$^1$HNMR ($CDCl_3$, 400MHz): δ 7.83 (d, *J* = 3.2 Hz, 1H), 7.51-7.46 (m, 1H), 7.40 (dd, $J_1$ = 8.8Hz, $J_2$ = 5.2 Hz, 1H), 7.20 (dd, $J_1$ = 8.8Hz, $J_2$ = 2.8 Hz, 1H), 7.06-7.01 m, 1H), 6.17 (s, 1H), 4.89 (s, 1H), 4.89-4.63 (m, 1H), 4.11(q, *J* = 7.2 Hz, 2H), 3.46-3.43 (m, 1H), 1.15 (t, *J* = 7.2 Hz, 3H).

**Step 3: Synthesis of SZ-023089RAC**

To a solution of compound **023119A3** (100 mg, 0.22 mmol) in dichloromethane (3 mL) at room temperature, compound **023029A9** (62 mg, 0.22 mmol, prepared with reference to Patent CN108718527A) was added, followed by diisopropylethylamine (58 mg, 0.45 mmol). The reaction solution was stirred at room temperature for 4 hours. The reaction solution was concentrated to remove the solvent. The resulting residue was purified by preparative HPLC to give the yellow solid compound **SZ-023089RAC** (30 mg, 22.2% yield).

Liquid phase mass spectrometry [Mobile phase: elution was maintained for 6 minutes at a flow rate of 1.5 mL per minute on a gradient from 80% water (with 0.05% trifluoroacetic acid) and 20% acetonitrile to 50% water (with 0.05% trifluoroacetic acid) and 50% acetonitrile at a column temperature of 40°C. Column: Xbridge C18, 50×4.6 mm. A purity of 98.681% at 214 nm., Rt = 3.877 min. LCMS $(M+H)^+$ m/z calcd. 609.4, found 609.4.

$^1$H NMR ($CD_3OD$, 400 MHz): δ 7.93-7.94 (m, 1H), 7.72-7.73 (m, 1H), 7.39-7.43 (m, 1H), 7.19-7.22 (q, *J* = 9.2 Hz, 2.8 Hz, 1H), 7.06-7.11 (m, 1H), 6.19 (s, 1H), 4.01-4.06 (m, 3H), 3.75-3.88 (m, 4H), 3.47-3.57 (m, 1H), 3.37-3.41 (m, 1H), 3.07-3.27 (m, 3H), 2.88-2.98 (m, 1H), 2.72-2.82 (m, 1H), 2.12-2.32 (m, 2H), 1.18-1.19 (m, 6H), 1.13 (t, *J* = 7.2 Hz, 3H).

Referring to the synthesis of **SZ-023089RAC,** the yellow solid compound **SZ-023089** (31 mg, 22.4% yield) was obtained from **023119A2P1.**

Liquid phase mass spectrometry [Mobile phase: elution was maintained for 6 minutes at a flow rate of 1.5 mL per minute on a gradient from 90% water (with 0.1% trifluoroacetic acid) and 10% acetonitrile to 40% water (with 0.1% trifluoroacetic acid) and 60% acetonitrile at a column temperature of 40°C. Column: Kinetex 5um EVO C18, 50x4.6mm. A purity of 99.59% at 214nm, Rt = 3.055 min. LCMS $(M+H)^+$ m/z calcd. 609.2, found 609.1.

$^1$H NMR (DMSO-*d6*, 400 MHz): δ 9.64-9.51 (brs, 1H), 8.03 (d, *J* = 3.2 Hz, 1H), 7.93 (d, *J* = 3.2 Hz, 1H), 7.38 (dd, $J_1$ = 8.8 Hz, $J_2$ = 6.0 Hz, 1H), 7.32 (dd, $J_1$ = 9.6 Hz, $J_2$ = 2.8 Hz, 1H), 7.19 (dt, $J_1$ = 8.8 Hz, $J_2$ = 2.8 Hz, 1H), 6.08 (s, 1H), 4.47 (s, 1H), 3.93 (q, *J* = 7.2 Hz, 2H), 3.92-3.81 (m, 2H), 3.71-3.60 (m, 2H), 3.39-3.35 (m, 2H), 3.32-3.14 (m, 2H), 3.01-2.93 (m, 2H), 2.84-2.78 (m, 2H), 2.16-2.11 (m, 1H), 2.01 (t, *J* = 10.8 Hz, 1H), 1.07 (s, 6H), 1.05 (t, *J* = 7.2 Hz, 3H).

**Example 2**

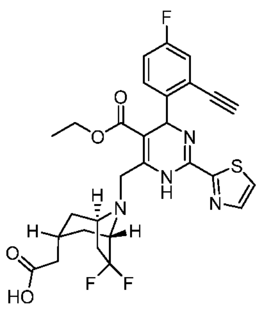

SZ-023091RAC

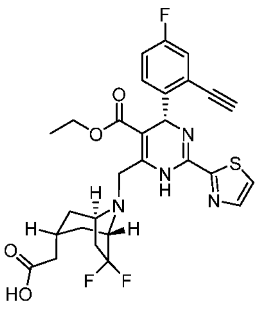

SZ-023091

或

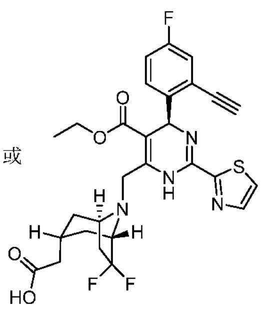

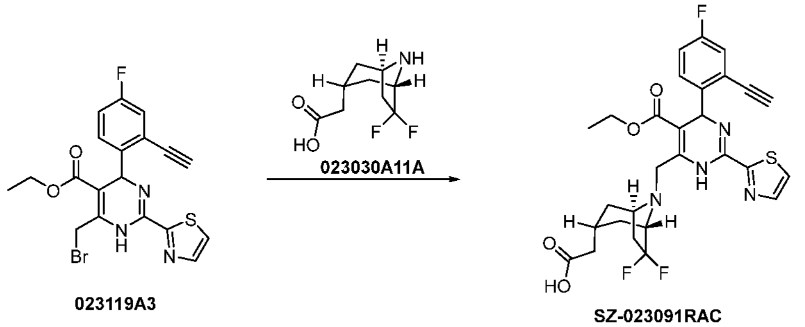


023119A3 SZ-023091RAC

To a solution of compound **023119A3** (100 mg, 0.22 mmol) in dichloromethane (3 mL) at room temperature, compound **023030A11A** (54 mg, 0.22 mmol, prepared with reference to Patent CN105209470 B) was added, followed by diisopropylethylamine (58 mg, 0.45 mmol). The reaction solution was stirred at room temperature for 4 hours. The reaction solution was concentrated to remove the solvent. The resulting residue was purified by preparative HPLC to give the yellow solid compound **SZ-023091RAC** (30 mg, 25.2% yield).

Liquid chromatography mass spectrometry [Mobile phase: elution was maintained for 6 min at a flow rate of 1.5 mL per minute on a gradient from 80% water (with 0.1% trifluoroacetic acid) and 20% acetonitrile to 40% water (with 0.1% trifluoroacetic acid) and 60% acetonitrile at a column temperature of 40°C. Column: Kinetex 5 um EVO C18, 50×4.6 mm. A purity of 98.90% at 214 nm, Rt = 3.297 min. LCMS $(M+H)^+$ m/z calcd. 573.1, found 573.1.

$^1H$ NMR ($CD_3OD$, 400 MHz): δ 7.94 (d, *J* = 3.2 Hz, 1H), 7.71-7.72 (m, 1H), 7.39-7.43 (m, 1H), 7.19-7.22 (m, 1H), 7.08-7.11 (m, 1H), 6.19 (d, *J* = 4.4 Hz, 1H), 4.44-4.49 (m, 0.5H), 4.20 (s, 1H), 3.99-4.05 (m, 2H), 3.89-3.93 (m, 0.5H), 3.81-3.82 (m, 1H), 3.45-3.48 (m, 1H), 2.63-2.70 (m, 2H), 2.36-2.52 (m, 4H), 2.17-2.20 (m, 2H), 1.67-1.73 (m, 1H), 1.37-1.39 (m, 1H), 1.12-1.16 (m, 3H).

Referring to the synthesis of **SZ-023089RAC** and **SZ-023091RAC,** the yellow solid **SZ-023091** (39 mg, 31%) was obtained from **023119A2P1** as starting material. Liquid chromatography mass spectrometry [Mobile phase: elution was maintained for 6 min at a flow rate of 1.5 ml per minute on a gradient from 80% water (with 0.1% trifluoroacetic acid) and 20% acetonitrile to 40% water (with 0.1% trifluoroacetic acid) and 60% acetonitrile at a column temperature of 40°C. Column: Kinetex 5um EVO C18 , 50x4.6 mm, a purity of 99.09%, Rt = 3.295 min. LCMS$(M+H)^+$ m/z calcd. 573.2, found 573.1.

$^1$HNMR (DMSO-d6, 400MHz): δ 7.99 (d, *J* = 3.2 Hz, 1H), 7.92 (d, *J* = 3.2 Hz, 1H), 7.40 (dd, $J_1$ = 8.8 Hz, $J_2$ = 6.0 Hz, 1H), 7.31 (dd, $J_1$ = 9.6 Hz, $J_2$ = 2.4 Hz, 1H), 7.23-7.19 (m, 1H), 6.08 (s, 1H), 4.46 (s, 1H), 4.21-4.03 (m, 2H), 3.96-3.80 (m, 3H), 3.45-3.34 (m, 1H), 2.71-2.61 (m, 1H), 2.38-2.07 (m, 6H), 1.54-1.51 (m, 1H), 1.30-1.24 (m, 1H), 1.06 (t, *J* = 7.2 Hz, 3H).

**Example 3**

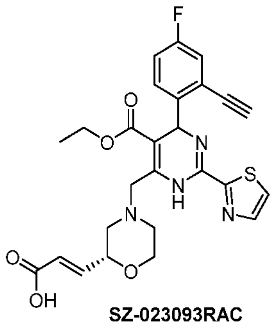

SZ-023093RAC

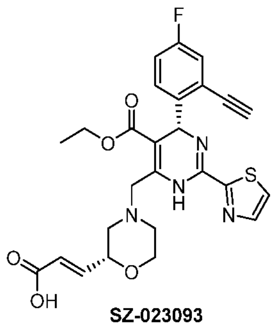

SZ-023093

或

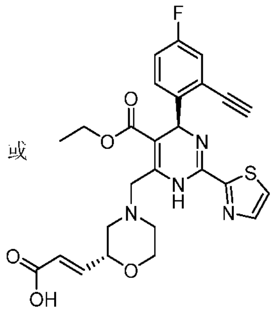

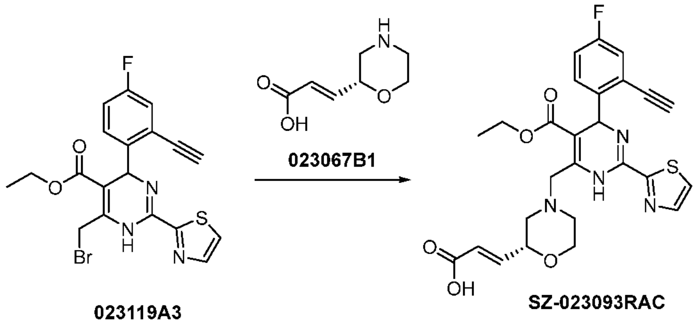


023119A3

SZ-023093RAC

To a solution of compound **023119A3** (100 mg, 0.22 mmol) in dichloromethane (3 mL) at room temperature, compound **023067B1** (72 mg, 0.22 mmol, prepared with reference to Patent CN109790145 B) was added, followed by diisopropylethylamine (86 mg, 0.669 mmol). The reaction solution was stirred at room temperature for 4 hours. The reaction solution was concentrated to remove the solvent. The resulting residue was purified by preparative HPLC to give the yellow solid compound **SZ-023093RAC** (85 mg, 73% yield).

Liquid chromatography mass spectrometry [Mobile phase: elution was maintained for 6 min at a flow rate of 1.5 mL per minute on a gradient from 80% water (with 0.1% trifluoroacetic acid) and 20% acetonitrile to 40% water (with 0.1% trifluoroacetic acid) and 60% acetonitrile at a column temperature of 40°C. Column: Kinetex 5 um EVO C18, 50×4.6mm. A purity of 95.90% at 214nm, Rt = 2.848 min. LCMS $(M+H)^+$ m/z calcd. 525.0, found 525.0.

$^1H$ NMR (DMSO-d6, 400 MHz): δ 9.50 (br s, 1H), 8.04-8.01 (m, 1H), 7.94-7.92 (m, 1H), 7.43-7.19 (m, 3H), 6.73-6.63 (m, 1H), 6.09-6.07 (m, 1H), 5.98-5.88 (m, 1H), 5.80-5.78 (m, 1H), 4.51-4.46 (m, 1H), 4.32-4.22 (m, 1H), 3.97-3.81 (m, 5H), 3.67-3.61 (m, 1H), 3.04-2.65 (m, 2H), 2.38-2.06 (m, 2H), 1.03 (t, $J$ = 7.6 Hz, 3H).

Referring to the synthesis of **SZ-023089RAC** and **SZ-023093RAC,** the yellow solid chemistry **SZ-023093** (33 mg, 28.2% yield) was obtained from 023119A2P1 as starting material.

Liquid Mass Spectrometry [Mobile phase: elution was maintained for 6 minutes at a flow rate of 1.5 mL per minute on a gradient from 80% water (with 0.02% ammonium acetate) and 20% acetonitrile to 50% water (with 0.02% ammonium acetate) and 50% acetonitrile at a column temperature of 40°C. Column: Xbridge C18, 5um 4.6×50mm. A purity of 96.00% at 214nm, Rt = 4.110min. LCMS $(M+H)^+$ m/z calcd. 525.2, found 525.3.

$^1H$ NMR (DMSO-d6, 400 MHz): δ 9.68-9.48 (br s, 1H), 8.02 (dd, $J_1$ = 6.4 Hz, $J_2$ = 3.2 Hz, 1H), 7.93 (dd, $J_1$ = 6.0 Hz, $J_2$ = 3.2 Hz, 1H), 7.40-7.37 (m, 1H), 7.32 (dd, $J_1$ = 9.6 Hz, $J_2$ = 2.8 Hz, 1H), 7.21 (dt, $J_1$ = 8.4 Hz, $J_2$ = 2.0 Hz, 1H), 6.69 (dd, $J_1$ = 16.0 Hz, $J_2$ = 4.0 Hz, 1H), 6.09 (s, 1H), 5.92 (dd, $J_1$ = 16.0 Hz, $J_2$ = 2.0 Hz, 1H), 4.47 (s, 1H), 4.22-4.20 (m, 1H), 3.95 (q, $J$ = 7.2 Hz, 2H), 3.92-3.85 (m, 3H), 3.68 (t, $J$ = 9.6 Hz, 1H), 2.93 (d, $J$ = 11.2 Hz, 1H), 2.83 (d, $J$ = 11.6 Hz, 1H), 2.38-2.35 (m, 1H), 2.07 (t, $J$ = 10.4 Hz, 1H), 1.05 (t, $J$ = 7.2 Hz, 3H).

**Example 4**

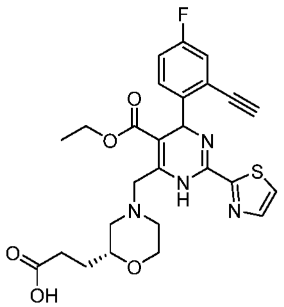

**SZ-023095RAC**

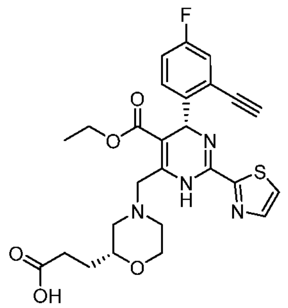

或

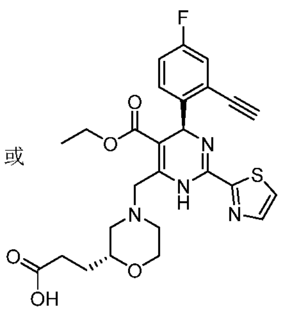

**SZ-023095**

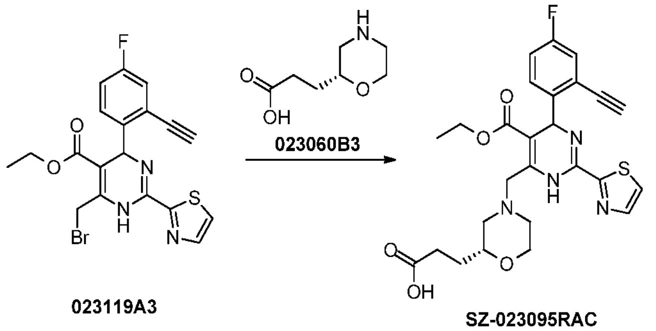


**023119A3**

**SZ-023095RAC**

To a solution of compound **023119A3** (100 mg, 0.22 mmol) in dichloromethane (3 mL) at room temperature, compound **023060B3** (72 mg, 0.22 mmol, prepared with reference to Patent CN104945395 B) was added, followed by diisopropylethylamine (86 mg, 0.669 mmol). The reaction solution was stirred at room temperature for 4 hours. The reaction solution was concentrated to remove the solvent. The resulting residue was purified by preparative HPLC to give the yellow solid compound **SZ-023095RAC** (72 mg, 62% yield).

Liquid phase mass spectrometry [Mobile phase: elution was maintained for 6 minutes at a flow rate of 1.5 mL per minute on a gradient from 85% water (with 0.1% trifluoroacetic acid) and 15% acetonitrile to 40% water (with 0.1% trifluoroacetic acid) and 60% acetonitrile at a column temperature of 40°C. Column: Kinetex 5 um EVO C18, 50×4.6 mm. A of purity 99.30% at 214 nm, Rt = 3.148 min. LCMS $(M+H)^+$ m/z calcd. 527.2, found 527.2.

$^1H$ NMR (DMSO-d6, 400 MHz): $\delta$ 9.60 (br s, 1H), 8.06-8.04 (m, 1H), 7.97-7.96 (m, 1H), 743-7.33 (m, 2H), 7.27-7.23 (m, 1H), 6.12 (s, 1H), 4.51 (s, 1H), 4.51-3.84 (m, 5H), 3.60-3.53 (m, 3H), 2.89-2.63 (m, 2H), 2.35-2.28 (m, 3H), 2.27-2.11 (m, 1H), 1.70-1.64 (m, 2H), 1.09 (t, *J* = 9.6 Hz, 3H).

Referring to the synthesis of **SZ-023089RAC** and **SZ-023095RAC,** the yellow solid chemistry **SZ-023095** (37 mg, 31.3% yield) was obtained from **023119A2P1** as raw material.

Liquid phase mass spectrometry Mobile phase: elution was maintained for 6 minutes at a flow rate of 1.5 mL per minute on a gradient from 90% water (with 0.1% trifluoroacetic acid) and 10% acetonitrile to 40% water (with 0.1% trifluoroacetic acid) and 60% acetonitrile at a column temperature of 40°C. Column: Kinetex 5 um EVO C18, 50x4.6 mm. A purity of 99.80% at 214 nm, Rt = 3.012 min. LCMS $(M+H)^+$ m/z calcd. 527.1, found 527.1.

$^1H$ NMR ($CD_3OD$, 400 MHz): $\delta$ 7.94 (d, *J* = 3.2 Hz, 1H), 7.72 (d, *J* = 3.2 Hz, 1H), 7.42 (dd, $J_1$ = 8.8 Hz, $J_2$ = 5.6 Hz, 1H), 7.21 (dd, $J_1$ = 9.2 Hz, $J_2$ = 2.8 Hz, 1H), 7.09 (dt, $J_1$ = 8.4 Hz, $J_2$ = 2.8 Hz, 1H), 6.21 (s, 1H), 4.03 (q, *J* = 7.2 Hz, 2H), 3.83-3.96 (m, 3H), 3.83 (s, 1H), 3.80-3.74 (m, 1H), 3.68-3.66 (m, 1H), 2.86-2.79 (m, 2H), 2.55-2.48 (m, 1H), 2.45-2.35 (m, 2H), 2.10-2.15 (m, 1H), 1.72-1.77 (m, 2H), 1.13 (t, *J* = 7.2 Hz, 3H).

### Example 5

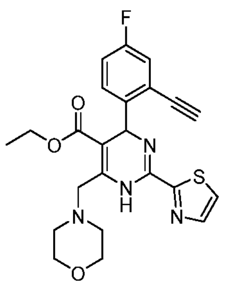

SZ-023115RAC

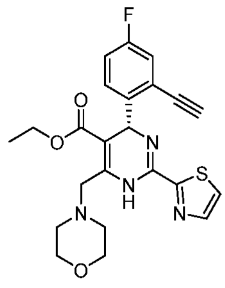

或

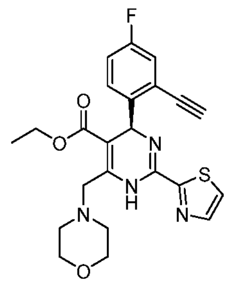

SZ-023115-P1 & SZ-023115-P2

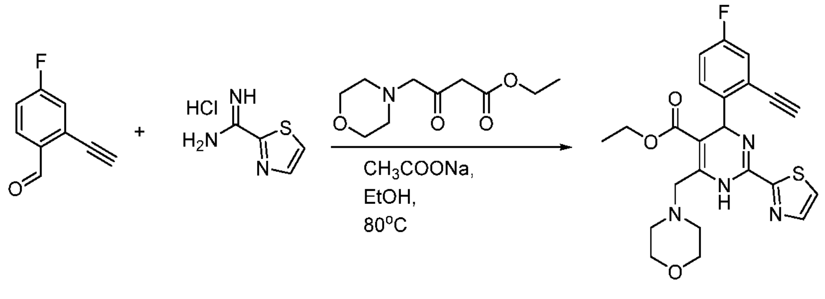


SZ-023115RAC

To a solution (8 mL) of compound 2-alkynyl-4-fluorobenzaldehyde (100 mg, 0.68 mmol) in ethanol was added 2-thiazolecarboxamide hydrochloride (112 mg, 0.68 mmol), ethyl 4-morpholin-4-yl-3-carbonyl-butyrate (146 mg, 0.68 mmol) and sodium acetate (61 mg, 0.74 mmol) at room temperature. The reaction solution was stirred at 80°C overnight. The reaction solution was concentrated to remove the solvent and the resulting residue was purified by silica gel column chromatography (EA/PE = 50%) to give **SZ-023115RAC** (100 mg) as a yellow solid. It was then further purified by preparative HPLC (ammonium bicarbonate) to give the yellow solid compound **SZ-023115RAC** (29 mg, 9% yield). Liquid chromatography mass spectrometry [Mobile phase: elution was maintained for 6 min at a flow rate of 1.5 mL per minute on a gradient from 90% water (with 0.1% trifluoroacetic acid) and 10% acetonitrile to 40% water (with 0.1% trifluoroacetic acid) and 60% acetonitrile at a column temperature of 40°C. Column: Xbridge C18; column 4.6×50mm, 3.5um. A purity of 99.78% at 214nm, Rt = 3.412 min. LCMS $(M+H)^+$ m/z calcd. 455.1, found 455.2.

$^1$H NMR (DMSO-d6, 400 MHz) δ 8.02 (d, *J* = 3.2 Hz, 1H), 7.94 (d, *J* = 2.8 Hz, 1H), 7.39-7.30 (m, 2H), 7.23-7.18 (m, 1H), 6.09 (s, 1H), 4.48 (s, 1H), 3.96-3.80 (m, 4H), 3.68-3.45 (m, 4H), 2.57-2.49 (m, 4H), 1.04 (t, *J* = 7.2 Hz, 3H).

27 mg of **SZ-023115RAC** product was chirally split into two isomers: **SZ-023115-P1** (8 mg) RT = 7.81 min and **SZ-023115-P2** (8 mg) RT = 9.80 min (chiral assay: IE (CHIRALPAK® 250mmL*4.6mm Particle Size: 5um)-Hex-EtOH-DEA-75-25-0.2; Flow: 1ml/min; T: 40°C; Wavelength: 230 nm; Elution time: 30 min).

**SZ-023115-P1**

Liquid chromatography mass spectrometry [Mobile phase: elution was maintained for 6 min at a flow rate of 1.5 mL per minute on a gradient from 90% water (with 0.1% trifluoroacetic acid) and 10% acetonitrile to 40% water (with 0.1% trifluoroacetic acid) and 60% acetonitrile at a column temperature of 40°C. Column: Xbridge C18; column 4.6×50mm, 3.5um. A purity of 99.80% at 214nm, Rt = 2.993 min. LCMS $(M+H)^+$ m/z calcd. 455.1, found 455.1.

$^1$H NMR ($CD_3OD$, 400 MHz) δ 7.84 (d, *J* = 3.2 Hz, 1H), 7.63 (d, *J* = 3.2 Hz, 1H), 7.30 (dd, *J* = 8.4 Hz, 5.6 Hz, 1H), 7.10 (dd, *J* = 9.6 Hz, 2.8 Hz, 1H), 7.00-6.95 (m, 1H), 6.09 (s, 1H), 3.95-3.88 (m, 3H), 3.75-3.69 (m, 6H), 2.51-2.49 (m, 4H), 1.03 (t, *J* = 7.2 Hz, 3H).

**SZ-023115-P2**

Liquid chromatography mass spectrometry [Mobile phase: elution was maintained for 6 min at a flow rate of 1.5 mL per minute on a gradient from 90% water (with 0.1% trifluoroacetic acid) and 10% acetonitrile to 40% water (with 0.1% trifluoroacetic acid) and 60% acetonitrile at a column temperature of 40°C. Column: Xbridge C18; column 4.6×50mm, 3.5um. A purity of 99.83% at 214nm, Rt = 2.996 min. LCMS $(M+H)^+$ m/z calcd. 455.1, found 455.0

$^1$H NMR ($CD_3OD$, 400 MHz) δ 7.84 (d, *J* = 3.2 Hz, 1H), 7.63 (d, *J* = 3.2 Hz, 1H), 7.30 (dd, *J* = 8.4 Hz, 5.6 Hz, 1H), 7.11 (dd, *J* = 9.6 Hz, 2.8 Hz, 1H), 7.00-6.96 (m, 1H), 6.09 (s, 1H), 3.95-3.88 (m, 3H), 3.74-3.69 (m, 6H), 2.51-2.49 (m, 4H), 1.03 (t, *J* = 7.2 Hz, 3H).

**Example 6**

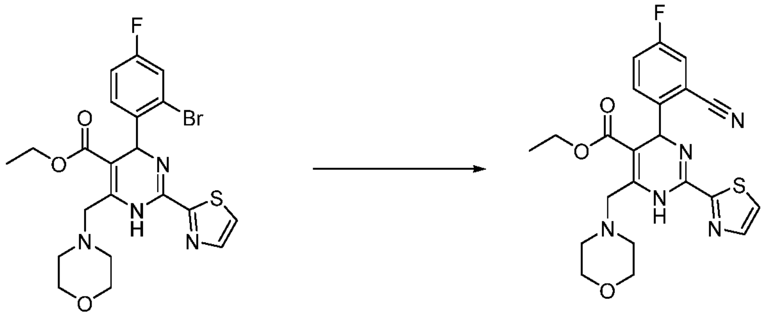

SZ-023001RAC SZ-023117RAC

**SZ-023001RAC** (60 mg, 0.118 mmol, prepared with reference to the method of Patent CN102066369 B) was dissolved in 10 ml of N,N-dimethylformamide, followed by tris(tert-butylphosphine)palladium (120 mg, 0.236 mmol), zinc cyanide (27.6 mg, 0.236 mmol), degassed and protected by nitrogen. The reaction was stirred at 120°C overnight. The reaction solution was diluted with water, extracted twice with dichloromethane, the organic phases were combined, washed with saturated brine, dried with anhydrous magnesium sulfate, filtered, and the residue obtained from the concentration of the organic phase under reduced pressure was purified by preparative HPLC (buffered with trifluoroacetic acid) to obtain the yellow solid mixture, **SZ-023117RAC** racemate (5 mg, 10%).

Liquid Mass Spectrometry [Mobile phase: elution was maintained for 6 minutes at a flow rate of 1.5 mL per minute on a gradient from 95% water (with 0.02% ammonium acetate) and 5% acetonitrile to 5% water (with 0.02% ammonium acetate) and 95% acetonitrile at a column temperature of 40°C. Column: Xbridge C18, 5um 4.6×50mm. A purity of 96.95% at 214nm, Rt = 4.231 min. LCMS $(M+H)^+$ m/z calcd. 456.2, found 456.2.

$^1H$ NMR ($CD_3OD$, 400 MHz): δ 8.01 (d, *J* = 3.2 Hz, 1H), 7.92 (d, *J* = 3.2 Hz, 1H), 7.75-7.72 (m, 1H), 7.64 (dd, $J_1$ = 8.4 Hz, $J_2$ = 2.8 Hz, 1H), 7.08 (td, $J_1$ = 8.4 Hz, $J_2$ = 2.8 Hz, 1H), 6.11 (s, 1H), 4.65 (d, *J* = 16.0 Hz, 1H), 4.57 (d, *J* = 16.0 Hz, 1H), 4.12 (q, *J* = 7.2 Hz, 2H), 4.10-4.04 (m, 4H), 3.70-3.47 (m, 4H), 1.18 (t, *J* = 7.2 Hz, 3H).

**Example 7**

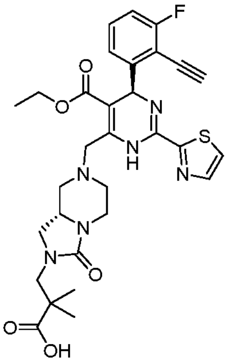

或

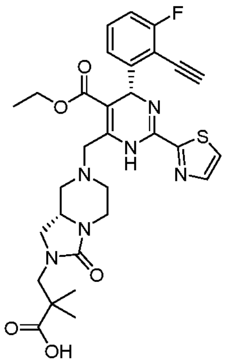

**SZ-023084A&SZ-023084B**

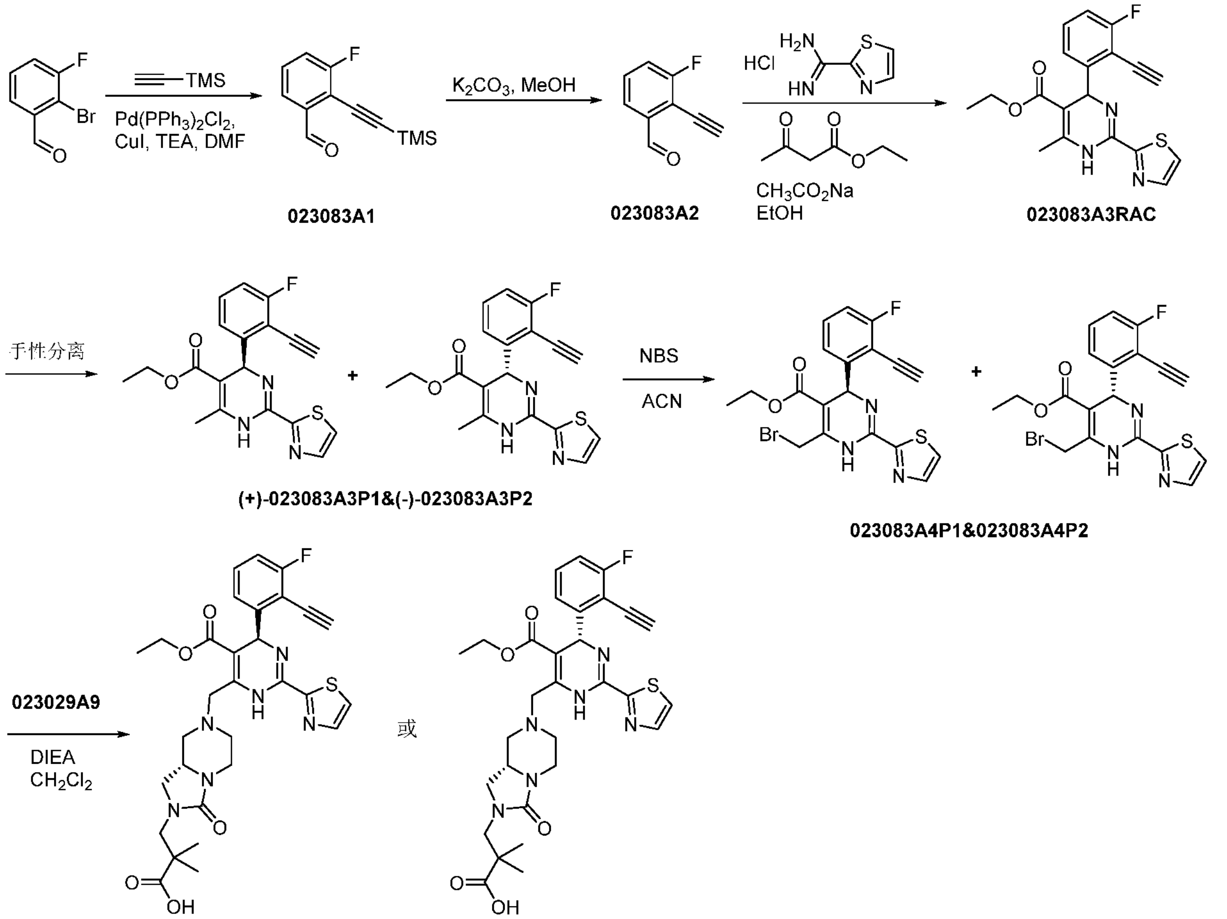


**023083A1** **023083A2** **023083A3RAC**

**(+)-023083A3P1&(-)-023083A3P2**

**023083A4P1&023083A4P2**

**SZ-023084A&SZ-023084B**

### Step 1: Synthesis of 023083A1

To 2-bromo-3-fluorobenzaldehyde (23.8 g, 117.24 mmol) in N,N,-dimethylformamide (150 mL) at room temperature were added trimethylethynylsilyl (35 g, 351.72 mmol), triethylamine (23.7 g, 234.48 mmol), bis(triphenylphosphine)palladium(II) dichloride (4.1 g, 5.86 mmol), and iodinated cuprous iodide (2.3 g, 11.72 mmol). The reaction solution was stirred overnight at room temperature under nitrogen protection. After the reaction was completed, water (200 mL) and ethyl acetate (30 mL) were added, then filtered and the filtrate was extracted with ethyl acetate (3 × 80 mL). The organic phases were combined, the solvents were removed by concentration and the resulting residue was purified by silica gel column chromatography (EA/PE = 5%-10%) to give **023083A1** (23.1 g, 89%) as a brown oil.

$^1$H NMR ($CDCl_3$, 400 MHz): $\delta$ 10.48 (d, *J* = 7.2 Hz, 1H), 7.71 (d, *J* = 7.6 Hz, 1H), 7.43-7.38 (m, 1H), 7.34-7.27 (m, 1H), 0.30 (s, 9H).

**Step 2: Synthesis of 023083A2**

To a solution of 3-fluoro-2-((trimethylsilyl)ethynyl)benzaldehyde **023083A1** (23.1 g, 105 mmol) in methanol (500 mL) at room temperature was added potassium carbonate (14.5 g, 105 mmol) and the reaction solution was stirred at room temperature overnight. After the reaction was completed, the reaction solution was concentrated to remove the solvent. The residue was extracted with water (100 mL), ethyl acetate (3×50 mL). The organic phases were combined and concentrated. The residue was separated and purified by silica gel column chromatography (EA/PE = 10%) to afford 2-alkynyl-3-fluorobenzaldehyde **023083A2** (13 g, 83%) as a yellow solid.

$^1$H NMR ($CDCl_3$, 400 MHz): δ 10.48 (s, 1H), 7.74 (d, *J* = 7.6 Hz, 1H), 7.49-7.44 (m, 1H), 7.38-7.33 (m, 1H), 3.69 (s, 1H).

**Step 3: Synthesis of 023083A3RAC**

To a solution of compound 2-alkynyl-3-fluorobenzaldehyde (5.0 g, 33.78 mmol) in ethanol (40 mL) at room temperature was added 2-thiazolecarboxamide hydrochloride (5.5 g, 33.78 mmol), ethyl acetoacetate (4.4 g, 33.78 mmol), and sodium acetate (3.0 g, 37.16 mmol), and the reaction solution was stirred overnight at 80°C. The reaction solution was concentrated to remove the solvent and the resulting residue was purified by silica gel column chromatography (EA/PE = 25%-30%) to give **023083A3RAC** (6.8 g, 54%) as black oil. Then 6.8 g of crude product was pulped with ether for 2 h. The filter cake was collected after filtration and dried to obtain the pure product **023083A3RAC** (3.7 g). Then 3.7 g of pure **023083A3RAC** was further purified and separated by chiral HPLC (Chiral assay: SFC: IG (CHIRALPAK® 250mmL*4.6mm Particle Size: 5um)-Hex-EtOH-DEA-85-15-0.2; Flow Rate: 1.00 (ml /min); T: 30°C; wavelength: 254 nm; elution time: 15 min) to obtain two isomers (+)-**023083A3P1** (1.6 g), RT = 8.72 min and **(-)-023083A3P2** (1.5 g), RT = 10.44 min. LCMS (ESI) m/z = 370.1 $[M+H]^+$.

**Step 4: Synthesis of 023083A4P1 and 023083A4P2**

To a solution (5 mL) of compound **(+)-023083A3P1** (500 mg, 1.35 mmol) in acetonitrile was added N-bromosuccinimide (240 mg, 1.35 mmol) at room temperature and the reaction solution was stirred for 10 min at room temperature. The reaction solution was concentrated to remove the solvent and the residue was purified by silica gel column chromatography (ethyl acetate/petroleum ether = 1:8) to give **023083A4P1** (365 mg, 60.1%) as yellow oil. LCMS (ESI) m/z = 448.1 $[M+H]^+$.

To a solution (5 mL) of compound **(-)-023083A3P2** (500 mg, 1.35 mmol) in acetonitrile was added N-bromosuccinimide (240 mg, 1.35 mmol) at room temperature and the reaction solution was stirred for 10 min at room temperature. The reaction solution was concentrated to remove the solvent and the residue was purified by silica gel column chromatography (ethyl acetate/petroleum ether = 1:8) to give **023083A4P2** (360 mg, 59.7%) as yellow oil. LCMS (ESI) m/z = 448.1 $[M+H]^+$.

**Step 5: Synthesis of SZ-023084A and SZ-023084B**

To a solution (5 mL) of compound **023083A4P1** (100 mg, 0.224 mmol) in dichloromethane was added N,N-diisopropylethylamine (86 mg, 0.672 mmol) and **023029A9** (80 mg, 0.287 mmol) at room temperature and the reaction solution was stirred overnight at room temperature. The reaction solution was concentrated to remove the solvent and the residue was isolated and purified by preparative HPLC to give **SZ-023084A** (31 mg, 22.8%) as a yellow solid. Liquid Mass Spectrometry [Mobile phase: elution was maintained for 6 minutes at a flow rate of 1.5 ml per minute in a gradient from 80% water (with 0.02% ammonium acetate) and 20% acetonitrile to 40% water (with 0.02% ammonium acetate) and 60% acetonitrile at a column temperature of 40°C. Column: waters XBridge C18 5um, 50*4.6mm, purity 97.41%, Rt = 3.460 min; LCMS (ESI) m/z = 609.3 $[M+H]^+$.

$^1$HNMR ($CD_3OD$, 400MHz): δ 7.93 (d, *J* = 3.2 Hz, 1H), 7.73 (d, *J* = 3.2 Hz, 1H), 7.36-7.31 (m, 1H), 7.23 (d, *J* = 7.2 Hz, 1H), 7.04 (t, *J* = 8.8 Hz, 1H), 6.21 (s, 1H), 4.07-3.99 (m, 4H), 3.89-3.75 (m, 3H), 3.55 (t, *J* = 8.8 Hz , 1H), 3.41-3.38 (m, 1H), 3.29-3.26 (m, 1H), 3.18-3.11 (m, 2H), 2.97 (d, *J* = 8.0 Hz, 1H), 2.73 (d, *J* = 12.0 Hz, 1H), 2.77(d, *J* = 10.8 Hz, 1H), 2.21-2.15 (m, 1H), 1.19 (s, 3H), 1.18 (s, 3H), 1.11 (t, *J* = 7.2 Hz, 3H).

To a solution of compound **023083A4P2** (66 mg, 0.147 mmol) in dichloromethane (3 mL) at room temperature, compound **023029A9** (35.5 mg, 0.147 mmol) was added, followed by diisopropylethylamine (38 mg, 0.294 mmol). The reaction solution was stirred at room temperature for 4 hours. The reaction solution was concentrated to remove the solvent. The resulting residue was purified by preparative HPLC to give the yellow solid compound **SZ-023084B** (26.8 mg, 30% yield).

Liquid phase mass spectrometry [Mobile phase: elution was maintained for 6 minutes at a flow rate of 1.5 mL per minute on a gradient from 90% water (with 0. 02% ammonium acetate) and 10% acetonitrile to 40% water (with

0.1% trifluoroacetic acid) and 60% acetonitrile at a column temperature of 40°C. Column: XBridge C18 5um, 50*4.6mm. A purity of 97.75% at 214nm, Rt = 3.988 min. LCMS (ESI) m/z = 609.3 $[M+H]^+$.

$^1H$ NMR (DMSO-d6, 400 MHz): δ 9.62-9.58 (brs, 1H), 8.03 (d, *J* = 3.2 Hz, 1H), 7.94 (d, *J* = 3.2 Hz, 1H), 7.43-7.37 (m, 1H), 7. 23-7.17 (m, 2H), 6.09 (s, 1H), 4.65 (s, 1H), 3.96-3.81 (m, 4H), 3.72-3.61(m, 2H), 3.31-3.14 (m, 1H), 3.00-2.93 (m, 2H), 2.84-2.80 (m, 2H), 2.68-2.62 (m, 1H), 2.16-2.10 (m, 1H), 2.05-1.99 (m, 1H), 1.07(s, 3H), 1.06 (s, 3H), 1.03 (t, *J* = 7.2 Hz, 9H).

**Example 8**

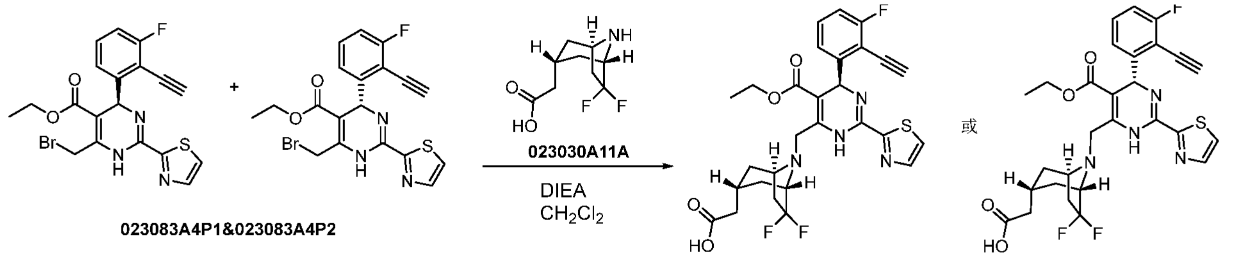

023083A4P1&023083A4P2

SZ-023085A&SZ-023085B

To a solution (6 mL) of compound **023083A4P1** (110 mg, 0.25 mmol) in dichloromethane was added N,N-diisopropylethylamine (0.5 mL) and **023030A11A** (83 mg, 0.34 mmol, prepared with reference to Patent CN105209470 B) at room temperature and the reaction solution was stirred overnight at room temperature. The reaction solution was concentrated to remove the solvent and the residue was isolated and purified by preparative HPLC to give **SZ-023085A** (32 mg, 22%) as a yellow solid. Liquid chromatography mass spectrometry [Mobile phase: elution was maintained for 6 min at a flow rate of 1.5 ml per minute in a gradient from 70% water (with 0.1% trifluoroacetic acid) and 30% acetonitrile to 30% water (with 0.1% trifluoroacetic acid) and 70% acetonitrile at a column temperature of 40°C. Column: Kinetex 5um EVO C18, 50*4.6 mm, purity 97.38%, Rt = 2.807 min; LCMS (ESI) m/z = 573.3 $[M+H]^+$.

$^1$HNMR (DMSO-d6, 400MHz): δ 9.76 (s, 1H), 7.97 (d, *J* = 3.2 Hz, 1H), 7.93 (d, *J* = 3.2 Hz, 1H), 7.42-7.36 (m, 1H), 7.24-7.16 (m, 2H), 6.09 (s, 1H), 4.65 (s, 1H), 4.31 (d, *J* = 16.4Hz, 1H), 3.97-3.89 (m, 3H), 3.41-3.37 (m, 2H), 2.67-2.58 (m, 1H), 2.39-2.10 (m, 5H), 1.57-1.53 (m, 1H), 1.27-1.23 (m, 2H), 1.04 (t, *J* = 7.2 Hz, 3H).

To a solution of compound **023083A4P2** (66 mg, 0.147 mmol) in dichloromethane (3 mL) at room temperature, compound **023030A11A** (30.2 mg, 0.147 mmol) was added, followed by diisopropylethylamine (38 mg, 0.294 mmol). The reaction solution was stirred at room temperature for 4 hours. The reaction solution was concentrated to remove the solvent. The resulting residue was purified by preparative HPLC to give compound **SZ-023085B** (18.5 mg, 22% yield) as a yellow solid.

Liquid Mass Spectrometry [Mobile phase: elution was maintained for 6 minutes at a flow rate of 1.5 mL per minute on a gradient from 80% water (with 0. 02% ammonium acetate) and 20% acetonitrile to 30% water (with 0.1% trifluoroacetic acid) and 70% acetonitrile at a column temperature of 40°C. Column: XBridge C18 5um , 50*4.6mm. A purity of 94.84% at 214nm, Rt = 3.753 min. LCMS (ESI) m/z = 573.2 $[M+H]^+$.

$^1H$ NMR (DMSO-d6, 400 MHz): δ 9.74 (brs, 1H), 7.98 (d, *J* = 3.2 Hz, 1H), 7.93 (d, *J*= 3.2 Hz, 1H), 7.43-7.37 (m, 1H), 7.25-7.16 (m, 2H), 6.09 (s, 1H), 4.64 (s, 1H), 4.19 (d, *J* = 16.8 Hz, 1H), 4.06 (d, *J* = 17.6 Hz, 1H), 3.94 (q, *J* = 7.2 Hz, 2H), 3.46-3.45 (m, 2H), 2.34-2.31 (m, 4H), 2.21-2.17 (m, 2H), 1.55-1.51 (m, 1H), 1.31-1.24 (m, 2H), 1.05 (t, *J* = 7.2 Hz, 3H).

**Example 9**

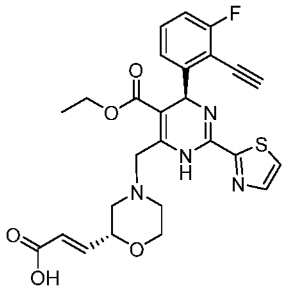

或

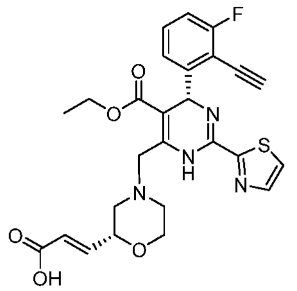

SZ-023088A&SZ-023088B

To a solution (5 mL) of compound **023083A4P1** (100 mg, 0.224 mmol) in dichloromethane was added N,N-diisopropylethylamine (86 mg, 0.672 mmol) and **023060A5** (52 mg, 0.33 mmol) at room temperature and the reaction solution was stirred overnight at room temperature. The reaction solution was stirred at room temperature overnight. The reaction solution was concentrated to remove the solvent and the residue was isolated and purified by preparative HPLC to give **SZ-023088A** (35 mg, 30.4%) as a yellow solid. Liquid chromatography mass spectrometry [Mobile phase: elution was maintained for 6 min at a flow rate of 1.5 ml per minute on a gradient from 80% water (with 0.1% trifluoroacetic acid) and 20% acetonitrile to 40% water (with 0.1% trifluoroacetic acid) and 60% acetonitrile at a column temperature of 40°C. Column: waters XBridge C18 5um, 50*4.6mm, purity 98.43% (E/Z mixture), Rt = 3.349 min; LCMS (ESI) m/z = 525.2 $[M+H]^+$.

$^1$HNMR ($CD_3OD$, 400MHz): δ 7.98 (d, *J* = 2.8 Hz, 1H), 7.76 (d, *J* = 2.8 Hz, 1H), 7.40-7.36 (m, 1H), 7.33-7.27 (m, 1H), 7.10-7.04 (m, 1H), 6.76 (dd, $J_1$ = 16.0 Hz, $J_2$ = 4.4 Hz, 0.8H), 6.24 (s, 1H), 6.12 (dd, $J_1$ = 16.0 Hz, $J_2$ = 2.0 Hz, 0.8H), 6.06-6.02 (m, 0.2H), 5.92-5.89 (m, 0.2H), 5.23-5.19 (m, 0.2H), 4.38-4.35 (m, 0.8H), 4.11-4.03 (m, 4H), 4.01-3.83 (m, 3H), 3.18-3.16 (m, 0.3H), 3.07-3.04 (m, 0.8H), 2.78-2.73 (m, 1H), 2.47-2.39 (m, 1H), 2.32-2.22 (m, 1H), 1.15 (t, *J* = 7.2 Hz, 3H).

To a dichloromethane solution (3 mL) of compound **023083A4P2** (65 mg, 0.145 mmol) was added N,N-diisopropylethylamine (46 mg, 0.29 mmol) and **023060A5** (35 mg, 0.22 mmol) at room temperature and the reaction solution was stirred overnight at room temperature. The reaction solution was concentrated to remove the solvent and the residue was isolated and purified by preparative HPLC to give **SZ-023088B** (20.3 mg, 26.7%) as a yellow solid. Liquid Mass Spectrometry [Mobile phase: elution was maintained for 6 minutes at a flow rate of 1.5 ml per minute in a gradient from 75% water (with 0.02% ammonium acetate) and 25% acetonitrile to 50% water (with 0.02% ammonium acetate) and 50% acetonitrile at a column temperature of 40°C. Column: waters XBridge C18 5um, 50*4.6mm, purity 97.06% (E/Z mixture), Rt = 3.504 min; LCMS (ESI) m/z = 525.2 $[M+H]^+$.

$^1$HNMR (DMSO-d6, 400MHz): δ 9.58 (s, 1H), 8.04-8.01 (m, 1H), 7.95-7.92 (m, 1H), 7.41-7.37 (m, 1H), 7.23-7.17 (m, 2H), 6.71 (dd, $J_1$ = 15.6 Hz, $J_2$ = 4.0 Hz, 0.8H), 6.10-6.07 (m, 1H), 5.92 (dd, $J_1$ = 15.6 Hz, $J_2$ = 1.6 Hz, 0.8H), 5.82-5.79 (m, 0.2H), 5.04-5.01 (m, 0.2H), 4.65 (s, 1H), 4.23-4.21 (m, 0.8H), 3.98-3.90 (m, 5H), 3.68-3.65 (m, 1H), 2.94 (d, *J* = 10.8Hz, 1H), 2.82 (d, *J* = 11.2Hz, 1H), 2.40-2.35 (m, 1H), 2.09-2.04 (m, 1H), 1.03 (t, *J* = 7.2 Hz, 3H).

**Example 10**

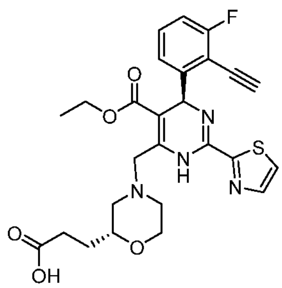

或

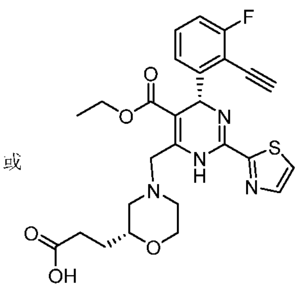

SZ-023090A&SZ-023090B

To a solution of compound **023083A4P1** (86.6 mg, 0.19 mmol) in dichloromethane (3 mL) at room temperature, compound **023060B3** (44 mg, 0.285 mmol) was added, followed by diisopropylethylamine (94 mg, 0.57 mmol). The reaction solution was stirred at room temperature for 4 hours. The reaction solution was concentrated to remove the

solvent. The resulting residue was purified by preparative HPLC to give the yellow solid compound **SZ-023090A** (52.8 mg, 52% yield).

Liquid phase mass spectrometry [Mobile phase: elution was maintained for 6 minutes at a flow rate of 1.5 mL per minute on a gradient from 95% water (with 0. 02% ammonium acetate) and 5% acetonitrile to 40% water (with 0.1% trifluoroacetic acid) and 60% acetonitrile at a column temperature of 40°C. Column: XBridge C18 5um , 50*4.6mm. A purity 99.72% at 214nm, Rt = 4.159 min. LCMS (ESI) m/z = 527.3 $[M+H]^+$.

$^1H$ NMR (DMSO-d6, 400 MHz): $\delta$ 9.63-9.60 (brs, 1H), 8.02 (d, *J* = 2.8 Hz, 1H), 7.94-7.93 (m, 1H), 7.40 (dd, $J_1$ = 6.0 Hz, $J_2$ = 8.0 Hz, 1H), 7.24-7.17 (m, 2H), 6.09 (s, 1H), 4.66 (s, 1H), 3.97-3.89 (m, 3H), 3.84-3.78 (m, 2H), 3.56-3.40 (m, 2H), 2.87 (d, *J* = 11.2 Hz, 1H), 2.68-2.60 (m, 1H), 2.44-2.30 (m, 4H), 2.17-2.12 (m, 1H), 1.73-1.68 (m, 2H), ,1.05 (t, *J* = 7.2 Hz, 3H).

To a solution of compound **023083A4P2** (65 mg, 0.145 mmol) in dichloromethane (3 mL) at room temperature, compound **023060B3** (46 mg, 0.29 mmol) was added, followed by diisopropylethylamine (38 mg, 0.294 mmol). The reaction solution was stirred at room temperature for 4 hours. The reaction solution was concentrated to remove the solvent. The resulting residue was purified by preparative HPLC to give **SZ-023090B** (43 mg, 56.6% yield) as a yellow solid.

Liquid phase mass spectrometry [Mobile phase: elution was maintained for 6 min at a flow rate of 1.5 mL per minute on a gradient from 80% water (with 0. 02% ammonium acetate) and 20% acetonitrile to 50% water (with 0.1% trifluoroacetic acid) and 50% acetonitrile at a column temperature of 40°C. Column: XBridge C18 5um , 50*4.6mm. A purity of 99.26% at 214nm, Rt = 3.717 min. LCMS (ESI) m/z = 527.2 $[M+H]^+$.

$^1H$ NMR ($CD_3OD$, 400 MHz): $\delta$ 7.94 (d, *J* = 3.2 Hz, 1H), 7.72 (d, *J* = 3.2 Hz, 1H), 7.36-7.31 (m, 1H), 7.22 (d, *J* = 7.2 Hz, 1H), 7.04 (t, *J* = 8.4 Hz, 1H), 6.21 (s, 1H), 4.05-3.91 (m, 5H), 3.80-3.66 (m, 2H), 2.86-2.80 (m, 2H), 2.50-2.35 (m, 3H), 2.17-2.12 (m, 1H), 1.74 (q, *J* = 7.2 Hz, 2H), 1.13 (t, *J* = 7.2 Hz, 3H).

**Example 11**

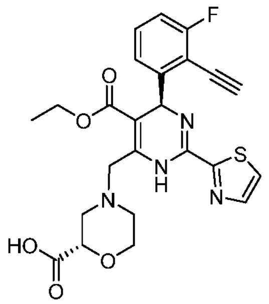
或
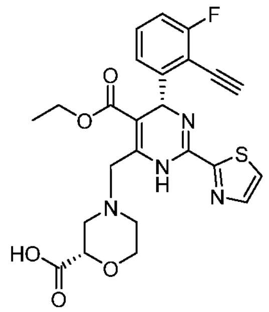

**SZ-023097A&SZ-023097B**

To a solution (5 mL) of compound **023083A4P1** (100 mg, 0.224 mmol) in dichloromethane was added N,N-diisopropylethylamine (86 mg, 0.672 mmol) and (S)-morpholine-2carboxylic acid hydrochloride (48 mg, 0.287 mmol) at room temperature and the reaction solution was stirred overnight at room temperature. The reaction solution was concentrated to remove the solvent and the residue was isolated and purified by preparative HPLC to give **SZ-023097A** (35 mg, 31.5%) as a yellow solid. Liquid chromatography mass spectrometry [Mobile phase: elution was maintained for 6 min at a flow rate of 1.5 ml per minute on a gradient from 90% water (with 0.1% trifluoroacetic acid) and 10% acetonitrile to 40% water (with 0.1% trifluoroacetic acid) and 60% acetonitrile at a column temperature of 40°C. Column: waters XBridge C18 5um, 50*4.6mm, purity 99.53%, Rt = 3.648 min; LCMS (ESI) m/z = 499.2 $[M+H]^+$.

$^1HNMR$ ($CD_3OD$, 400MHz): $\delta$ 7.97 (d, *J* = 2.8 Hz, 1H), 7.78 (d, *J* = 3.2 Hz, 1H), 7.42-7.36 (m, 1H), 7.31 (d, *J* = 7.6 Hz, 1H), 7.09 (t, *J* = 8.8 Hz, 1H), 6.24 (s, 1H), 4.24 (dd, $J_1$ = 10.0 Hz, $J_2$ = 2.0 Hz, 1H), 4.18-4.14 (m, 1H), 4.09-4.02 (m, 5H), 3.88-3.82 (m, 1H), 3.40-3.34 (m, 1H), 2.90-2.87 (m, 1H), 2.63-2.55 (m, 2H), 1.16 (t, *J* = 7.2 Hz, 3H).

To a solution of compound **023083A4P2** (65 mg, 0.145 mmol) in dichloromethane (3 mL) at room temperature, compound (S)-morpholine-2-carboxylic acid (20 mg, 0.145 mmol) was added followed by diisopropylethylamine (38 mg, 0.294 mmol). The reaction solution was stirred at room temperature for 4 hours. The reaction solution was concentrated to remove the solvent. The resulting residue was purified by preparative HPLC to give the yellow solid **SZ-023097B** (31 mg, 43% yield).

Liquid phase mass spectrometry [Mobile phase: elution was maintained for 6 minutes at a flow rate of 1.5 mL per minute on a gradient from 90% water (with 0. 02% ammonium acetate) and 10% acetonitrile to 40% water (with

0.1% trifluoroacetic acid) and 60% acetonitrile at a column temperature of 40°C. Column: XBridge C18 5um , 50*4.6mm. A purity of 97.72% at 214nm, Rt = 3.649 min. LCMS (ESI) m/z = 499.2[M+H]$^+$.

$^1$HNMR (DMSO-d6, 400 MHz): δ 9.57 (brs, 1H), 8.00 (d, *J* = 3.2 Hz, 1H), 7.93 (d, *J* = 3.2 Hz, 1H), 7.40-7.36 (m, 1H), 7.23-7.17 (m, 2H), 6.09 (s, 1H), 4.67 (s, 1H), 3.97-3.92 (m, 4H), 3.88(s, 2H), 3.60-3.54 (m, 1H), 2.87 (d, *J* = 6.0 Hz, 1H), 2.70-2.67 (m, 2H), 2.43-2.32 (m, 2H), 1.05 (t, *J* = 7.2 Hz, 3H).

**Example 12**

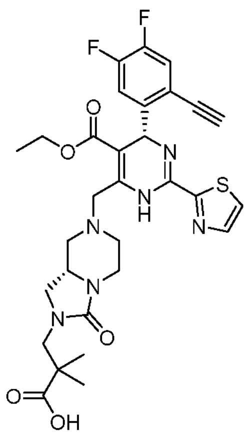

**SZ-023129**

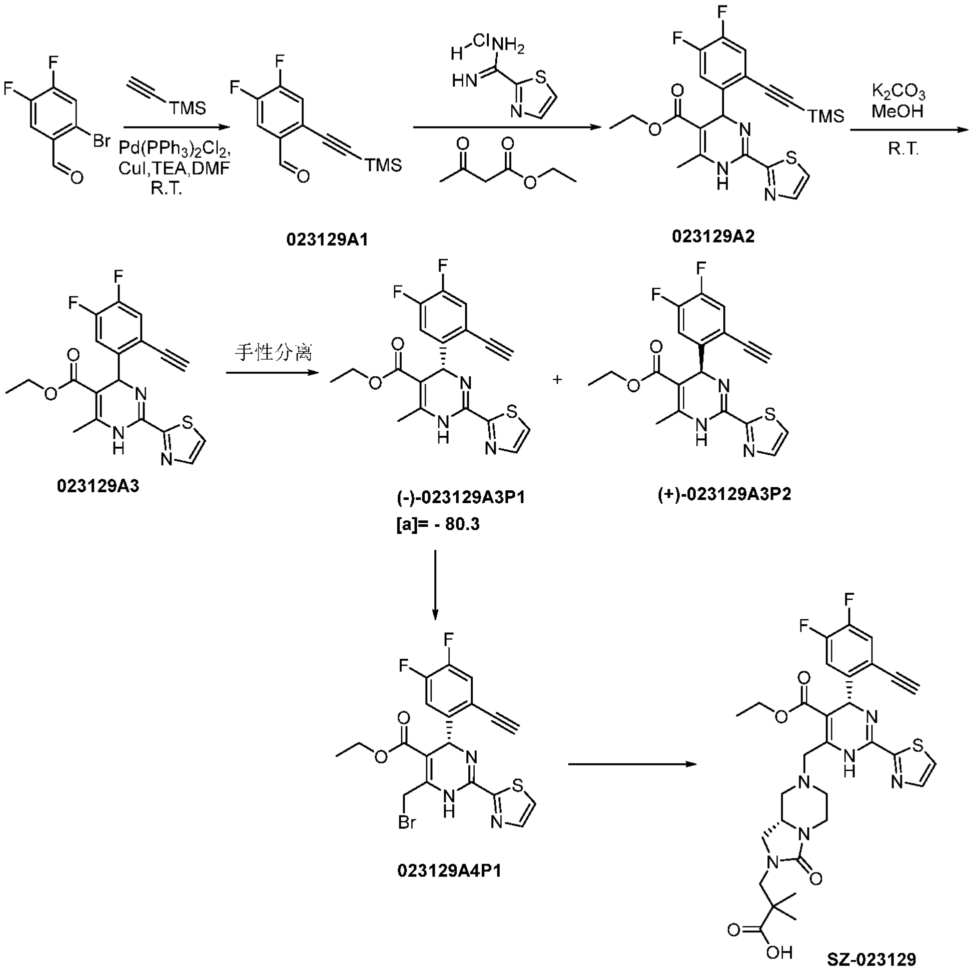


023129A1

023129A2

023129A3

(-)-023129A3P1
[a]= - 80.3

(+)-023129A3P2

023129A4P1

SZ-023129

**Step 1: Synthesis of 023129A1**

To 2-bromo-4,5-difluorobenzaldehyde (5.0 g, 22.6 mmol) in N,N,-dimethylformamide (60 mL) at room temperature were added trimethylethynylsilyl (6.64 g, 67.8 mmol), triethylamine (3.43 g, 33.9 mmol), bis(triphenylphosphine)palladium dichloride (793 mg, 1.13 mmol), and cuprous iodide (422 mg, 2.22 mmol). The reaction solution was stirred overnight at room temperature under nitrogen protection. After the reaction was completed, water (200 mL) and ethyl acetate (30 mL) were added, then filtered and the filtrate was extracted by ethyl acetate (3×100 mL). The organic phases were combined, the solvents were removed by concentration and the resulting residue was purified by silica gel column chromatography (ethyl acetate/petroleum ether = 1:10) to give **023129A1** (23.1 g, 89%) as a brown oil.

$^1$H NMR (DMSO-d6, 400 MHz): δ 10.26 (d, *J* = 3.2 Hz, 1H), 7.84 (m, 2H), 0.24 (s, 9H).

**Step 2: Synthesis of 023129A2**

To a solution of 4,5-difluoro-2-((trimethylsilyl)ethynyl)benzaldehyde **023129A1** (1.2 g, 5.0 mmol) in ethanol (20 mL) was added 2-thiazolecarboxamide hydrochloride (902 mg, 5.5 mmol), ethyl acetoacetate (715 mg, 5.5 mmol) and sodium acetate (615 mg, 7.5 mmol) at room temperature, and the reaction solution was stirred at 80°C overnight. The reaction solution was concentrated to remove the solvent and the resulting residue was purified by silica gel column chromatography (ethyl acetate/petroleum ether = 1:7) to give **023129A2** (1.75 g, 75.7%) as a yellow oil. LCMS (ESI) m/z = 460.1 $[M+H]^+$.

**Step 3: Synthesis of 023129A3**

To a solution of **023129A2** (1.75 g, 3.8 mmol) in methanol (30 mL) at room temperature was added potassium carbonate (420 mg, 3.0 mmol) and the reaction solution was stirred overnight at room temperature. After the reaction was completed, the reaction solution was concentrated to remove the solvent. The residue was extracted with water (100 mL), ethyl acetate (3×50 mL). The organic phases were combined and concentrated. The residue was purified by silica gel column chromatography (ethyl acetate/petroleum ether = 1:6) to give **023129A3** (1.4 g, 95%) as a pale yellow solid. LCMS (ESI) m/z = 388.1 $[M+H]^+$.

$^1$H NMR ($CDCl_3$, 400 MHz): δ 7.81 (d, *J* = 3.2 Hz, 1H), 7.44 (d, *J* = 3.2 Hz, 1H), 7.33-7.28 (m, 1H), 7.18-7.14 (m, 1H), 6.20 (s, 1H), 4.05 (q, *J* = 7.2 Hz, 2H), 3.30 (s, 1H), 2.50 (s, 3H), 1.15 (t, *J* = 7.2 Hz, 3H)

The pure product of this racemate **023129A3** (500 mg) was further purified and separated by chiral HPLC (Chiral method: SFC: IB N5 (CHIRALPAK® 250mmL*4.6mm Particle Size: 5um) -Hex-EtOH-90-10-30min; Flow Rate: 1.00 (ml/min); T: 30°C; wavelength: 254 nm; elution time: 30 min) to obtain two stereoisomers **(-)-023129A3P1** (200 mg) RT = 9.490 min and **(+)-023129A3P2** (200 mg) RT = 11.924 min.

**Step 4: Synthesis of 023129A4P1**

To a solution (5 mL) of compound **(-)-023129A3P1** (100 mg, 0.258 mmol) in acetonitrile was added N-bromosuccinimide (46 mg, 0.258 mmol) at room temperature and the reaction solution was stirred for 10 min at room temperature. The reaction solution was concentrated to remove the solvent and the residue was purified by silica gel column chromatography (ethyl acetate/petroleum ether = 1:7) to give **023129A4P1** (70 mg, 58.3%) as yellow oil. LCMS (ESI) m/z = 466.1 $[M+H]^+$.

**Step 5: Synthesis of SZ-023129**

To a solution (5 mL) of compound **023129A4P1** (70 mg, 0.15 mmol) in dichloromethane was added N,N-diisopropylethylamine (39 mg, 0.30 mmol) and **023029A9** (54 mg, 0.196 mmol) at room temperature and the reaction solution was stirred overnight at room temperature. The reaction solution was concentrated to remove the solvent and the residue was isolated and purified by preparative HPLC to give **SZ-023129** (30 mg, 32%) as a yellow solid. Liquid Mass Spectrometry [Mobile phase: elution was maintained for 6 minutes at a flow rate of 1.5 ml per minute in a gradient from 95% water (with 0.02% ammonium acetate) and 5% acetonitrile to 5% water (with 0.02% ammonium acetate) and 95% acetonitrile at a column temperature of 40°C. Column: waters XBridge C18 5um, 50*4.6mm, purity 99.85%, Rt = 3.408 min; LCMS (ESI) m/z = 627.3 $[M+H]^+$.

$^1$HNMR ($CD_3OD$, 400MHz): δ 7.94 (d, *J* = 3.2 Hz, 1H), 7.73 (d, *J* = 3.2 Hz, 1H), 7.43-7.38 (m, 1H), 7.27-7.22 (m, 1H), 6.17 (s, 1H), 4.08-4.00 (m, 3H), 3.88-3.83 (m, 4H), 3.48 (t, *J* = 8.8 Hz , 1H), 3.40-3.36 (m, 1H), 3.30-3.26 (m, 1H), 3.18 (td, $J_1$ = 12.8 Hz, $J_2$ = 3.2 Hz, 1H), 3.08 (dd, $J_1$ = 10.0 Hz, $J_2$ = 4.4 Hz, 1H), 2.90 (d, *J* = 10.4 Hz, 1H), 2.80 (d, *J* = 10.4 Hz, 1H), 2.33 (td, $J_1$ = 11.6 Hz, $J_2$ = 3.2 Hz, 1H), 2.14 (t, *J* = 10.8 Hz, 1H), 1.18 (s, 3H), 1.17 (s, 3H), 1.13 (t, *J* = 7.2 Hz, 3H).

**Example 13**

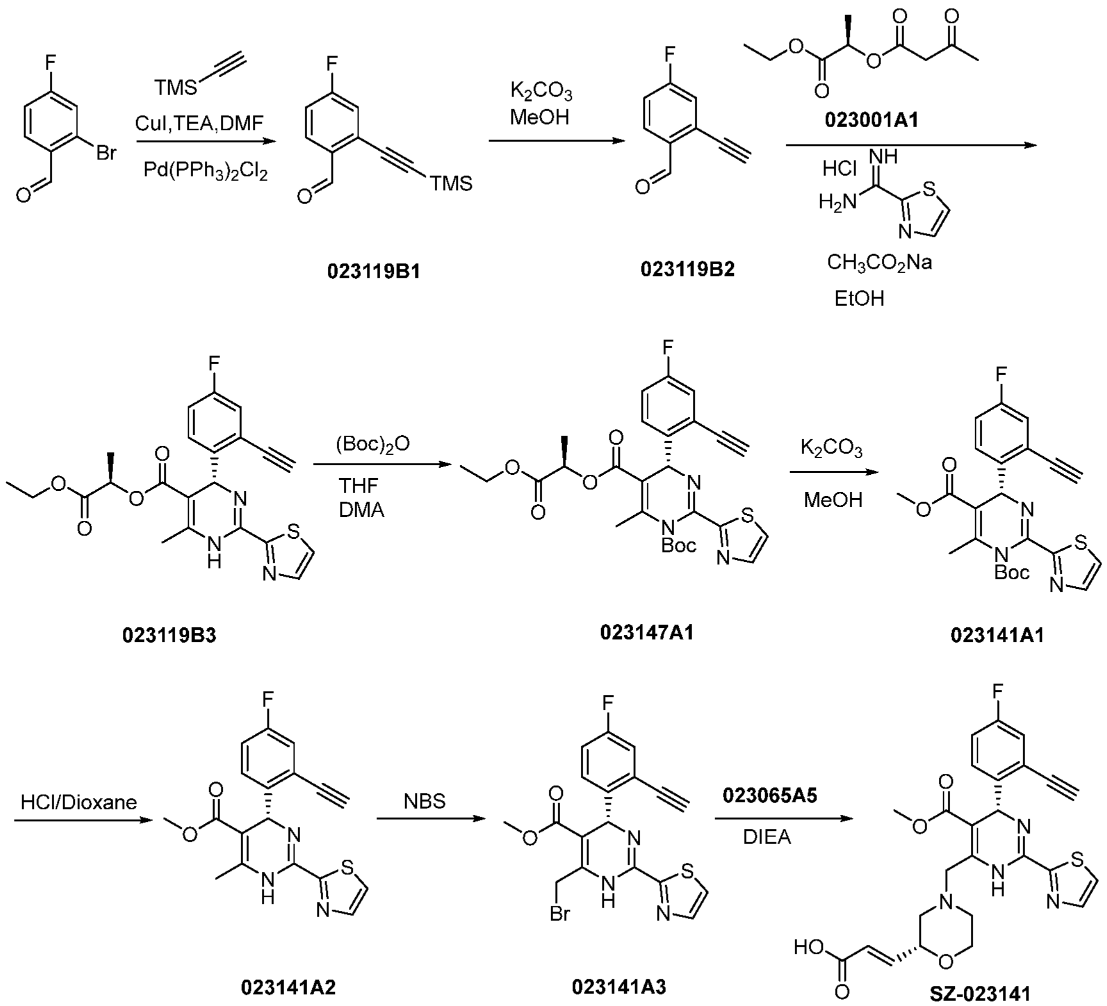


023119B1 023119B2 023119B3 023147A1 023141A1 023141A2 023141A3 SZ-023141

**Step 1: Synthesis of 023119B1**

To 2-bromo-4-fluorobenzaldehyde (25.0 g, 123 mmol) in N,N,-dimethylformamide (250 mL) at room temperature were added trimethylethynylsilyl (36.0 g, 369 mmol), triethylamine (18.6 g, 184.5 mmol), bis(triphenylphosphine)palladium dichloride (4.3 g, 6.15 mmol) and cuprous iodide (2.34 g, 12.3 mmol). The reaction solution was stirred overnight at room temperature under nitrogen protection. After the reaction was completed, it was filtered and washed with ethyl acetate. Water (500 mL) and ethyl acetate (300 mL) were added to the filtrate, the organic layer was separated and extracted with ethyl acetate. The organic phases were combined, the solvents were removed by concentration and the resulting residue was purified by silica gel column chromatography (EA/PE = 5%-10%) to give **023119B1** (22 g, 80%) as a pale yellow solid.

**Step 2: Synthesis of 023119B2**

To a solution of **023119B1** (22.0 g, 98.7 mmol) in methanol (500 mL) was added potassium carbonate (9.8 g, 71.5 mmol) at room temperature and the reaction solution was stirred at room temperature overnight. After the reaction was completed, the reaction solution was filtered and concentrated to remove the solvent. The residue was extracted with water (100 mL), ethyl acetate (3×50 mL). The organic phases were combined and concentrated. The residue was separated and purified by silica gel column chromatography (EA/PE = 10%) to give **023119B2** (14.5 g, 96%) as a yellow solid.

$^1$HNMR (DMSO-d6, 400MHz): δ 10.34 (s, 1H), 7.98-7.93 (m, 1H), 7.64-7.61(m, 1H), 7.59-7.47(m, 1H), 4.88(s, 1H).

**Step 3: Synthesis of 023119B3**

To a solution of compound **023119B2** (14 g, 95.23 mmol) in ethanol (300 mL) was added 2-thiazolecarboxamidine hydrochloride (15.6 g, 95.23 mmol), **023001A1** (19.23 g, 95.23 mmol), and sodium acetate (8.6 g, 104.75 mmol) at room temperature and the reaction solution was stirred overnight at 80°C. The reaction solution was poured into water, extracted with ethyl acetate, dried, and concentrated to remove the solvent, and the resulting residue was separated and purified by silica gel column chromatography (EA/PE = 25%-30%) to give **023119B3RAC** 22 g as a pale yellow oil. It was then ether pulped for 2 h. The filter cake was collected by filtration and dried to give the pure product 023119B3 (6.2 g, 15%). ([a] = -154.6, c = 0.3 g/100 mL, MeOH, T = 19.7°C). LCMS (ESI) m/z = 442.1 $(M+H)^+$.

**Step 4: Synthesis of 023147A1**

To a solution (30 mL) of compound **023119B3** (3.0 g, 6.787 mmol) in tetrahydrofuran was added 4-dimethylaminopyridine (82 mg, 0.68 mmol) and di-tert-butyl dicarbonate (1.8 g, 8.136 mmol), and the reaction solution was stirred at room temperature overnight. The reaction solution was concentrated to remove the solvent and the residue was purified by silica gel column chromatography (ethyl acetate/petroleum ether = 1:8) to give **023147A1** (3.7 g, 100 %) as a pale yellow solid. LCMS (ESI) m/z = 542.1 $(M+H)^+$.

**Step 5: Synthesis of 023141A1**

To a solution of **023147A1** (1.0 g, 1.74 mmol) in methanol (10 mL) was added potassium carbonate (0.509 g, 3.69 mmol) at room temperature, and the reaction solution was stirred overnight at room temperature. After the reaction was completed, the residue was extracted with water (100 mL), ethyl acetate (3×50 mL). The organic phases were combined and concentrated. The residue was separated and purified by silica gel column chromatography (EA/PE = 10%) to give 023141A1 (820 mg, 95%) as a pale yellow solid. LCMS (ESI) m/z = 456.1 $(M+H)^+$.

**Step 6: Synthesis of 023141A2**

A solution of **023141A1** (780 mg, 1.71 mmol) in 4N hydrochloric acid/dioxane (10 mL) was stirred overnight at room temperature. After the reaction was completed, the solvent was removed by vacuum concentration, the residue was added with water, the pH was adjusted to neutral with saturated sodium bicarbonate solution, and extracted with ethyl acetate. The organic phases were combined and concentrated to give **023141A2** (500 mg, 82%) as a pale yellow solid. LCMS (ESI) m/z = 356.1 $(M+H)^+$.

**Step 7: Synthesis of 023141A3**

To a solution (5 mL) of compound **023141A2** (150 mg,0.42 mmol) in acetonitrile was added N-bromosuccinimide (75 mg, 0.42 mmol) at room temperature and the reaction solution was stirred at room temperature for 30 min to give a solution of **023141A3.** This reaction solution was used directly in the next step without treatment. LCMS (ESI) m/z = 434.0 $(M+H)^+$.

**Step 8: Synthesis of SZ-023141**

To this solution was added **023065A5** (172 mg, 0.86 mmol), N,N-diisopropylethylamine (271 mg, 2.1 mmol), and the reaction was stirred at room temperature for 2 hours. After the reaction was completed, the solvent was removed by vacuum concentration and the residue was isolated and purified by preparative HPLC to give **SZ-023141** (22.4 mg, 10%) as a yellow solid. Liquid Mass Spectrometry [Mobile phase: elution was maintained for 6 minutes at a flow rate of 1.5 ml per minute in a gradient from 75% water (with 0.02% ammonium acetate) and 25% acetonitrile to 50% water (with 0.02% ammonium acetate) and 50% acetonitrile at a column temperature of 40°C. Column: waters XBridge C18 5um, 50*4.6mm, purity 93.7 %, Rt = 3.212 min; LCMS (ESI) m/z = 511.2$(M+H)^+$.

$^1$HNMR (DMSO-d6, 400MHz): δ 9.62 (s, 1H), 8.01 (t, *J* = 3.2 Hz, 1H), 7.93 (d, *J* = 3.2 Hz, 1H), 7.38-7.30 (m, 2H), 7.23-7.18 (m, 1H), 6.71 (dd, $J_1$ = 16.0 Hz , $J_2$ = 4.4 Hz, 1H), 6.08 (s, 1H), 5.92 (dd, $J_1$ = 16.0 Hz , $J_2$ = 1.6 Hz, 1H),4.47 (s, 1H),4.23-4.21(m, 1H), 3.98-3.84 (m, 3H), 3.70-3.65(m, 1H), 3.54 (s, 3H), 2.93(d, *J* = 11.2 Hz, 1H), 2.82 (d, *J* = 10.8 Hz, 1H), 2.41-2.33 (m, 1H), 2.07 (t, *J* = 10.8 Hz, 1H).

**Example 14**

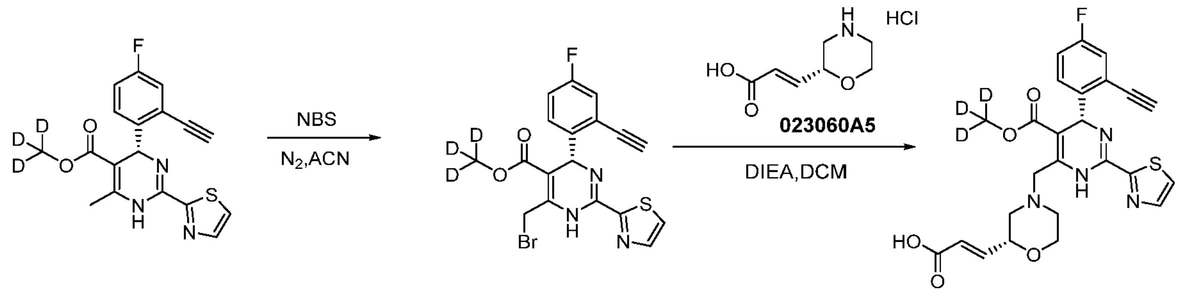


023147A4 023147A5 SZ-023143

**Step 1: Synthesis of 023147A5**

To a solution of compound **023147A4** (280 mg, 0.78 mmol) in acetonitrile (5 mL) at room temperature, displaced three times with nitrogen, was added a solution of N-bromosuccinimide (140 mg, 0.78 mmol) in acetonitrile and the reaction was carried out for 20 min at 38°C under $N_2$ protection. The reaction solution was concentrated and the residue was purified by silica gel column chromatography (EA/PE = 1:10) to give the pale yellow colloidal substance **023147A5** (300 mg, 87.8% yield). LCMS (ESI) m/z = 437.0 $(M+H)^+$.

**Step 2: Synthesis of SZ-023143**

To a solution of compound **023147A5** (150 mg, 0.34 mmol) in dichloromethane (3 mL) at room temperature, compound **023060A5** (67 mg, 0.35 mmol) was added, followed by diisopropylethylamine (133 mg, 1.03 mmol). The reaction solution was stirred at room temperature for 4 hours. The reaction solution was concentrated to remove the solvent. The resulting residue was purified by preparative HPLC to give the yellow solid compound **SZ-023143** (18 mg, 4.1% yield). Liquid Mass Spectrometry [Mobile phase: elution was maintained for 6 minutes at a flow rate of 1.5 mL per minute on a gradient from 85% water (with 0.02% ammonium acetate) and 15% acetonitrile to 40% water (with 0.02% ammonium acetate) and 60% acetonitrile at a column temperature of 40°C. Column: Xbridge C18, 5um 50*4.6mm. A purity of 97.45% at 214nm, Rt = 3.627min. LCMS (ESI) m/z = 514.2 $(M+H)^+$.

$^1$H NMR (CDsOD, 400 MHz): 7.94 (d, $J$ = 3.2 Hz, 1H), 7.74 (d, $J$ = 3.2 Hz, 1H), 7.40 (dd, $J_1$ = 8.8 Hz, $J_2$ = 5.6 Hz, 1H), 7.21 (dd, $J_1$ = 9.2 Hz, $J_2$ = 2.8 Hz, 1H), 7.08 (td, $J_1$ = 8.8 Hz, $J_2$ = 2.8 Hz, 1H), 6.79 (dd, $J_1$ = 15.6 Hz, $J_2$ = 4.0 Hz, 1H), 6.19 (s, 1H), 6.03 (dd, $J_1$ = 15.6 Hz, $J_2$ = 4.0 Hz, 1H), 4.38-4.34 (m, 1H), 4.04-4.00 (m, 2H), 3.92-3.83 (m, 3H), 2.94-2.86 (m, 2H), 2.56-2.54 (m, 1H), 2.19 (t, $J$ = 10.4 Hz, 1H).

**Example 15**

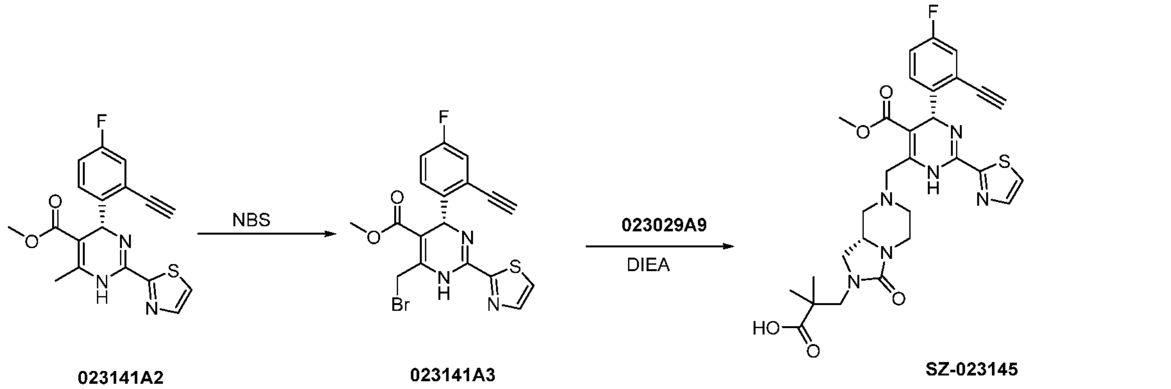


023141A2 023141A3 SZ-023145

**Step 1: Synthesis of 023141A3**

To a solution (5 mL) of compound 023141A2 (150 mg,0.42 mmol) in acetonitrile was added N-bromosuccinimide (75 mg, 0.42 mmol) at room temperature and the reaction solution was stirred at room temperature for 30 min to give 023141A3 reaction solution which was used directly in the next step.

**Step 2: SZ-023145**

**023029A9** (400 mg, 0.84 mmol), N,N-diisopropylethylamine (271 mg, 2.1 mmol) were added directly to the above solution and the reaction was stirred for 1 hour at room temperature. After the reaction was completed, the solvent was removed by vacuum concentration and the residue was isolated and purified by preparative HPLC to give **SZ-023145** (31.7 mg, 12%) as a yellow solid. [Mobile phase: elution was maintained for 6 minutes at a flow rate of 1.5 ml per minute in a gradient from 75% water (with 0.02% ammonium acetate) and 25% acetonitrile to 50% water (with 0.02% ammonium acetate) and 50% acetonitrile at a column temperature of 40°C. Column: waters XBridge C18 5um, 50*4.6mm, purity 99.4 %, Rt = 3.139 min; LCMS (ESI) m/z = 595.3 $(M+H)^+$.

$^1$HNMR (DMSO-d6, 400MHz): δ 9.63 (brs, 1H), 8.03 (d, *J* = 3.2 Hz, 1H), 7.93 (d, *J* = 3.2 Hz, 1H), 7.38-7.30 (m, 2H), 7.20 (td, $J_1$ = 8.8 Hz, $J_2$ = 2.8 Hz, 1H), 6.07 (s, 1H), 4.48 (s, 1H), 3.72-3.62 (m, 2H), 3.69-3.64 (m, 2H), 3.53 (s, 3H), 3.39-3.14 (m, 3H),3.17-2.77 (m, 4H), 2.17-2.16 (m, 1H), 2.02(d, *J* = 10.8 Hz, 1H), 1.07 (s, 3H), 1.04 (s, 3H).

**Example 16**

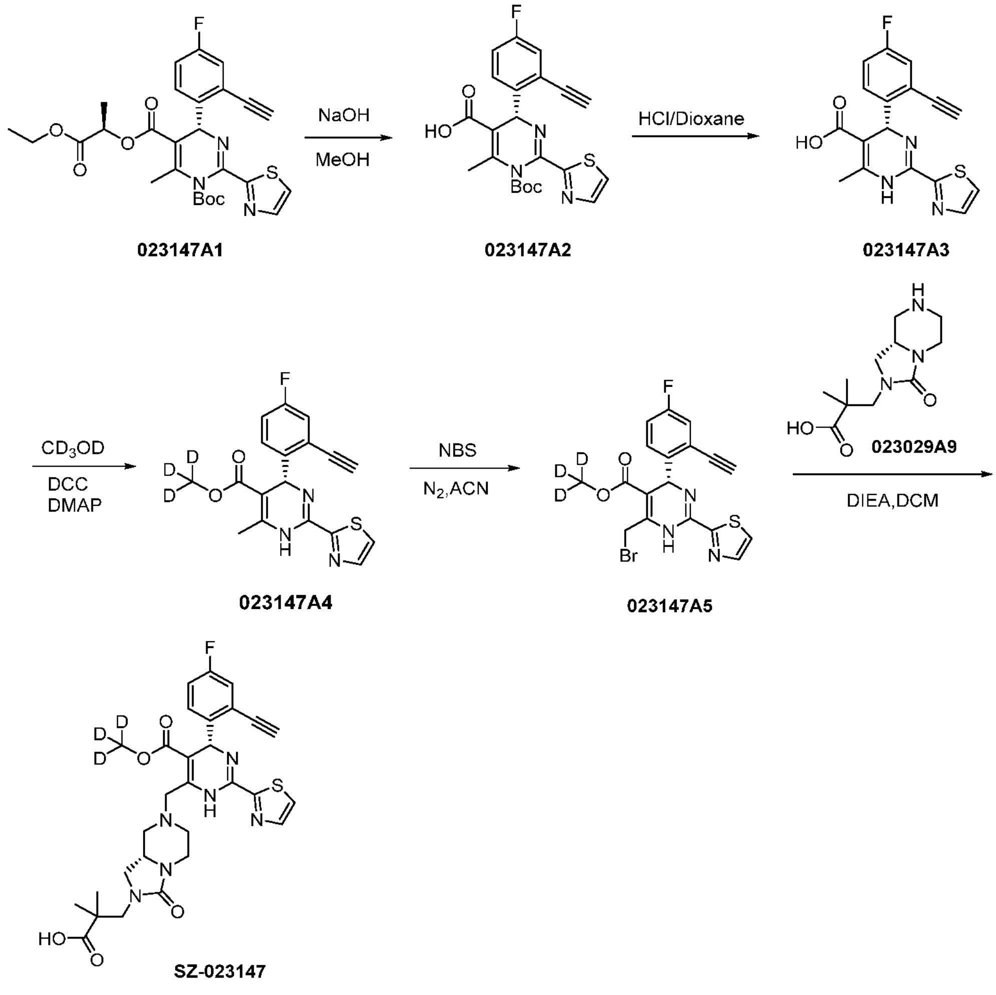


**023147A1** **023147A2** **023147A3** **023147A4** **023147A5** **023029A9** **SZ-023147**

**Step 1: Synthesis of 023147A2**

**023147A1** (900 mg,1.66 mmol) was dissolved in methanol (10 mL) and added dropwise to a solution of sodium hydroxide (664 mg, 16.6 mmol) in water (5 mL). The reaction was carried out overnight at 30°C. The methanol solvent was removed by spinning and evaporation, the remaining aqueous phase was extracted with ether (5 mL), the pH of the remaining aqueous phase was adjusted to 5 with 2N hydrochloric acid solution, extracted with ethyl acetate (2*20 mL), the organic phase was washed with water and brine, dried with magnesium sulphate, and the 600 mg of orange solid **023147A2** obtained by concentration was used for the next step of the reaction directly. LCMS (ESI) m/z = 442.2

$(M+H)^+$.

**Step 2: Synthesis of 023147A3**

**023147A2** was added to 4M hydrochloric acid/dioxane solution (40 mL), stirred overnight, the solvent was removed by spinning evaporation, ethyl acetate (50 mL) was added to the residue, washed with water (50 mL) and brine (50 mL), dried over magnesium sulfate, and the residue obtained by concentration was purified by silica gel column chromatography (EA/PE = 1:1) to obtain 023147A3 (400 mg, 86% yield) as a light yellow solid. LCMS (ESI) m/z = 342.1 $(M+H)^+$.

**Step 3: Synthesis of 023147A4**

To a solution of compound **023147A3** (400 mg, 1.17 mmol) in dichloromethane (8 mL) at room temperature was added a solution of deuterated methanol (49 mg, 1.4 mmol) in dichloromethane, followed by the addition of N,N-dimethylaminopyridine (143 mg, 1.17 mmol) and DCC (290 mg, 1.4 mmol) at 15°C. The reaction was carried out for 60 min at 15°C. The reaction was carried out for 60 min at 15°C. The reaction solution was concentrated and the residue was purified by silica gel column chromatography (EA/PE = 1:10) to give 023147A4 (200 mg, 48% yield) as a yellow solid. LCMS (ESI) m/z = 359.1 $(M+H)^+$.

**Step 4: Synthesis of 023147A5**

To a solution of compound **023147A4** (140 mg, 0.39 mmol) in acetonitrile (5 mL) at room temperature, displaced three times with nitrogen, was added a solution of N-bromosuccinimide (70 mg, 0.39 mmol) in acetonitrile and the reaction was carried out for 20 min at 38°C under $N_2$ protection. The reaction solution was concentrated and the residue was purified by silica gel column chromatography (EA/PE = 1:10) to give the pale yellow colloidal substance **023147A5** (150 mg, 87.8% yield). LCMS (ESI) m/z = 437.0 $(M+H)^+$.

**Step 5: Synthesis of SZ-023147**

To a solution of compound **023147A5** (150 mg, 0.34 mmol) in dichloromethane (3 mL) at room temperature, compound **023029A9** (96 mg, 0.35 mmol) was added, followed by diisopropylethylamine (133 mg, 1.03 mmol). The reaction solution was stirred at room temperature for 4 hours. The reaction solution was concentrated to remove the solvent. The resulting residue was purified by preparative HPLC to give the yellow solid compound **SZ-023147** (66 mg, 32.2% yield). Liquid Mass Spectrometry [Mobile phase: elution was maintained for 6 minutes at a flow rate of 1.5 mL per minute on a gradient from 85% water (with 0.02% ammonium acetate) and 15% acetonitrile to 40% water (with 0.02% ammonium acetate) and 60% acetonitrile at a column temperature of 40°C. Column: Xbridge C18 ,5um 50*4.6mm. A of purity 99.23% at 214nm, Rt = 3.653min. LCMS (ESI) m/z = 598.3 $(M+H)^+$.

$^1$H NMR (DMSO-d6, 400 MHz): 9.63 (brs, 1H), 8.02 (d, $J$ = 3.2 Hz, 1H), 7.93 (d, $J$ = 3.2 Hz, 1H), 7.38-7.30 (m, 2H), 7.20 (td, $J_1$ = 8.4 Hz, $J_2$ = 2.8 Hz, 1H), 6.07 (s, 1H), 4.47 (s, 1H), 3.94-3.81 (m, 2H), 3.72-3.68 (m, 1H), 3.63-3.61 (m, 1H), 3.50-3.45 (m, 1H), 3.26-3.23 (m, 1H), 3.16-3.12 (m, 1H), 3.01-2.94 (m, 2H), 2.83-2.76 (m, 2H), 2.14 (td, $J_1$ = 11.2 Hz, $J_2$ = 3.2 Hz, 1H), 2.02 (t, $J$ = 11.2 Hz, 1H), 1.05 (s, 6H).

**Example 17**

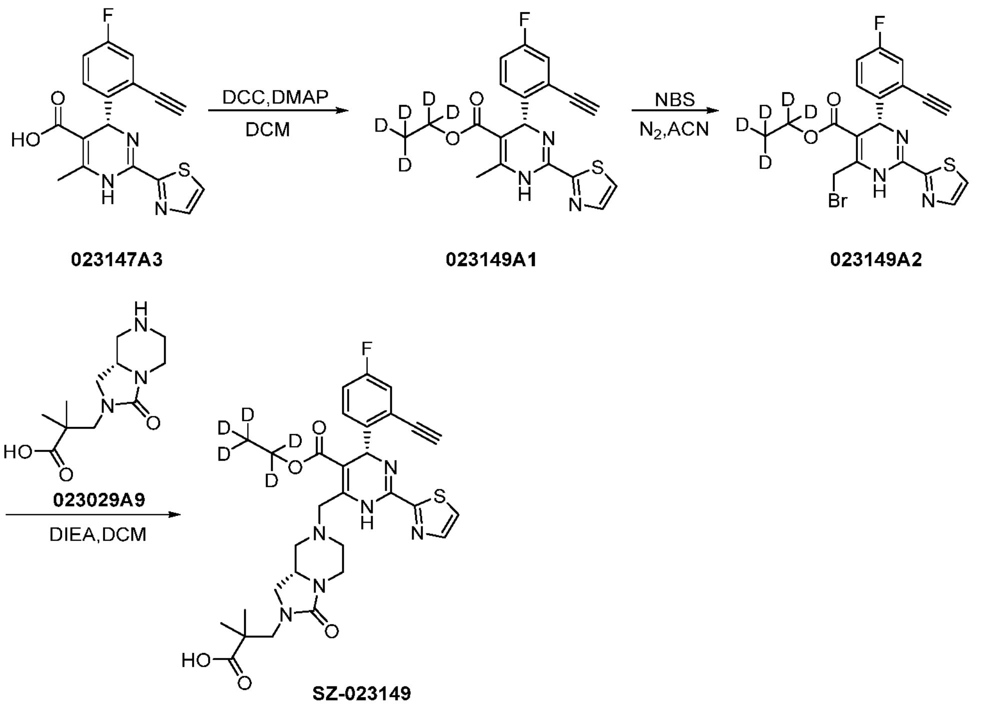


023147A3 023149A1 023149A2

SZ-023149

**Step 1: Synthesis of 023149A1**

To a solution of compound **023147A3** (160 mg, 0.47 mmol) in dichloromethane (3 mL) at room temperature was added a solution of deuterated ethanol (31 mg, 0.59 mmol) in dichloromethane, followed by the addition of N,N-dimethylaminopyridine (60 mg, 0.49 mmol) and DCC (122 mg, 0.59 mmol) at 15°C. The reaction was carried out for 60 min at 15°C. The reaction was carried out for 60 min at 15°C. The reaction solution was concentrated and the residue was purified by silica gel column chromatography (EA/PE = 1:10) to give **023149A1** (70 mg, 39.9% yield) as a yellow solid. LCMS (ESI) m/z = 375.2 $(M+H)^+$.

**Step 2: Synthesis of 023149A2**

To a solution of compound **023149A1** (70 mg, 0.19 mmol) in acetonitrile (2 mL) was added an acetonitrile solution of N-bromosuccinimide (34 mg, 0.19 mmol) at room temperature, and the reaction was carried out for 20 min at 38°C under $N_2$ protection. The reaction solution was concentrated and the residue was purified by silica gel column chromatography (EA/PE = 1:10) to give **023149A2** (60 mg, 69.6% yield) as a pale yellow solid. LCMS (ESI) m/z = 453.0 $(M+H)^+$.

**Step 3: Synthesis of SZ-023149**

To a solution of compound **023149A2** (60 mg, 0.13 mmol) in dichloromethane (3 mL) at room temperature, compound **023029A9** (37 mg, 0.13 mmol) was added, followed by diisopropylethylamine (52 mg, 0.40 mmol). The reaction solution was stirred at room temperature for 4 hours. The reaction solution was concentrated to remove the solvent. The resulting residue was purified by preparative HPLC to give the yellow solid compound **SZ-023149** (20 mg, 24.5% yield). Liquid Mass Spectrometry [Mobile phase: elution was maintained for 6 minutes at a flow rate of 1.5 mL per minute on a gradient from 80% water (with 0.02% ammonium acetate) and 20% acetonitrile to 30% water (with 0.02% ammonium acetate) and 70% acetonitrile at a column temperature of 40°C. Column: Xbridge C18 ,5um 50*4.6mm. A purity of 94.72% at 214nm, Rt = 3.287min. LCMS (ESI) m/z = 614.3 $(M+H)^+$.

$^1$H NMR ($CD_3OD$, 400 MHz): 7.94 (d, $J$ = 3.2 Hz, 1H), 7.73 (d, $J$ = 3.2 Hz, 1H), 7.41 (dd, $J_1$ = 8.8 Hz, $J_2$ = 2.0 Hz, 1H), 7.20 (dd, $J_1$ = 9.2 Hz, $J_2$ = 2.8 Hz, 1H), 7.08 (td, $J_1$ = 8.4 Hz, $J_2$ = 2.8 Hz, 1H), 6.19 (s, 1H), 4.06-4.01 (m, 1H), 3.87-3.80 (m, 4H), 3.51-3.47 (m, 1H), 3.40-3.37 (m, 1H), 3.30-3.27 (m, 1H), 3.21-3.14 (m, 1H), 3.10-3.07 (m, 1H), 2.91-2.80 (m, 2H), 2.35-2.29 (m, 1H), 2.15 (t, $J$ = 10.8 Hz, 1H), 1.16 (s, 6H).

**Example 18**

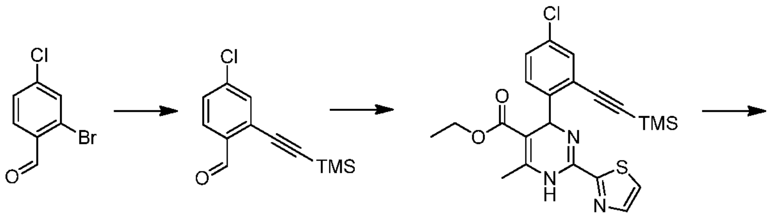

023366A1 023366A2

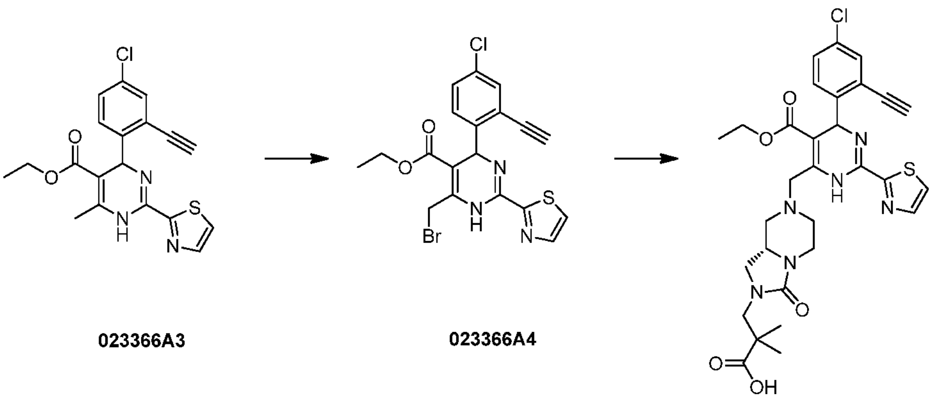

023366A3 023366A4

SZ-023366

**Step 1: Synthesis of 023366A1**

A solution of 4-chloro-2-bromobenzaldehyde (4 g, 18.3 mmol), trimethylethynylsilyl (3.2 mL, 18.3 mmol), triphenylphosphine palladium(II) dichloride (256 mg, 0.36 mmol), copper(II) iodide (140 mg, 0.73 mmol), triethylamine (3 g, 29.7 mmol), and tetrahydrofuran (20 ml) was stirred at room temperature under nitrogen protection. At the end of the reaction, the filtrate was filtered, the filtrate was added with water, extracted with dichloromethane, partitioned, the organic phase was separated, concentrated under reduced pressure and prepared at medium pressure to give **023366A1** (4.2 g, 97.0%), LCMS (ESI) m/z = 236 $[M+H]^+$.

**Step 2: Synthesis of 023366A2**

**023366A1** (1 g, 4.2 mmol), 2-thiazolecarboxamidine hydrochloride (0.7 g, 4.2 mmol), ethyl acetoacetate (0.6 g, 4.6 mmol), sodium acetate (0.4 g, 5.0 mmol), 20 ml of ethanol were reacted at 80°C overnight. The solvent was evaporated under medium pressure to give 023366A2 (1.6 g, 82.6%), LCMS (ESI) m/z = 457 $[M+H]^+$.

**Step 3: Synthesis of 023366A3**

**023366A2** (1.2 g, 2.63 mmol), potassium carbonate (2 g, 14.4 mmol), and methanol (20 mL) were stirred at room temperature, at the end of the reaction, the solvent was evaporated, water (10 mL), DCM (20 mL) was added, the organic phase was separated and evaporated to give **023366A3** (1 g, 98.9%). LCMS (ESI) m/z = 385 $[M+H]^+$.

**Step 4: Synthesis of 023366A4**

**023366A3** (100 mg, 0.25 mmol), dissolved in acetonitrile (10 mL), NBS (46 mg, 0.25 mmol) was added at room temperature, the reaction was quenched by adding water, extracted by DCM, and the solvent was evaporated to give the crude product **023366A4** (120 mg, 99.0%). LCMS (ESI) m/z = 463 $[M+H]^+$.

**Step 5: Synthesis of SZ-023366**

A mixture of **023366A4** (120 mg, 0.25 mmol), **023029A9** (55 mg,0.25 mmol), potassium carbonate (138 mg,

1 mmol), dioxane (10 mL) and water (5 ml) was stirred at room temperature. At the end of the reaction, the solvent was evaporated and purified by high pressure preparation to give **SZ-023366** (6 mg, purity 98.0%). LCMS (ESI) m/z = 624.9 $[M+H]^+$.

$^1H$ NMR (400 MHz, $CD_3OD$) δ 7.99 (dd, *J* = 3.2, 1.4 Hz, 1H), 7.78 (d, *J* = 3.1 Hz, 1H), 7.52 (d, *J* = 2.2 Hz, 1H), 7.46-7.34 (m, 2H), 6.25(s, 1H), 4.10-4.05(m, 3H), 3.91-3.80(m, 4H), 3.62-3.41(m,2H), 3.25-3.11(m, 2H), 3.02-2.76(m, 2H), 2.38-2.16(m, 2H), 1.22 (dd, *J* = 4.8, 2.8 Hz, 6H), 1.17 (t, *J* = 7.1 Hz, 3H).

**Example 19**

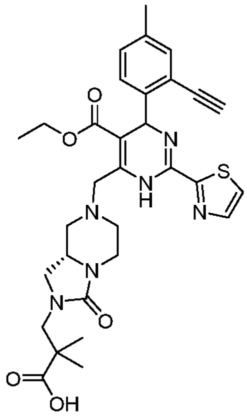

SZ-023368

Referring to the synthesis of **SZ-023366,** 4-chloro-2-bromobenzaldehyde was replaced with 4-methyl-2-bromobenzaldehyde to prepare **SZ-023368** (77 mg, purity 99.6%) as a light yellow solid. LCMS (ESI) m/z = 605.3 $[M+H]^+$.

$^1H$ NMR (400 MHz, $CD_3OD$) δ 7.99 (d, *J* = 3.2 Hz, 1H), 7.77 (d, *J* = 3.2 Hz, 1H), 7.41-7.27 (m, 2H), 7.19 (d, *J* = 7.9 Hz, 1H), 6.21 (s, 1H), 4.12-4.00 (m, 3H), 3.95-3.76 (m, 3H), 3.71 (s, 1H), 3.56 (dt, *J* = 24.8, 9.1 Hz, 1H), 3.25-3.14(m, 2H), 3.04-2.69 (m, 2H), 2.37-2.29 (m, 4H), 2.28-2.08 (m, 1H), 1.26-1.06 (m, 9H).

**Example 20**

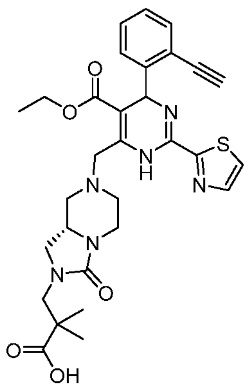

SZ-023370

Referring to the synthesis of **SZ-023366,** 2-ethynylbenzaldehyde was used as raw material to prepare **SZ-023370** (50 mg, purity 99.7%) as a light yellow solid. LCMS (ESI) m/z = 591.3 $[M+H]^+$.

$^1H$ NMR (400 MHz, $CD_3OD$) δ 7.98 (dd, *J* = 3.2, 1.6 Hz, 1H), 7.77 (d, *J* = 3.2 Hz, 1H), 7.54 (dd, *J* = 7.6, 1.4 Hz, 1H), 7.45 (d, *J* = 7.8 Hz, 1H), 7.39-7.34 (m, 1H), 7.30-7.25 (m, 1H), 6.27 (s, 1H), 4.16-3.99 (m, 3H), 3.97-3.79 (m, 3H), 3.77 (s, 1H), 3.57 (dt, *J* = 25.0, 8.9 Hz, 1H), 3.44 (dd, *J* = 14.0, 3.8 Hz, 1H), 3.24-3.09 (m, 2H), 2.99 (dd, *J* = 29.2, 10.8 Hz, 1H), 2.83 (dd, *J* = 31.8, 11.1 Hz, 1H), 2.43-2.28 (m, 1H), 2.28-2.13 (m, 1H), 1.23 (dd, *J* = 5.3, 2.1 Hz, 6H), 1.16

(t, *J* = 7.1 Hz, 3H).

**Example 21**

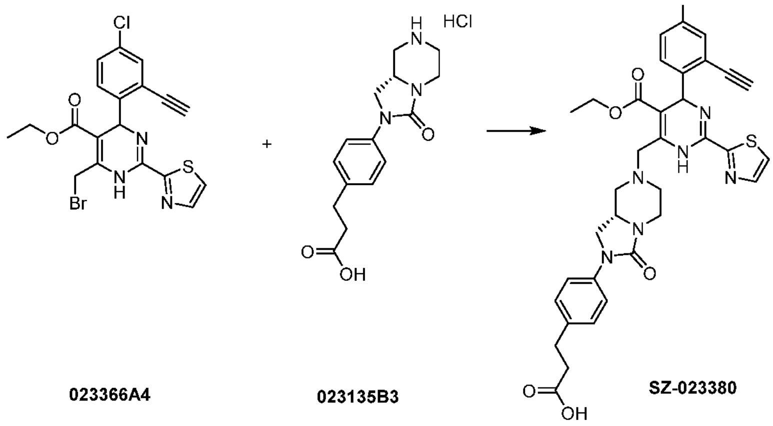

023366A4 023135B3 SZ-023380

A mixture of **023366A4** (140 mg, 0.30 mmol), Intermediate **023135B3** (100 mg,0.30 mmol, synthesized with reference to Patent CN109678859), potassium carbonate (138 mg, 1 mmol), dioxane (10 mL) and water (5 mL) was stirred at room temperature. At the end of the reaction, the solvent was evaporated and purified by high pressure preparation to give **SZ-023380** (2 mg, purity 94.4%). LCMS (ESI) m/z = 672 $[M+H]^+$.

$^1H$ NMR (400 MHz, $CD_3OD$) δ 8.03-7.97 (m, 1H), 7.79(d, *J* = 3.1 Hz, 1H , 1H), 7.53-7.38(m, 5H), 7.27-7.24(m, 2H), 6.25(s, 1H), 4.18-3.91(m, 8H), 361-3.53(m, 1H), 3.29-3.08(m,1H), 3.02-2.76(m, 3H),2.47-2.24(m, 4H), 1. 17(t, *J* = 7.1 Hz, 3H).

**Example 22**

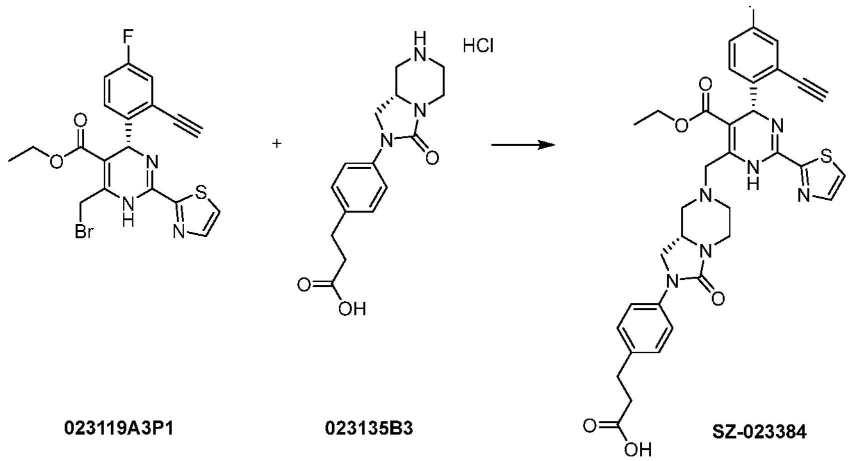

023119A3P1 023135B3 SZ-023384

**023119A3P1** (72 mg, 0.16 mmol, synthesized with reference to 023119A3), **023135B3** (53 mg, 0.16 mmol), potassium carbonate (138 mg, 1 mmol), dioxane (10 ml) and water (5 ml) were reacted. At the end of the reaction, the solvent was evaporated and purified by high pressure preparation to give **SZ-023384** (75.5 mg, purity 99.8%). LCMS (ESI) m/z = 657.2 $[M+H]^+$.

$^1H$ NMR (400 MHz, $CD_3OD$)δ 8.0(d, *J* = 3.2 Hz, 1H), 7.79(d, *J* = 3.2 Hz, 1H), 7.56-7.40(m, 3H), 7.27-7.23(m, 3H), 7.17-7.12(m, 1H), 6.25(s, 1H), 4.16-3.89(m, 8H), 3.56-3.52(dd, *J* = 9.2, 4.3 Hz,1H), 3.30-3.24(m,1H), 3.01(dd, *J* = 11.6, 3.2 Hz, 2H), 2.91(t, 3H), 2.54-2.42(m, 3H), 2.27(t, J = 10.8 Hz,1H), 1.17(t, J = 7.1 Hz, 3H).

**Example 23**

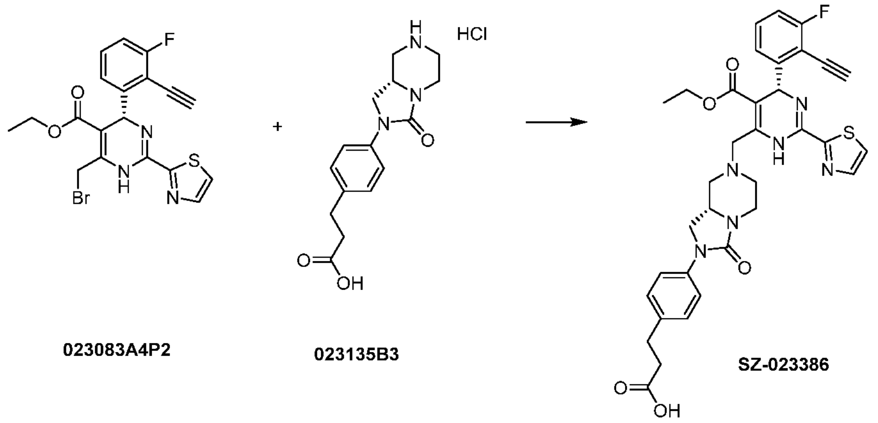

023083A4P2 023135B3 SZ-023386

A mixture of **023083A4P2** (40 mg, 0.09 mmol), **023135B3** (32 mg, 0.09 mmol), potassium carbonate (138 mg, 1 mmol), dioxane (10 ml) and water (5 ml) was stirred at room temperature. At the end of the reaction, the solvent was evaporated and purified by high pressure preparation to give **SZ-023386** (28 mg, purity 99.9%). LCMS (ESI) m/z = 657.2 $[M+H]^+$.

$^1$H NMR (400 MHz, $CD_3OD$) δ 8.00 (d, *J* = 3.2 Hz, 1H), 7.78 (d, *J* = 3.2 Hz, 1H), 7.49-7.44 (m, 2H), 7.41-7.36 (m, 1H), 7.30 (dd, *J* = 7.9, 1.1 Hz, 1H), 7.27-7.21 (m, 2H), 7.09 (t, *J* = 8.2Hz, 1H), 6.27 (s, 1H), 4.17-3.90 (m, 8H), 3.55 (dd, *J* = 9.2, 4.3 Hz, 1H), 3.28 (td, *J* = 12.9, 3.4 Hz, 1H), 3.01 (d, *J* = 11.2 Hz, 2H), 2.91 (dd, *J* = 9.2, 6.8 Hz, 2H), 2.53-2.40 (m, 3H), 2.27 (t, *J* = 10.8 Hz, 1H), 1.16 (t, *J* = 7.1 Hz, 3H).

**Example 24**

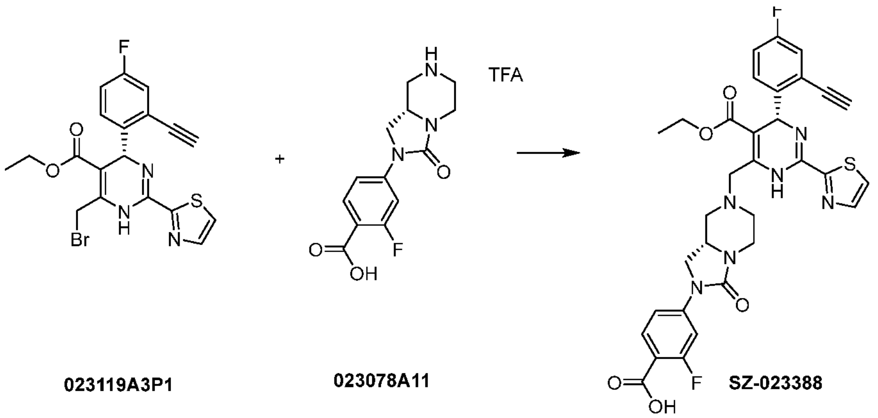

023119A3P1 023078A11 SZ-023388

**023119A3P1** (72 mg, 0.16 mmol, synthesized with reference to 023119A3), **023078A11** (64 mg,0.16 mmol, synthesized with reference to Patent CN111825676), potassium Carbonate (138 mg,1 mmol), dioxane (10 mL), and water (5 mL) were reacted. At the end of the reaction, the solvent was evaporated and purified by high pressure preparation to give SZ-023388 (67.5 mg, purity 99.9%). LCMS (ESI) m/z = 647.3 $[M+H]^+$.

$^1$H NMR (400 MHz,$CD_3OD$) δ 8.00 (d, *J* = 3.1 Hz, 1H), 7.84 (t, *J* = 8.6 Hz, 1H), 7.79 (d, *J* = 3.1 Hz, 1H), 7.62 (dd, *J* = 13.8, 2.2 Hz, 1H), 7.48 (dd, *J* = 8.7, 5.7 Hz, 1H), 7.31 (dd, *J* = 8.8, 2.2 Hz, 1H), 7.26 (dd, *J* = 9.2, 2.7 Hz, 1H), 7.14 (td, *J* = 8.5, 2.8 Hz, 1H), 6.25 (s, 1H), 4.19-3.89 (m, 8H), 3.59 (dd, *J* = 9.2, 4.3 Hz, 1H), 3.34-3.26 (m, 1H), 3.05 (dd, *J* = 10.9, 4.5 Hz, 2H), 2.48 (td, *J* = 11.7, 3.5 Hz, 1H), 2.25 (t, *J* = 10.7 Hz, 1H), 1.17 (t, *J* = 7.1 Hz, 3H).

**Example 25**

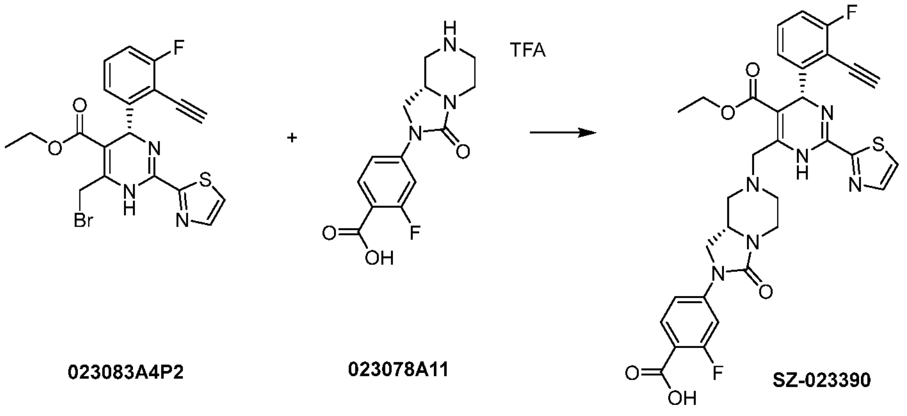

023083A4P2 023078A11 SZ-023390

**023083A4P2** (40 mg, 0.09 mmol), **023078A11** (38 mg, 0.09 mmol), potassium carbonate (138 mg, 1 mmol), dioxane (10 mL) and water (5 mL) were stirred at room temperature. At the end of the reaction, the solvent was evaporated and purified by high pressure preparation to give **SZ-023390** (30 mg, purity 99.8%). LCMS (ESI) m/z = 647.3 $[M+H]^+$.

$^1H$ NMR (400 MHz, $CD_3OD$) δ 8.00 (d, *J* = 3.2 Hz, 1H), 7.78 (d, *J* = 3.2 Hz, 1H), 7.73 (t, *J* = 8.6 Hz, 1H), 7.56 (dd, *J* = 13.4, 2.2 Hz, 1H), 7.39 (td, *J* = 8.0, 5.6 Hz, 1H), 7.30 (dd, *J* = 7.9, 1.2 Hz, 1H), 7.24 (dd, *J* = 8.6, 2.2 Hz, 1H), 7.10 (t, *J* = 8.1, 1H), 6.27 (s, 1H), 4.19-3.90 (m, 8H), 3.58 (dd, *J* = 9.2, 4.3 Hz, 1H), 3.30 (t, *J* = 12.5 Hz, 1H), 3.02 (d, *J* = 10.0 Hz, 2H), 2.48 (td, *J* = 11.6, 3.5 Hz, 1H), 2.25 (t, *J* = 10.7 Hz, 1H), 1.16 (t, *J* = 7.1 Hz, 3H).

**Example 26**

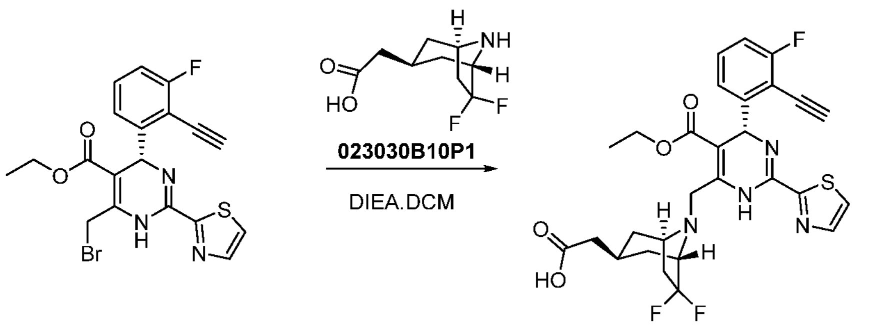


023083A4P2 SZ-023085C

To a solution of **023083A4P2** (90 mg, 0.20 mmol) in dichloromethane (10 mL) was added **023030B10P1** (41.2 mg, 0.20 mmol, prepared with reference to Patent CN105209470 B), N,N-diisopropylethylamine (77.4 mg, 0.60 mmol), and the reaction was stirred at room temperature overnight. After the reaction was completed, the solvent was removed by vacuum concentration and the residue was isolated and purified by preparative HPLC to give **SZ-023085C** (48.1 mg, 42%) as a yellow solid. Liquid Mass Spectrometry [Mobile phase: elution was maintained for 6 minutes at a flow rate of 1.5 ml per minute in a gradient from 95% water (with 0.02% ammonium acetate) and 5% acetonitrile to 5% water (with 0.02% ammonium acetate%) and 95% acetonitrile at a column temperature of 40°C. Column: waters XBridge C18 5um, 50*4.6mm, purity 99.28 %, Rt = 3.643 min. LCMS (ESI) m/z = 573.3 $[M+H]^+$.

$^1H$ NMR ($CD_3OD$, 400 MHz): δ 7.94 (d, J = 3.2 Hz, 1H), 7.73 (d, J = 3.2 Hz, 1H), 7.36-7.31 (td, J1 = 8.0 Hz, J2 = 1.6 Hz, 1H), 7.24-7.22 (m, 1H), 7.06-7.02 (m, 1H), 6.20 (s, 1H), 4.20 (s, 2H), 4.01 (q, J = 7.2 Hz, 2H), 3.98 (s, 1H), 3.48-3.46 (m, 1H), 3.31-2.28 (m, 1H), 2.71-2.61 (m, 1H), 2.39-2.25 (m, 4H), 1.96-1.90 (m, 1H), 1.78-1.66 (m, 3H), 1.14 (t, J = 7.2 Hz, 3H).

**Example 27**

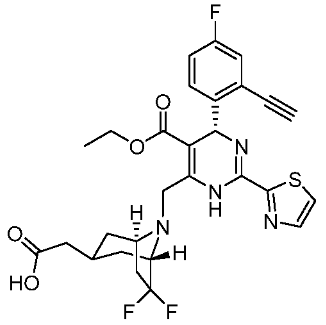

**SZ-023091C**

To a solution of **023119A3P1** (350 mg, 0.78 mmol, synthesized with reference to 023119A3) in dichloromethane (20 ml) was added **023030B10P1** (180 mg, 0.87 mmol, prepared with reference to Patent CN105209470 B), N,N-diisopropylethylamine (301 mg, 2.34 mmol). The reaction was stirred at room temperature overnight. After the reaction was completed, the solvent was removed by vacuum concentration and the residue was isolated and purified by preparative HPLC to give SZ-023091C (178 mg, 40%) as a yellow solid. Liquid Mass Spectrometry [Mobile phase: elution was maintained for 6 minutes at a flow rate of 1.5 ml per minute in a gradient from 70% water (with 0.02% ammonium acetate) and 30% acetonitrile to 30% water (with 0.02% ammonium acetate) and 70% acetonitrile at a column temperature of 40°C. Column: waters XBridge C18 5um, 50*4.6mm, purity 98.78 %, Rt = 3.205 min. LCMS (ESI) m/z = 573.2 $[M+H]^+$.

$^1H$ NMR ($CD_3OD$, 400 MHz): 7.94 (d, J = 3.2 Hz, 1H), 7.73 (d, J = 3.2 Hz, 1H), 7.40 (dd, J1 = 8.8 Hz, J2 = 5.6 Hz, 1H), 7.20 (dd, J1 = 9.6 Hz, J2 = 2.4 Hz, 1H), 7.05 (td, J1 = 8.4 Hz, J2 = 2.8 Hz, 1H), 6.19 (s, 1H), 4.19 (s, 2H), 4.03 (q, J = 6.8 Hz, 2H), 3.80 (s, 1H), 3.48-3.46 (m, 1H), 3.30-3.27 (m, 1H), 2.79-2.65 (m, 1H), 2.43-2.34 (m, 1H), 2.31-2.18 (m, 3H), 1.97-1.94 (m, 1H), 1.81-1.66 (m, 3H), 2.16 (t, J = 6.8 Hz, 3H).

**Example 28**

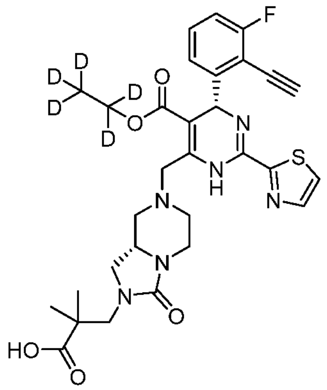

**SZ-023133**

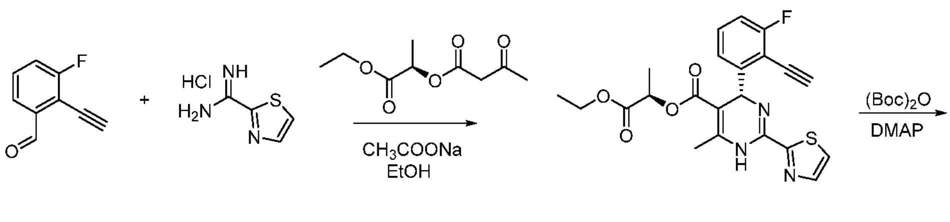


**023083A2** **(-)023084A1P2**

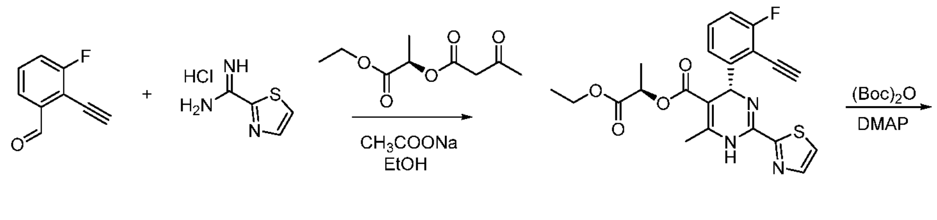


023083A2 (-)023084A1P2

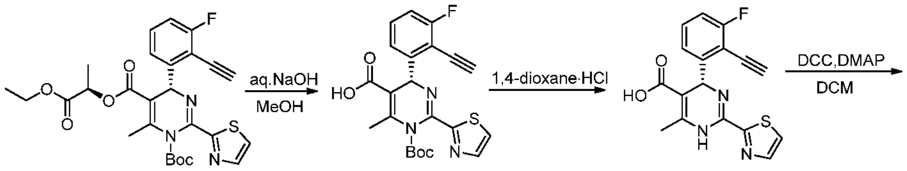


023133A1 023133A2 023133A3

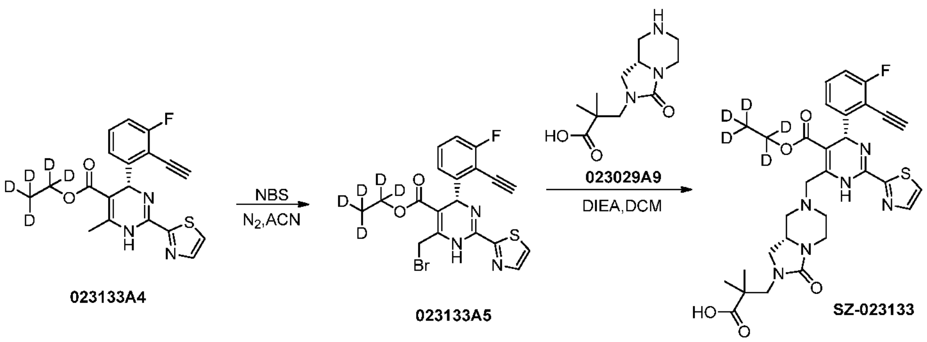


**Step 1: Synthesis of (-)-023084A1P2**

To a solution of compound 2-alkynyl-3-fluorobenzaldehyde (2.0 g, 13.5 mmol) in ethanol (30 mL) at room temperature was added 2-thiazolecarboxamidine hydrochloride (2.2 g, 13.5 mmol), chiral ester (2.7 g, 13.5 mmol), and sodium acetate (1.2 g, 14.9 mmol), and the reaction solution was stirred and reacted at 80°C overnight. The reaction solution was cooled and concentrated to remove the solvent, the residue was extracted with ethyl acetate (300 mL*2) and washed with water (300 mL*2), the organic phase was dried with anhydrous magnesium sulfate, then filtered and concentrated to dryness, and the resulting residue was separated and purified by silica gel column chromatography (EA/PE = 25%-30%) to give the yellow semi-oleic material **023084A1RAC** (1.3 g, Yield 22.0%). **023084A1RAC** was pulped twice with ether and filtered to give **(-)-023084A1P2** (350 mg, ee > 98%) as a yellow solid. LCMS (ESI) m/z = 442.1[M+H]+.

**Step 2: Synthesis of 023133A1**

To a solution of compound **(-)-023084A1P2** (300.0 mg, 0.68 mmol) in tetrahydrofuran (10 mL) at room temperature, $(Boc)_2O$ (296.5 mg, 1.36 mmol), DMAP (83.0 mg, 0.68 mmol) were added, and the reaction solution was stirred at room temperature overnight. Ethyl acetate (50 mL) and water (50 mL) were added to the reaction solution, and after partition, the layer was washed with saturated sodium chloride, dried with anhydrous sodium sulfate, and the solvent was removed by concentrating the ethyl acetate, and the resulting residue was separated and purified by silica gel column chromatography (EA/PE = 1:8) to give 023133A1 (350 mg, 95.1%) as a yellow solid. LCMS (ESI) m/z = 542.1[M+H]+.

**Step 3: Synthesis of 023133A2**

To a solution of compound **023133A1** (390 mg, 0.72 mmol) in methanol (8 mL) at room temperature, a solution of NaOH (288 mg, 7.2 mmol) in water (3 mL) was added and the reaction was carried out for 4 hours at 25°C. Ethyl acetate (50 mL) and water (50 mL) were added, and after separation, dilute hydrochloric acid (2 N) was added slowly and dropwise to the aqueous phase to adjust the pH ≈ 5. After extraction with ethyl acetate, it was washed with saturated sodium chloride, dried with anhydrous sodium sulfate, and concentrated to give **023133A2** (240 mg, 75.5% yield) as a yellow solid. LCMS (ESI) m/z = 442.2 [M+H]+.

**Step 4: Synthesis of 023133A3**

To compound **023133A2** (240 mg, 0.54 mmol), hydrogen chloride/dioxane solution (3 mL) was added at room temperature and the reaction was carried out for 3 hours at 25°C. The solvent was removed by concentration, and ethyl acetate (20 mL) and saturated aqueous NaHCOs (15 mL) were added to the residue. After separation, the organic phase was washed with saturated sodium chloride, dried over anhydrous sodium sulfate, and concentrated to give **023133A3** (160 mg, 86.2% yield) as a yellow solid. LCMS (ESI) m/z = 342.2 $[M+H]^+$.

**Step 5: Synthesis of 023133A4**

To a solution of compound **023133A3** (160 mg, 0.47 mmol) in dichloromethane (3 mL) at room temperature was added a solution of deuterated ethanol (31 mg, 0.59 mmol) in dichloromethane, followed by the addition of N,N-dimethylaminopyridine (60 mg, 0.49 mmol) and DCC (122 mg, 0.59 mmol), the reaction was carried out for 60 min at 15°C. The reaction solution was concentrated and the residue was purified by silica gel column chromatography (EA/PE = 1:10) to give **023133A4** (138 mg, 78.6% yield) as a yellow solid. LCMS (ESI) m/z = 375.2 $[M+H]^+$.

**Step 6: Synthesis of 023133A5**

To a solution of compound **023133A4** (70 mg, 0.19 mmol) in acetonitrile (2 mL) was added a solution of N-bromosuccinimide (34 mg, 0.19 mmol) in acetonitrile at room temperature, and the reaction was carried out for 20 min at 30°C under $N_2$ protection. The reaction solution was concentrated and the residue was purified by silica gel column chromatography (EA/PE = 1:10) to give 023133A5 (60 mg, 69.6% yield) as a pale yellow solid. LCMS (ESI) m/z = 453.0 $[M+H]^+$.

**Step 7: Synthesis of SZ-023133**

To a solution of compound **023133A5** (60 mg, 0.13 mmol) in dichloromethane (3 mL) at room temperature, compound **023029A9** (37 mg, 0.13 mmol) was added, followed by diisopropylethylamine (52 mg, 0.40 mmol). The reaction solution was stirred at room temperature for 4 hours. The reaction solution was concentrated to remove the solvent. The resulting residue was purified by preparative HPLC to give the yellow solid compound SZ-**023133** (20 mg, 24.5% yield). Liquid Mass Spectrometry [Mobile phase: elution was maintained for 6 minutes at a flow rate of 1.5 mL per minute on a gradient from 80% water (with 0.02% ammonium acetate) and 20% acetonitrile to 40% water (with 0.02% ammonium acetate) and 60% acetonitrile at a column temperature of 40°C. Column: Xbridge C18 ,5um 50*4.6mm, a purity 99.03% at 214 nm, Rt = 3.485 min. LCMS (ESI) m/z = 614.3 $[M+H]^+$.

$^1$H NMR ($CD_3OD$, 400 MHz): 7.94 (d, J = 3.2 Hz, 1H), 7.73 (d, J = 3.2 Hz, 1H), 7.36-7.31 (m, 1H), 7.23 (d, J = 6.8 Hz, 1H), 7.05 (td, J1 = 9.2 Hz, J2 = 1.2 Hz, 1H), 6.21 (s, 1H), 4.06-4.00 (m, 2H), 3.88-3.81 (m, 3H), 3.49 (t, J = 9.2 Hz, 1H), 3.40-3.37 (m, 1H), 3.30-3.27 (m, 1H), 3.21-3.14 (m, 1H), 3.10-3.07 (m, 1H), 2.91-2.80 (m, 2H), 2.34-2.31 (m, 1H), 2.16 (t, J = 11.2 Hz, 1H), 1.18 (s, 6H).

**Example 29**

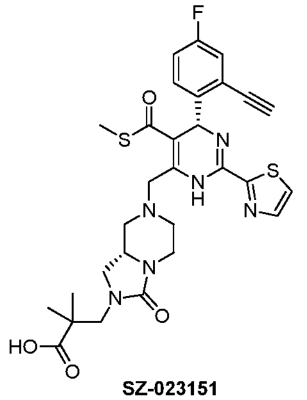

SZ-023151

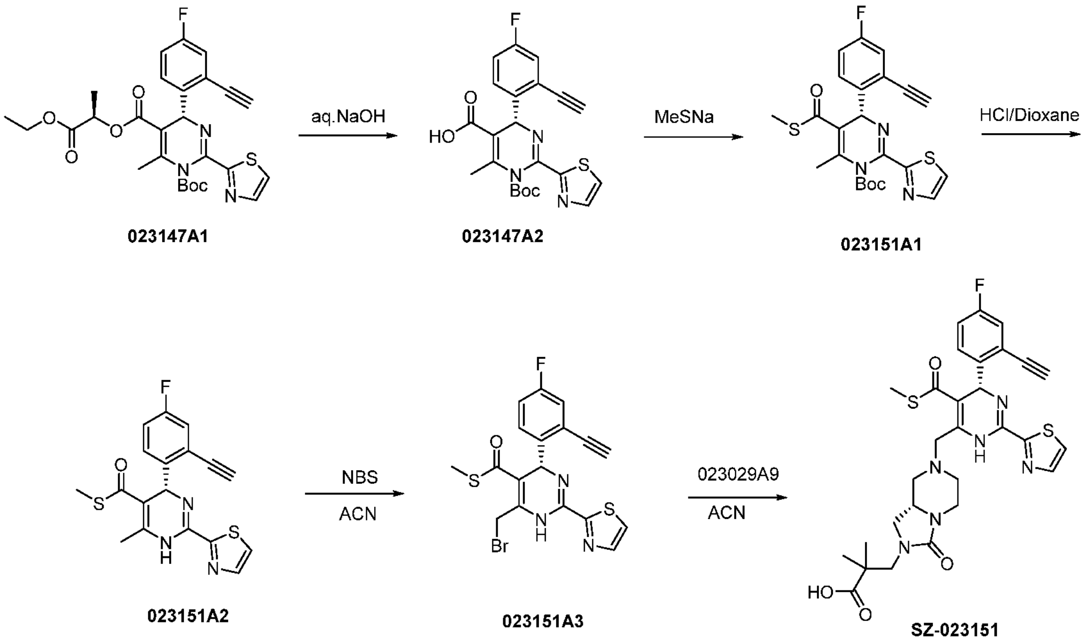


023147A1 023147A2 023151A1

023151A2 023151A3 SZ-023151

**Step 1: Synthesis of 023147A2**

To a solution of **023147A1** (1.0 g, 1.84 mmol) in methanol (10 mL) at room temperature, sodium hydroxide solution (2 N, 9.2 mL, 18.4 mmol) was added. The reaction solution was stirred overnight at room temperature under nitrogen protection. After the reaction was completed, the reaction solution was poured into liquid water (50 mL) and ether (50 mL) and the organic layer was separated. The aqueous layer was adjusted to pH 5.0 with 2N hydrochloric acid solution and extracted with ethyl acetate. The organic phases were combined and concentrated to remove the solvent to give 023147A2 (800 mg, 100%) as a pale yellow solid. LCMS (ESI) m/z = 442.1 $[M+H]^+$.

**Step 2: Synthesis of 023151A1**

To a solution of **023147A2** (750 mg, 1.70 mmol) in dichloromethane (30 mL) at room temperature, N,N-dimethylformamide (146 mg, 2.04 mmol) and oxalyl chloride (259 mg, 2.04 mmol) were added. The reaction solution was stirred at room temperature for 15 min, and then the solvent was removed. The resulting residue was dissolved in tetrahydrofuran (20 mL), half of the solution was added dropwise to a solution of sodium methanethiol (1.2 g, 17 mmol) in water (10 mL) and tetrahydrofuran (20 mL), and the resulting reaction solution was stirred for 10 min at room temperature. The reaction solution was added with water (100 mL) and extracted with ethyl acetate (50 mL*3). The organic phases were combined and concentrated. The residue was separated and purified by silica gel column chromatography (EA/PE = 4:1) to give 023151A1 (180 mg, 45%) as a yellow solid. LCMS (ESI) m/z = 472.1 $[M+H]^+$.

**Step 3: Synthesis of 023151A2**

A solution of compound 023151A1 (180 mg, 0.38 mmol) in HCl/Dioxane (4N, 5 mL) was stirred overnight at

room temperature. The solvent was removed from the reaction solution, extracted with ethyl acetate, dried and concentrated to give 023151A2 (120 mg, 92%) as a pale yellow solid. LCMS (ESI) m/z = 372.1 [M+H]$^+$.

**Step 4: Synthesis of 023151A3**

To a solution (10 mL) of compound **023151A1** (90 mg, 0.0.26 mmol) in acetonitrile was added N-bromosuccinimide (46 mg, 0.26 mmol) at room temperature, and the reaction solution was stirred for 30 min at room temperature to obtain a solution of **023151A3.** This reaction solution is used directly in the next step without treatment. LCMS (ESI) m/z = 450.0 /452.0[M+H]$^+$.

**Step 5: Synthesis of SZ-023151**

**023029A9** (100 mg, 0.26 mmol), and N,N-diisopropylethylamine (100 mg, 0.78 mmol) were added directly to the solution of **023151A3** and the reaction was stirred for 2 hours at room temperature. After the reaction was completed, the solvent was removed by vacuum concentration and the residue was isolated and purified by preparative HPLC to give SZ-**023151** (2.0 mg, 1.2%) as a yellow solid. Liquid phase mass spectrometry [Mobile phase: elution was maintained for 6 minutes at a flow rate of 1.5 ml per minute in a gradient from 80% water (with 0.1% trifluoroacetic acid) and 20% acetonitrile to 30% water (with 0.1% trifluoroacetic acid) and 70% acetonitrile at a column temperature of 40°C. Column: waters XBridge C18 5um, 50*4.6mm, purity 86.7 %, Rt = 3.128 min. LCMS (ESI) m/z = 611.3 [M+H]$^+$.

$^1$H NMR (dMSO-d6, 400 MHz): δ 12.22 (br s, 1H), 9.89 (s, 1H), 8.03 (d, J = 4.0 Hz, 1H), 7.95 (d, J = 3.2 Hz, 1H), 7.38-7.31 (m, 2H), 7.24-7.19 (m, 1H), 6.29 (s, 1H), 4.57 (s, 1H), 3.95-3.89 (m, 2H), 3.73-3.62 (m, 2H), 3.28-3.14 (m, 3H), 3.01-2.93 (m, 2H), 2.92-2.76 (m, 4H), 2.22-2.19 (m, 4H), 2.16-2.07 (m, 1H), 1.24 (s, 6H).

**Example 30**

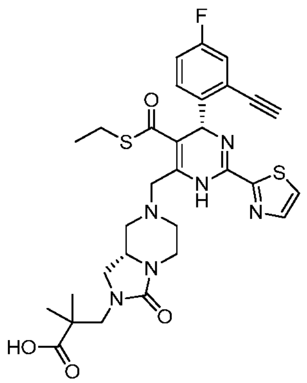

**SZ-023163**

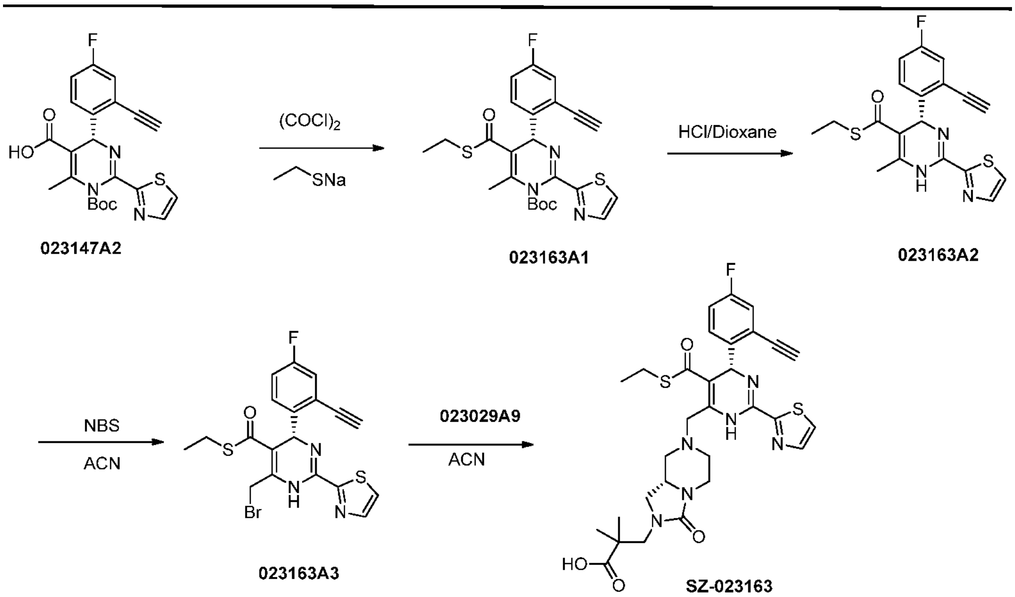


023147A2 023163A1 023163A2 023163A3 SZ-023163

**Step 1: Synthesis of 023163A1**

To a solution of **023147A2** (750 mg, 1.70 mmol) in dichloromethane (30 mL) at room temperature, N,N-dimethylformamide (146 mg, 2.04 mmol) and oxalyl chloride (259 mg, 2.04 mmol) were added. The reaction solution was stirred for 15 min at room temperature and the solvent was removed. The resulting residue was dissolved in tetrahydrofuran (20 mL), half of the solution was added dropwise to a solution of sodium ethanethiol (1.4 g, 17 mmol) in water (10 mL) and tetrahydrofuran (20 mL), and the resulting reaction solution was stirred for 10 min at room temperature. The reaction solution was added with water (100 mL) and extracted with ethyl acetate (50 mL*3). The organic phases were combined and concentrated. The residue was separated and purified by silica gel column chromatography (EA/PE = 4:1) to give **023163A1** (180 mg, 45% yield) as a yellow solid. LCMS (ESI) m/z = 486.1 $[M+H]^+$.

**Step 2: Synthesis of 023163A2**

A solution of compound **023163A1** (180 mg, 0.38 mmol) in HCl/Dioxane (4 N, 5 mL) was stirred overnight at room temperature. The reaction solution was removed from the solvent, extracted with ethyl acetate, dried and concentrated to give 023163A2 (130 mg, 88% yield) as a pale yellow solid. LCMS (ESI) m/z = 386.1 $[M+H]^+$.

**Step 3: Synthesis of 023163A3**

To a solution (10 mL) of compound **023163A2** (90 mg, 0.0.26 mmol) in acetonitrile was added N-bromosuccinimide (62.3 mg, 0.35 mmol) at room temperature, and the reaction solution was stirred for 30 min at room temperature to give a solution of 023163A3. This reaction solution was used directed in the next step without treatment. LCMS (ESI) m/z = 464.0 /466.0$[M+H]^+$.

**Step 4: Synthesis of SZ-023163**

**023029A9** (70 mg, 0.35 mmol), and N,N-diisopropylethylamine (135 mg, 1.05 mmol) were added directly to the solution of **023163A3** and the reaction was stirred for 2 hours at room temperature. After the reaction was completed, the solvent was removed by vacuum concentration and the residue was isolated and purified by preparative HPLC to give SZ-023163 (2.0 mg, 1.0%) as a yellow solid. Liquid Mass Spectrometry [Mobile phase: elution was maintained for 6 minutes at a flow rate of 1.5 ml per minute in a gradient from 75% water (with 0.02% ammonium acetate) and 25% acetonitrile to 50% water (with 0.02% ammonium acetate) and 50% acetonitrile at a column temperature of 40°C. Column: waters XBridge C18 Sum, 50*4.6mm, 90.5% purity, Rt = 4.304 min. LCMS (ESI) m/z = 625.3 $[M+H]^+$.

$^1$H NMR (DMSO-d6, 400 MHz): δ 12.33 (br s, 1H), 9.89 (s, 1H), 8.03 (d, J = 3.2 Hz, 1H), 7.95 (d, J = 3.2 Hz, 1H), 7.38-7.30 (m, 2H), 7.24-7.19 (m, 1H), 6.26 (s, 1H), 4.57 (s, 1H), 3.94 (d, J = 4.0 Hz, 2H), 3.73-3.63 (m, 2H) 3.69-3.35 (m, 1H), 3.38-3.14 (m, 2H), 3.00-2.92 (m, 2H), 2.86-2.70 (m, 4H), 2.22-2.16 (m, 1H), 2.07-2.02 (m, 1H), 1.09-1.06 (m, 9H).

**Example 31**

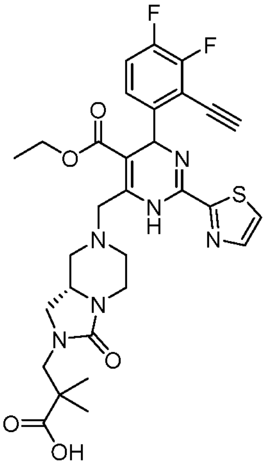

SZ-023127

Using suitable starting materials, **SZ-023127** can be prepared with reference to the synthesis of compound **SZ-023089RAC.** LCMS (ESI) m/z 627.3 $[M+H]^+$.

$^1H$ NMR ($CDCl_3$, 400 MHz): δ 9.60 (s, 1H), 9.88 (t, J = 3.2 Hz, 1H), 7.49-7.48 (m, 1H), 7.14-7.10 (m, 2H), 6.247-6.240 (m, 1H), 4.12-4.05 (m, 3H), 3.98-3.83 (m, 3H), 3.64 (s, 1H), 3.60-3.36 (m, 3H), 3.25-3.03 (m, 2H), 2.90-2.69 (m, 2H), 2.46-2.18 (m, 2H), 1.30 (s, 3H), 1.29 (s, 3H), 1.18 (t, J = 7.2 Hz, 3H).

**Example 32**

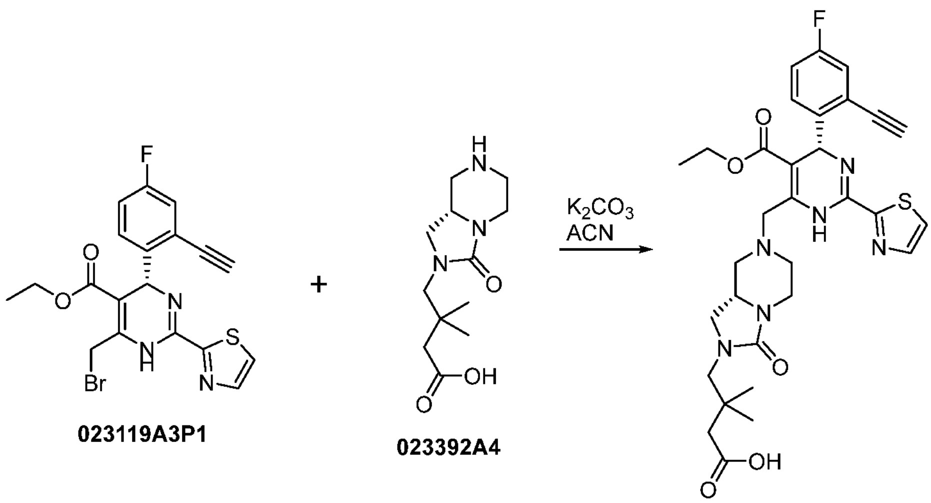


**023119A3P1** **023392A4** **SZ-023392**

**SZ-023392** (20 mg, 99.8%, 15% yield) was prepared by referring to the synthesis of compound **SZ-023089** using **023392A4** (prepared by method referring to patent US20160083383 A1) and **023119A3P1** as starting materials. LCMS (ESI) m/z 623.32 (M+H)+.

$^1H$ NMR (400 MHz, $CD_3OD$) δ 7.97 (t, J = 2.4 Hz, 1H), 7.75 (d, J = 3.1 Hz, 1H), 7.44 (ddd, J = 8.9, 5.6, 3.5 Hz, 1H), 7.22 (dd, J = 9.3, 2.7 Hz, 1H), 7.16-7.06 (m, 1H), 6.22 (s, 1H), 4.15-3.98 (m, 3H), 3.87 (d, J = 17.1 Hz, 4H), 3.62 (dt, J = 25.6, 8.9 Hz, 1H), 3.28-3.15 (m, 1H), 3.13-2.98 (m, 1H), 2.94-2.83 (m, 1H), 2.35 (t, J = 11.0 Hz, 1H), 2.27-2.12 (m, 3H), 1.15 (t, J = 7.1 Hz, 3H), 1.07 (d, J = 5.9 Hz, 6H).

**Example 33**

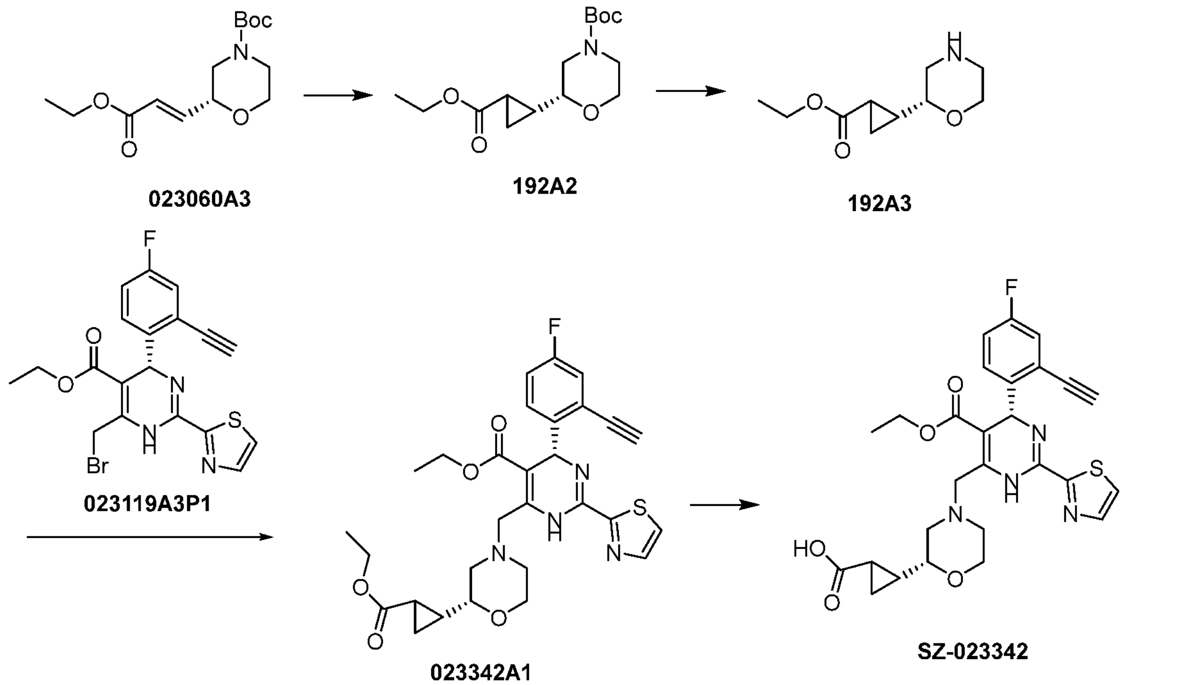

023060A3 192A2 192A3

023119A3P1

023342A1

SZ-023342

**Step 1: Synthesis of 192A2**

第一步: **192A2**的合成

To **023060A3** (57 mg,0.2 mmol) was added DMSO (5 ml), trimethylsulfoxide iodide (52.8 mg,0.24 mmol), potassium tert-butoxide (22.4 mg,0.2 mmol) and stirred at room temperature for 2 hours. At the end of the reaction, 50 ml of EA was added, and washed with water (3*20 ml) to give the crude 192A2 (60 mg. 100%), which was directly evaporated to dryness and set aside. LCMS (ESI) m/z 299 $[M+H]^+$.

**Step 2: Synthesis of 192A3**

To **192A2** (60 mg, 0.2 mmol) was added DCM (5 ml) and 4N hydrogen dioxide chloride solution (5 ml) was added dropwise at room temperature, the solvent was evaporated at the end of the reaction and washed with petroleum ether (2*5 ml), and the residual oil was evaporated to give **192A3** (25 mg, 62.5%), which was used directly in the next step. LCMS (ESI) m/z 199 $[M+H]^+$.

**Step 3: Synthesis of 023342A1**

To **023001A4** (338 mg, 0.75 mmol) was added 10 ml of dioxane, 10 ml of water, **192A3** (150 mg, 0.75 mmol), potassium carbonate (207 mg, 1.50 mmol), and after the reaction was completed, the solvent was evaporated to dryness and used directly in the next step.

**Step 4: Synthesis of SZ-023342**

To the crude product obtained above, ethanol (10 ml), water (5 ml), lithium hydroxide (120 mg, 3.0 mmol) were added. After the reaction was completed, the solvent was evaporated to give SZ-023342 (8 mg, 2.0%). LCMS (ESI) m/z 539.2 $[M+H]^+$.

$^1H$ NMR (400 MHz, $CD_3OD$-d4) δ 7.97(dd, J = 6.0, 3.2Hz, 1H), 7.77(dd, J = 5.4, 3.1 Hz, 1H), 7.45(dd, J = 8.7, 5.7Hz, 1H), 7.25(d, 1H), 7.12(td, J = 8.5, 2.8 Hz, 1H), 6.25(d, J = 4.9 Hz, 1H), 4.11-3.70(m, 7H), 3.20-3.11(m,1H), 2.98-2.81(m, 2H), 2.54-2.46(m, 1H), 2.26(t, J = 10.7 Hz, 1H), 1.19(td, J = 7.1, 4.6 Hz, 3H).

**Biological Assay Examples**

Evaluation of compounds for *in vitro* anti-HBV activity and cytotoxicity assay using HepG2.2.15 cell line

Compound preparation and cell culture: The test compounds were prepared as 20 mM storage solution using the appropriate volume of dimethyl sulfoxide (DMSO) (Sigma-D2650) according to the relative molecular weight and mass.

The growth of HepG2.2.15 cells (Wuhan Institute of Virology, Chinese Academy of Sciences) in the culture flasks were observed under a microscope, the cells were digested with trypsin (Invitrogen-25300062), and resuspended with cell culture medium (DMEM/F12, Invitrogen-11330032; containing 2% fetal bovine serum FBS, HyClone-SV30087.03, 100 U/Ml penicillin, Hyclone-SV30010, 100 μg/mL streptomycin, Hyclone-SV30010, 1% non-essential amino acids, Invitrogen-11140-050, 2 mM L-glutamine, Gibco-35050-061); the cells were counted using a Vi-Cell cell counter and the volume and concentration of cell suspension were adjusted according to the number of cell spreads and the number of cells required per well, and the cell spreading density of the 96-well cell culture plates (Costar- 3599) was 60,000 cells/well, 100 μL/well; the cell plates with the cells were placed in a 5% $CO_2$, 37°C incubator and incubated overnight.

The experiments were set at a starting concentration of 100 μM for the test compound and 20 nM for the reference compound entecavir (ETV, WuXi AppTec), with a 3-fold gradient dilution and 9 concentration points. A 96-well V-bottom plate was used, the corresponding test compounds and reference compounds ETV were diluted to obtain 200× compound source plate; a sterile 2 mL deep-well plate was added with 297 μL of cell culture medium, 3 μL of the compounds in the 200× compound source plate were added to the corresponding deep-well plate, diluted by 100-fold, and mixed well with a pipette tip blowing to obtain a 2× compound plate, and then the plate was sealed with parafilm and then stored in a nitrogen cabinet to be used for replacement of the compounds. The cell plate laid out the day before was took and added with 100 μL of diluted 2× compound to each of the corresponding cell wells with a final concentration of 0.5% DMSO. Example of compound distribution in the cell plate is shown below*:

<table>
<tr><td></td><td>1</td><td>2</td><td>3</td><td>4</td><td>5</td><td>6</td><td>7</td><td>8</td><td>9</td><td>10</td><td>11</td><td>12</td></tr>
<tr><td>A</td><td></td><td colspan="9">3-fold gradient dilution, 9 concentration points, single well test per compound per concentration</td><td></td><td></td></tr>
<tr><td>B</td><td>Test compound A</td><td>high concentration</td><td colspan="7">3-fold gradient dilution, 9 concentration points</td><td>low concentration</td><td rowspan="3">0.5% DMSO control well</td><td rowspan="3">Medium blank control well</td></tr>
<tr><td>C</td><td>Test compound B</td><td>high concentration</td><td colspan="7">3-fold gradient dilution, 9 concentration points</td><td>low concentration</td></tr>
<tr><td>D</td><td>Test compound C</td><td>high concentration</td><td colspan="7">3-fold gradient dilution, 9 concentration points</td><td>low concentration</td></tr>
<tr><td>E</td><td>Test compound D</td><td>high concentration</td><td colspan="7">3-fold gradient dilution, 9 concentration points</td><td>low concentration</td><td rowspan="3"></td><td rowspan="3"></td></tr>
<tr><td>F</td><td>Test compound E</td><td>high concentration</td><td colspan="7">3-fold gradient dilution, 9 concentration points</td><td>low concentration</td></tr>
<tr><td>G</td><td>Reference compound ETV</td><td>high concentration</td><td colspan="7">3-fold gradient dilution, 9 concentration points</td><td>low concentration</td></tr>
<tr><td>H</td><td></td><td colspan="9"></td><td></td><td></td></tr>
<tr><td colspan="13">*Test compounds A, B, C, D, E ...... refer generically to the compounds to be tested and the table is an illustrative chart; ETV is Entecavir.</td></tr>
</table>

After the cell plates were reintroduced into a 5% $CO_2$, 37°C cell incubator for 3 days, the medium was removed from the cell culture plates and 100 μL of cell culture solution was replenished to each well. The 200× compound source plate configured was removed from the nitrogen cabinet, a sterile 2 mL deep-well plate was added with 297 μL of cell

culture medium, 3 μL of the compounds in the 200× compound source plate was added to the corresponding deep-well plate, diluted by 100-fold, and mixed well with a pipette tip blowing to obtain a 2× compound plate. To the cell wells containing 100 μL of medium, 100 μL of diluted 2× compound was added, with a final concentration of DMSO of 0.5%; the cell plates were reintroduced into 5% $CO_2$ and incubated in a 37°C cell incubator for 3 days.

The 96-well cell plate was removed from the incubator, centrifuged at 3,000 rpm for 2 min with centrifuge (Beckman, Allegra-X15R), and the cell supernatant was collected for subsequent HBV DNA extraction. 50 μL of cell culture solution were added to each well in the cell plate, then 50 μL of CellTiter-Glo Detection Reagent (Promega-G7573) were added to each well, and the plate was shaken for 10 min away from light to allow the cells to be lysed; the luminescence value of each well was detected by using an enzyme labeller (BioTek Synergy 2).

HBV DNA Extraction: HBV DNA was extracted from cell supernatants using the QIAamp 96 DNA Blood Kit (Qiagen-51162) as described in the kit instructions; the volume of cell supernatant was 150 μL and the final DNA elution volume was 180 μL.

qPCR assay: the desired qPCR reaction mix was prepared as shown in the table below.

| Components | Volume required per well to configure a 384-well plate (μL) | Volume required to configure 1000 wells (μL) |
|---|---|---|
| FastStart Universal Probe Mast Mix (Roche-04914058001) | 5 | 5000 |
| upstream primer (10 μM) | 0.4 | 400 |
| downstream primer (10 μM) | 0.4 | 400 |
| probe (10 μM) | 0.2 | 200 |
| AE buffer (supplied with QIAamp 96 DNA Blood Kit) | 2 | 2000 |

Upstream primer sequence: 5'GTGTCTGCGGCGTTTTATCA3'
Downstream primer sequence: 5'GACAAACGGGCAACATACCTT3'
Probe Sequence: 5'+ FAM + CCTCTKCATCCTGCTGCTATGCCTCATC + TAMRA -3'

In a 384-well qPCR plate (Applied Biosystems-4309849), 8 μL of reaction mix was added to each well; 5 ng/μL of pAAVHBV1.3 plasmid (constructed by Biology Department, Research Services Division, Shanghai WuXi AppTec New Drug Development Co., Ltd, and prepared by Suzhou GENEWIZ Biotechnology Co.) was diluted 60-fold to obtain $1 \times 10^7$ copies/μL (estimated based on molecular weight, the plasmid had 7027bp bases, the average molecular weight of a single base was 670, and 5 ng was roughly equivalent to $6\times10^8$), which was the highest point of the standard, and then dilute down the 10-fold gradient to 10 copies/μL for a total of seven points. 2 μL of sample DNA or standards of HBV DNA were added to each well. The PCR system was 10 μL; the reaction conditions for PCR were: heating at 95°C for 10 min; then denaturation at 95°C for 15 s and extension at 60°C for 1 min for a total of 40 cycles. The qPCR instrument (Applied Biosystems, QuantStudio 6) or qPCR instrument (Applied Biosystems, 7900HT) was run with the above parameters.

Data analysis:

The following formula was applied to calculate the percentage of cellular activity: Cell viability (%) = (Chemiluminescence value in the sample - Chemiluminescence value in the medium control) / (Chemiluminescence value in the DMSO control - Chemiluminescence value in the medium control) × 100, and the 50% cytotoxicity concentration of the compounds, $CC_{50}$ value, was calculated using GraphPad Prism 6 software.

$$Y = Bottom + (Top\text{-}Bottom)/(1+10^{\wedge}((LogCC_{50}\text{-}X)*HillSlope))$$

Y: Cell viability.
X: Log [compound concentrations].

*In vitro* anti-hepatitis B virus (HBV) activity of compounds by q-PCR assay

A standard curve was obtained by linear regression based on the Ct value of the standard and the concentration of the standard, and the HBV DNA copy number in the sample was calculated from the standard curve by the Ct value

of the sample;

The inhibition rate of the compounds was calculated by the formula: Percentage inhibition (%) = (1-(Number of HBV DNA copies in the sample / Number of HBV DNA copies in the DMSO control group) × 100, and the $EC_{50}$ values of the compounds were calculated by GraphPad Prism 6 using the equation "log(inhibitor) vs. response-Variable slope".

$$Y = Bottom + (Top-Bottom) / (1+10^{((LogIC50-X)*HillSlope)})$$

Y: Efficacy/Inhibition activity.

X: Log [compound concentrations].

Biological assay results:

Table 1

| Compound No. | $EC_{50}$ (μM) |
|---|---|
| SZ-023089RAC | A |
| SZ-023089 | A |
| SZ-023091RAC | A |
| SZ-023091 | A |
| SZ-023093RAC | A |
| SZ-023093 | A |
| SZ-023095RAC | B |
| SZ-023095 | A |
| SZ-023115RAC | A |
| SZ-023115-P2 | A |
| SZ-023117RAC | B |
| SZ-023084B | A |
| SZ-023085B | A |
| SZ-023088B | A |
| SZ-023090B | A |
| SZ-023097B | C |
| SZ-023129 | C |
| SZ-023141 | A |
| SZ-023143 | A |
| SZ-023145 | A |
| SZ-023147 | A |
| SZ-023149 | A |
| SZ-023366 | A |
| SZ-023368 | C |
| SZ-023370 | B |
| SZ-023380 | A |
| SZ-023384 | A |
| SZ-023386 | A |

(continued)

| Compound No. | $EC_{50}$ (μM) |
|---|---|
| SZ-023388 | A |
| SZ-023390 | A |
| SZ-023085C | A |
| SZ-023091C | A |
| SZ-023133 | A |
| SZ-023151 | C |
| SZ-023084A | D |
| SZ-023085A | D |
| SZ-023088A | D |
| SZ-023090A | D |
| SZ-023097A | D |
| SZ-023115-P1 | D |
| SZ-023342 | B |
| SZ-023392 | A |
| SZ-023127 | A |
| where A is <0.050 μM, B is 0.050 μM to 0.10 μM, C is 0.10 μM to 1.00 μM, and D is >1.0 μM. | |

## Claims

1. A phenyldihydropyrimidine compound as shown in Formula I, a tautomer or a pharmaceutically acceptable salt thereof;

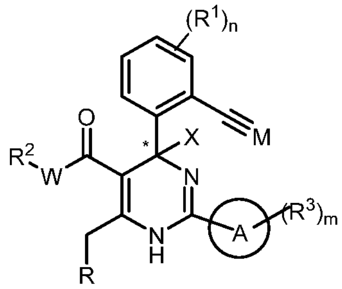

I

where M is CH or N;
X is H or D;
$R^1$ is independently halogen or $C_{1-4}$ alkyl;
n is 0, 1, 2, 3, or 4;
m is 0, 1, 2, 3, or 4;
$R^2$ is methyl or ethyl;
W is O or S;
ring A is a 5- to 6-membered heteroaryl; wherein said 5- to 6-membered heteroaryl comprises 1, 2 or 3 heteroatoms selected from N, O and S;
$R^3$ is independently H, halogen, $C_{1-4}$ alkyl, $C_{1-4}$ haloalkyl, $C_{3-6}$ cycloalkyl, or $-(CH_2)_{n3}-OH$;
n3 is 0, 1, 2, 3, or 4;
Ris

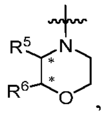, 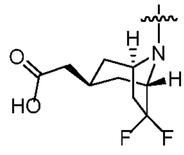, 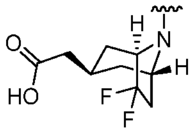, 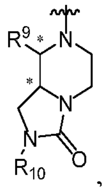, or 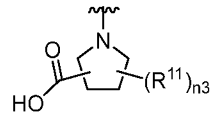;

One of $R^5$, $R^6$ is independently H, -COOH, $-(CH_2)_{n1}COOH$, $-CH=CH-(CH_2)_{n2}COOH$, or

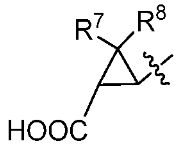,

the other is H or $C_{1-4}$ alkyl;
n1 is 1, 2, 3, or 4;
n2 is 0, 1, or 2;
$R^7$, $R^8$ are independently H or halogen;
when $R^9$ is H, $R^{10}$ is phenyl-$C_{1-5}$ alkanediyl-COOH or -$C_{1-5}$ alkanediyl-COOH substituted by one or more $R^{12}$; when there are multiple substituents, they are the same or different;
or when $R^9$ is -COOH, $R^{10}$ is independently $C_{1-4}$ alkyl;
n3 is 0, 1, 2, 3, or 4;
$R^{11}$ is independently halogen or $C_{1-4}$ alkyl; or, any two $R^{11}$ are joined together with the carbon to which they are attached to form $C_{3-6}$ cycloalkyl;
$R^{12}$ is independently H, halogen, or $C_{1-4}$ alkyl;
a carbon atom with "*" indicates that when it is a chiral carbon atom, it is in S configuration, R configuration or a mixture thereof.

**2.** The phenyldihydropyrimidine compound as shown in Formula I, a tautomer or a pharmaceutically acceptable salt thereof according to claim 1, **characterized in that**,

the tautomer of the phenyldihydropyrimidine compound as shown in Formula I is

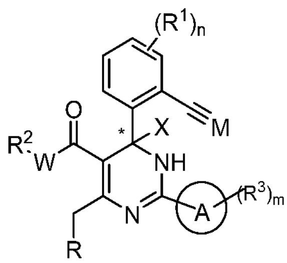

I ;

and/or the phenyldihydropyrimidine compound as shown in Formula I has the following structure:

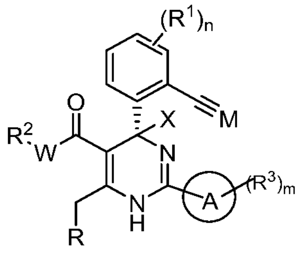

**Ia** , or

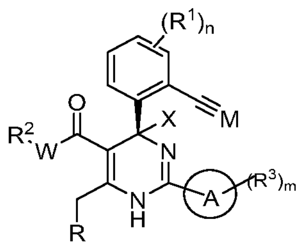

**Ib** ;

and/or, $R^1$ is independently located at an ortho, meta or para position of the dihydropyrimidine ring;
and/or, when $R^1$ is halogen, said halogen is fluorine, chlorine, bromine, or iodine;
and/or, when $R^1$ is $C_{1-4}$ alkyl, said $C_{1-4}$ alkyl is methyl, ethyl, n-propyl, isopropyl, n-butyl, isobutyl, sec-butyl, or tert-butyl;
and/or, when ring A is a 5- to 6-membered heteroaryl group, said 5- to 6-membered heteroaryl group is thienyl, thiazolyl, oxazolyl, imidazolyl, thiadiazolyl, or pyridyl;
and/or, when $R^3$ is independently halogen or $C_{1-4}$ haloalkyl, said halogen and the halogen of said $C_{1-4}$ haloalkyl is fluorine, chlorine, bromine, or iodine;
and/or, when $R^3$ is independently $C_{1-4}$ alkyl or $C_{1-4}$ haloalkyl, said $C_{1-4}$ alkyl and the $C_{1-4}$ alkyl of said $C_{1-4}$ haloalkyl is methyl, ethyl, n-propyl, isopropyl, n-butyl, isobutyl, sec-butyl, or tert-butyl;
and/or, when $R^3$ is independently $C_{1-4}$ haloalkyl, the number of halo is 1, 2, or 3;
and/or, when $R^3$ is independently $C_{3-6}$ cycloalkyl, said $C_{3-6}$ cycloalkyl is cyclopropyl, cyclobutyl, cyclopentyl, or cyclohexyl;
and/or, when one of $R^5$ and $R^6$ is independently $C_{1-4}$ alkyl, said $C_{1-4}$ alkyl is methyl, ethyl, n-propyl, isopropyl, n-butyl, isobutyl, sec-butyl, or tert-butyl;
and/or, when $R^7$ and $R^8$ are independently halogen, said halogen is fluorine, chlorine, bromine, or iodine;
and/or, when $R^{10}$ is phenyl-$C_{1-5}$ alkanediyl-COOH or -$C_{1-5}$ alkanediyl-COOH substituted by one or more $R^{12}$, the $C_{1-5}$ alkanediyl of said phenyl-$C_{1-5}$ alkanediyl-COOH and -$C_{1-5}$ alkanediyl-COOH substituted by one or more $R^{12}$ is methylene, $-CH_2CH_2-$, $-CH(CH_3)-$, - $CH_2CH_2CH_2-$, $-CH(CH_3)CH_2-$, $-CH_2CH(CH_3)-$, $-C(CH_3)_2-$, $-CH_2CH_2CH_2CH_2-$, - $CH(CH_3)CH_2CH_2-$, $-CH_2CH(CH_3)CH_2-$, $-CH_2CH_2CH(CH_3)-$, $-C(CH_3)_2CH_2-$, $-CH_2C(CH_3)_2-$, or $-CH_2C(CH_3)_2-CH_2-$;
and/or, when $R^{10}$ is $C_{1-4}$ alkyl, said $C_{1-4}$ alkyl is methyl, ethyl, n-propyl, isopropyl, n-butyl, isobutyl, sec-butyl, or tert-butyl;
and/or, when $R^{11}$ is independently halogen, said halogen is fluorine, chlorine, bromine, or iodine;
and/or, when $R^{11}$ is $C_{1-4}$ alkyl, said $C_{1-4}$ alkyl is methyl, ethyl, n-propyl, isopropyl, n-butyl, isobutyl, sec-butyl, or tert-butyl;
and/or, when any two $R^{11}$ are joined together with the carbon to which they are attached to form $C_{3-6}$ cycloalkyl, said $C_{3-6}$ cycloalkyl is cyclopropyl, cyclobutyl, cyclopentyl, or cyclohexyl;
and/or, when $R^{12}$ is independently halogen, said halogen is fluorine, chlorine, bromine, or iodine;
and/or, when $R^{12}$ is $C_{1-4}$ alkyl, said $C_{1-4}$ alkyl is methyl, ethyl, n-propyl, isopropyl, n-butyl, isobutyl, sec-butyl, or tert-butyl;
and/or,

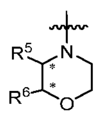 is 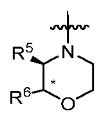 or 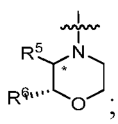 ;

and/or,

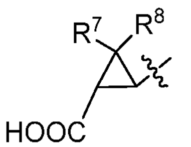 is 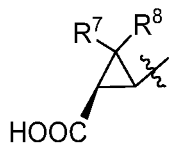 or 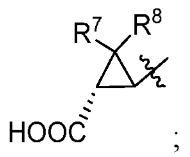 ;

and/or, n3 is 0, 1, or 2;

and/or,

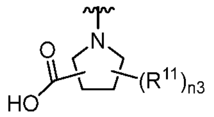 is 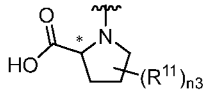;

and/or,

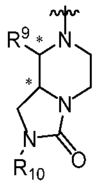 is 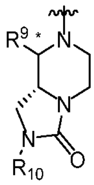, 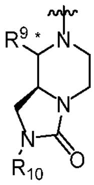, 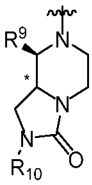, or 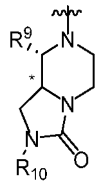;

and/or,

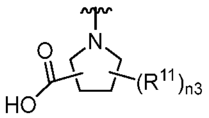 is 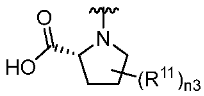 or 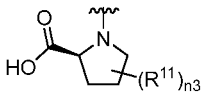.

**3.** The phenyldihydropyrimidine compound as shown in Formula I, a tautomer or a pharmaceutically acceptable salt thereof according to claim 1, **characterized in that**,

when $R^1$ is halogen, said halogen is fluorine or chlorine;
and/or, when $R^1$ is $C_{1-4}$ alkyl, said $C_{1-4}$ alkyl is methyl;
and/or, when ring A is a 5- to 6-membered heteroaryl group, said 5- to 6-membered heteroaryl group is

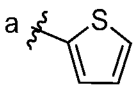, 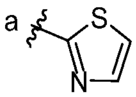, 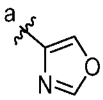, 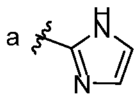, 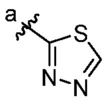, or 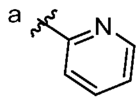,

end a indicates the position of attachment to the pyrimidine ring;
and/or, when $R^3$ is independently halogen or $C_{1-4}$ haloalkyl, said halogen and the halogen of said $C_{1-4}$ haloalkyl is fluorine or chlorine;
and/or, when $R^3$ is independently $C_{1-4}$ alkyl or $C_{1-4}$ haloalkyl, the $C_{1-4}$ alkyl and the $C_{1-4}$ alkyl of said $C_{1-4}$ haloalkyl is methyl;
and/or, when $R^3$ is independently $C_{1-4}$ haloalkyl, the number of halo is 3;
and/or, when $R^3$ is independently $C_{1-4}$ haloalkyl, said $C_{1-4}$ haloalkyl is trifluoromethyl;
and/or, when $R^3$ is independently $C_{3-6}$ cycloalkyl, said $C_{3-6}$ cycloalkyl is cyclopropyl;
and/or, when one of $R^5$ and $R^6$ is independently $C_{1-4}$ alkyl, said $C_{1-4}$ alkyl is methyl;
and/or, when $R^7$ and $R^8$ are independently halogen, said halogen is fluorine or chlorine;
and/or, when $R^{10}$ is phenyl-$C_{1-5}$ alkanediyl-COOH or -$C_{1-5}$ alkanediyl-COOH substituted by one or more $R^{12}$, the $C_{1-5}$ alkanediyl of said phenyl-$C_{1-5}$ alkanediyl-COOH and -$C_{1-5}$ alkanediyl-COOH substituted by one or more $R^{12}$ is $-CH_2CH_2-$, $-CH_2C(CH_3)_2-$, or - $CH_2C(CH_3)_2-CH_2-$;
and/or, when $R^{10}$ is $C_{1-4}$ alkyl, said $C_{1-4}$ alkyl is tert-butyl;
and/or, when $R^{11}$ is independently halogen, said halogen is fluorine or chlorine;
and/or, when $R^{11}$ is $C_{1-4}$ alkyl, said $C_{1-4}$ alkyl is methyl, ethyl, n-propyl, isopropyl, n-butyl, isobutyl, sec-butyl, or tert-butyl;
and/or, when any two $R^{11}$ are joined together with the carbon to which they are attached to form $C_{3-6}$ cycloalkyl, said $C_{3-6}$ cycloalkyl is cyclopropyl;
and/or, when $R^{12}$ is independently halogen, said halogen is fluorine or chlorine;
and/or, when $R^{12}$ is $C_{1-4}$ alkyl, said $C_{1-4}$ alkyl is methyl;

and/or, when $R^5$ is independently -COOH, $R^6$ is independently H or methyl; or $R^5$ is independently H $R^6$ is independently-$(CH_2)_{n1}COOH$, $-CH=CH-(CH_2)_{n2}COOH$,

, 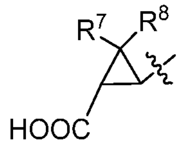 ;

and/or,

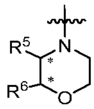 is 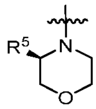 or 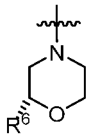 ;

and/or, M is CH;
and/or, m is 0, 1, or 2;
and/or, n is 1 or 2;
and/or, n1 is 1 or 2;
and/or, n2 is 0;
and/or, n3 is 2;
and/or,

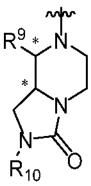 is 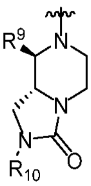 or 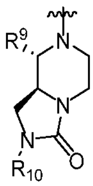 .

**4.** The phenyldihydropyrimidine compound as shown in Formula I, a tautomer or a pharmaceutically acceptable salt thereof according to claim 1, **characterized in that**,

$R^1$ is F or methyl;
and/or, W is O;
and/or, X is H;
and/or, $R^3$ is F, methyl, trifluoromethyl, cyclopropyl, or $-(CH_2)-OH$;
and/or, $R^5$, $R^6$ are independently H, methyl, -COOH, $-(CH_2)_2COOH$, or -CH=CH-COOH,

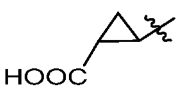 , or 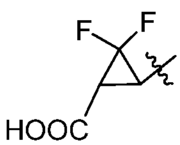 ;

and/or, $R^7$, $R^8$ are independently H or F;
and/or, when $R^{10}$ is phenyl-$C_{1-5}$ alkanediyl-COOH substituted by one or more $R^{12}$, said phenyl-$C_{1-5}$ alkanediyl-COOH substituted by one or more $R^{12}$ is

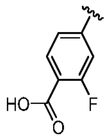 or 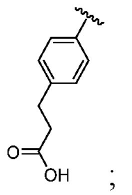 ;

and/or, when $R^{10}$ is -$C_{1-5}$ alkanediyl-COOH, said -$C_{1-5}$ alkanediyl-COOH is -$CH_2C(CH_3)_2$-COOH or -$CH_2C(CH_3)_2$-$CH_2$-COOH;
and/or, $R^{11}$ is F; or two $R^{11}$ are joined together with the carbon to which they are attached to form

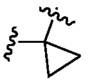 .

5. The phenyldihydropyrimidine compound as shown in Formula I, a tautomer or a pharmaceutically acceptable salt thereof according to claim 1, **characterized in that**,

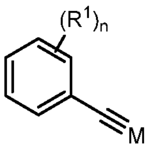 is 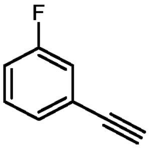, 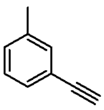, 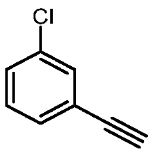, 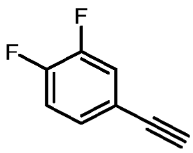, 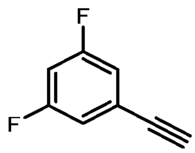,

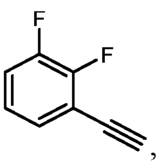, 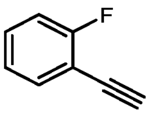, or 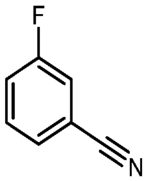;

and/or, $R^2$-W- is ethyl-O-, methyl-O-, or methyl-S-; for example ethyl-O-; and/or,

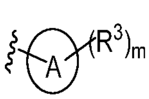 is a 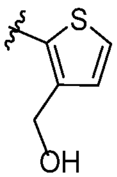, 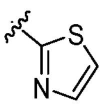, 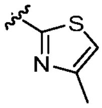, 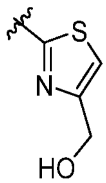, 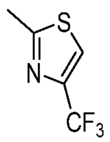, 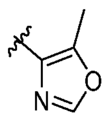,

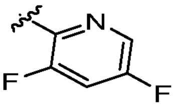; 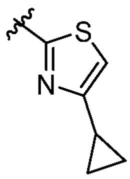 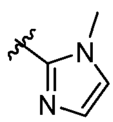 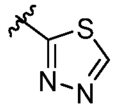 ;

for example

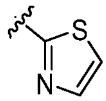;

and/or,

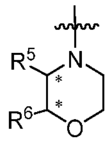 is 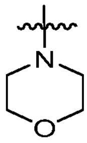, 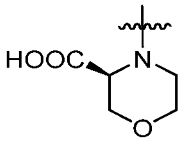, 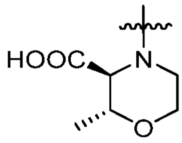, 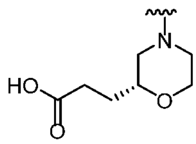,

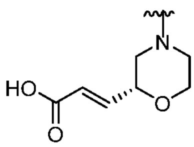, 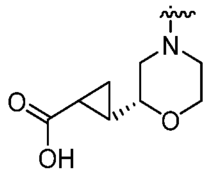, or 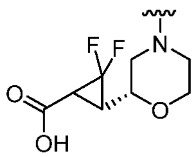;

for example

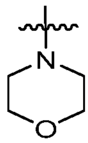, 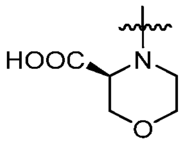,

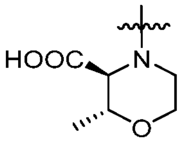, 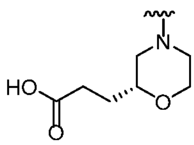, or 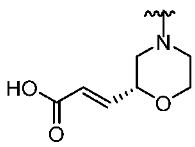;

and/or,

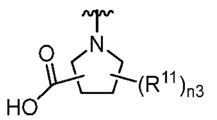 is 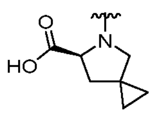 or 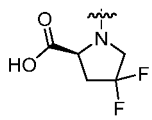;

and/or,

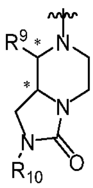 is 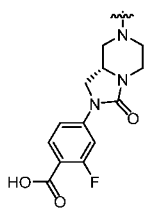, 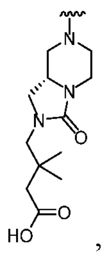, 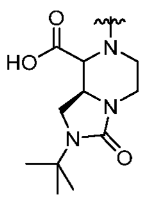, 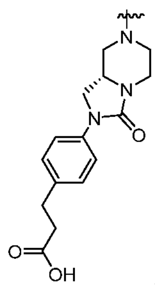, or 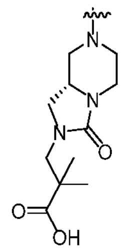.

**6.** The phenyldihydropyrimidine compound as shown in Formula I, a tautomer or a pharmaceutically acceptable salt thereof according to claim 1, **characterized in that**,
said phenyldihydropyrimidine compound as shown in Formula I is selected from the following schemes:

scheme 1,
M is CH or N;
X is H or D;
$R^1$ is independently halogen;
n is 1;
$R^2$ is ethyl or methyl;
W is O or S;

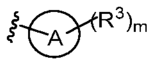 is 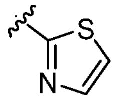;

Ris

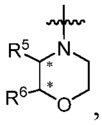, 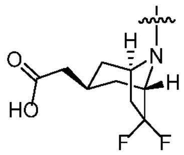, or 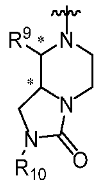;

$R^5$ is independently H, and $R^6$ is independently $-CH=CH-(CH_2)_{n2}COOH$;
$R^9$ is H, $R^{10}$ is $-C_{1-5}$ alkanediyl-COOH;
a carbon atom with "*" indicates that when it is a chiral carbon atom, it is in S configuration, R configuration or a mixture thereof;
scheme 2,
X is H;
$R^1$ is independently F;
n is 1;
$R^2$ is ethyl;
WisO;

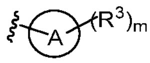 is 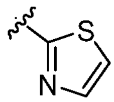;

Ris

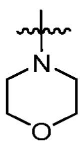, 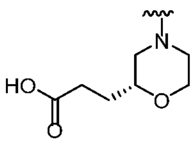, 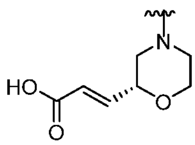, or 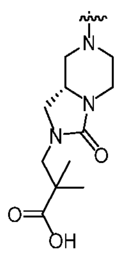;

a carbon atom with "*" indicates that when it is a chiral carbon atom, it is in S configuration, R configuration or a mixture thereof.

7. The phenyldihydropyrimidine compound as shown in Formula I, a tautomer or a pharmaceutically acceptable salt thereof according to claim 1, **characterized in that**,
said phenyldihydropyrimidine compound as shown in Formula I is selected from the following schemes:

scheme 1,
M is CH or N;
X is H or D;
$R^1$ is independently halogen;
n is 1;
$R^2$ is ethyl or methyl;
W is O or S;

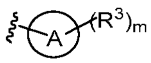 is 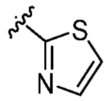 ;

Ris

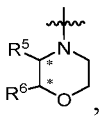 , 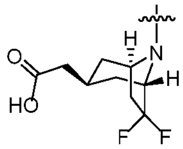 , or 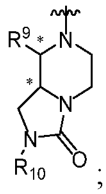 ;

$R^5$ is independently H, and $R^6$ is independently $-CH=CH-(CH_2)_{n2}COOH$;
n2 is 0 or 1;
$R^9$ is H, $R^{10}$ is $-C_{1-5}$ alkanediyl-COOH;
a carbon atom with "*" indicates that when it is a chiral carbon atom, it is in S configuration, R configuration or a mixture thereof.
scheme 2,
M is CH;
XisH;
$R^1$ is independently F;
n is 1;
$R^2$ is ethyl;
WisO;

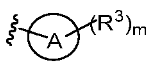 is 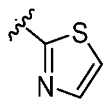 ;

Ris

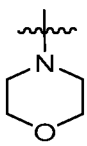 , 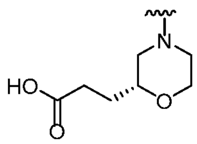 , 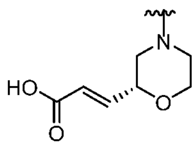 , or 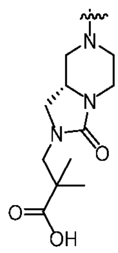 ;

a carbon atom with "*" indicates that when it is a chiral carbon atom, it is in S configuration, R configuration or a mixture thereof.

**8.** The phenyldihydropyrimidine compound as shown in Formula I, a tautomer or a pharmaceutically acceptable salt thereof according to claim 1, **characterized in that**,

said phenyldihydropyrimidine compound as shown in Formula I is selected from the following scheme:

M is CH;
XisH;
$R^1$ is independently F;
n is 1;
$R^2$ is ethyl;
WisO;

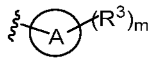 is 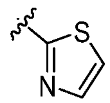 ;

Ris

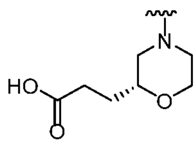 , 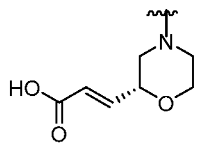 , or 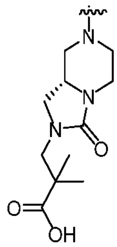 ;

a carbon atom with "*" indicates that when it is a chiral carbon atom, it is in S configuration, R configuration or a mixture thereof.

**9.** The phenyldihydropyrimidine compound as shown in Formula I, a tautomer or a pharmaceutically acceptable salt thereof according to claim 1, **characterized in that**, said phenyldihydropyrimidine compound as shown in Formula I is selected from the following structures:

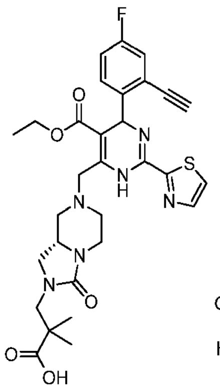

**SZ-023089RAC**

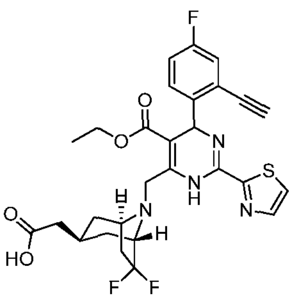

**SZ-023091RAC**

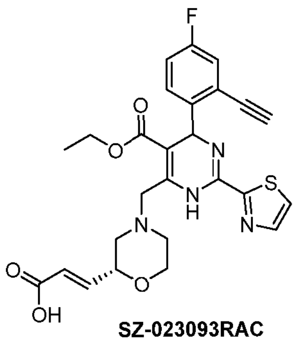

**SZ-023093RAC**

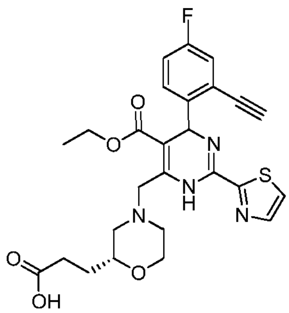

**SZ-023095RAC**

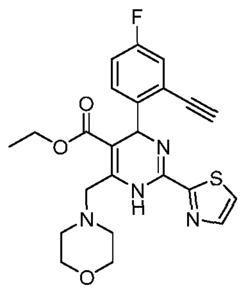
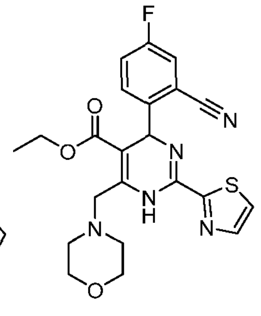
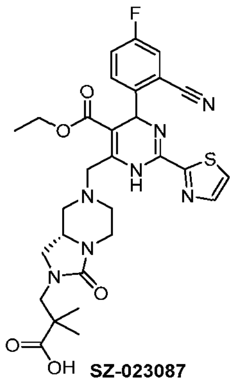
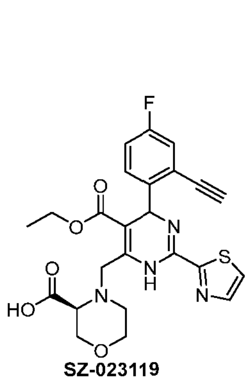

SZ-023115RAC SZ-023117RAC SZ-023087 SZ-023119

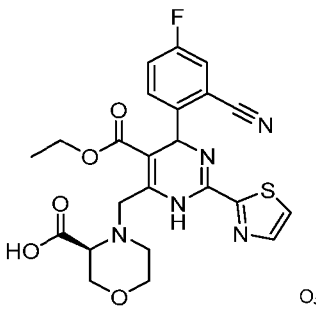

SZ-023121

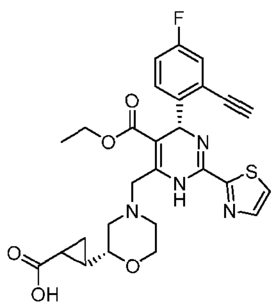
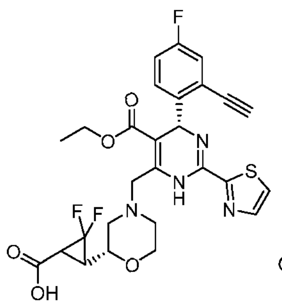
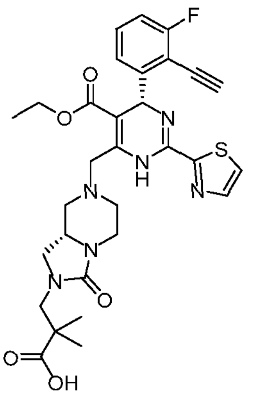

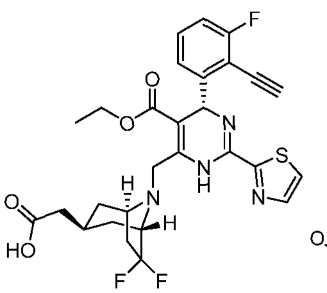
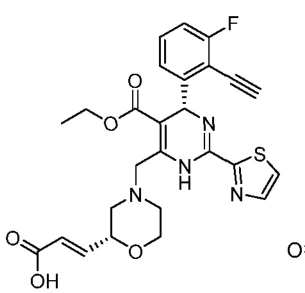
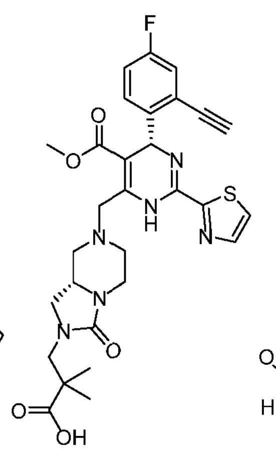
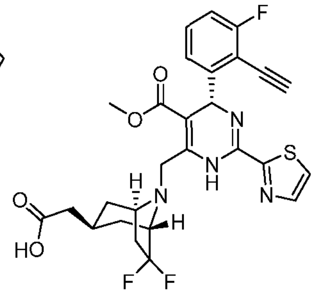

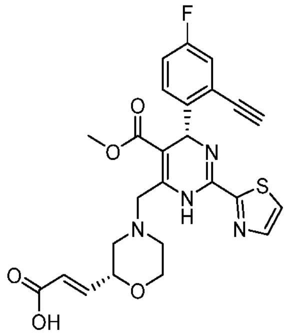
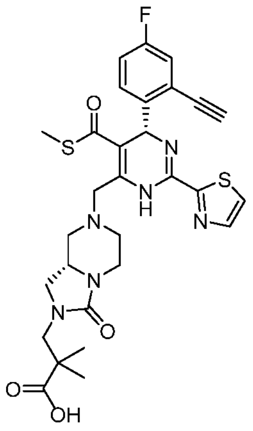
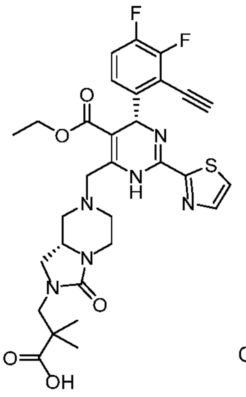
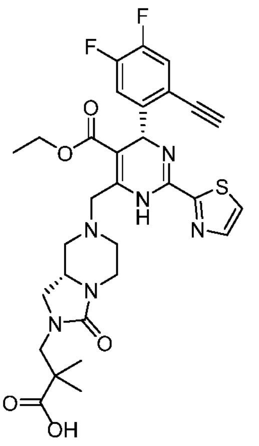

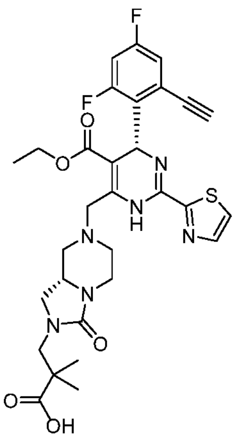
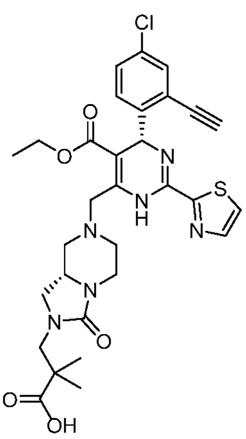
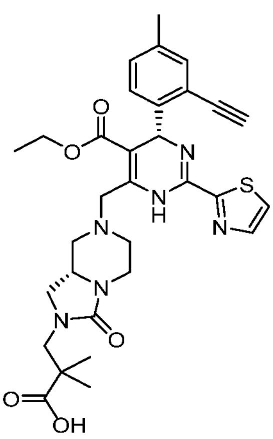
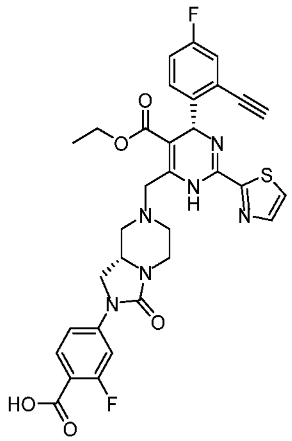

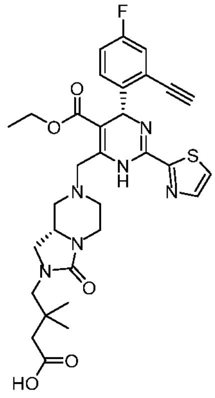
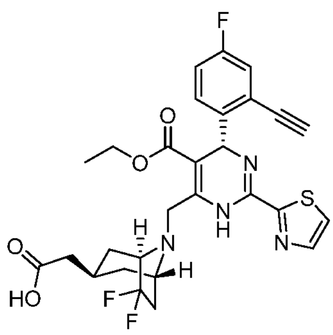
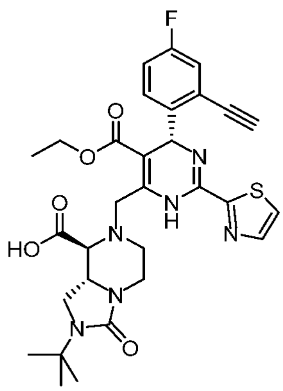
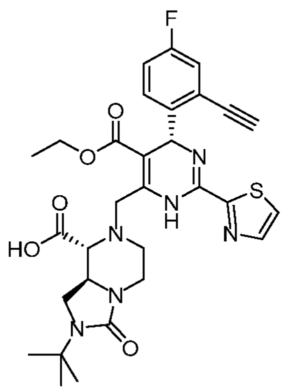

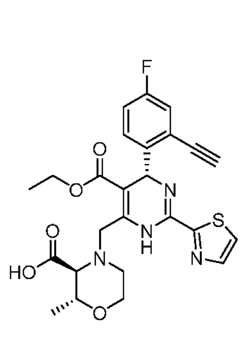
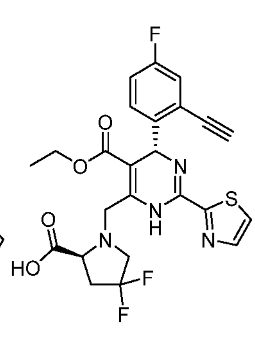
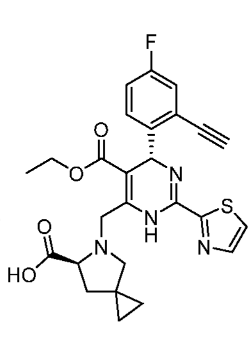
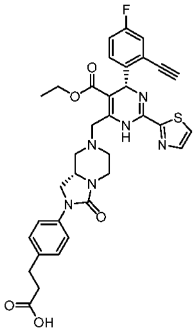

.

**10.** The phenyldihydropyrimidine compound as shown in Formula I, a tautomer or a pharmaceutically acceptable salt

thereof according to claim 1, **characterized in that**,
said phenyldihydropyrimidine compound as shown in Formula I is selected from the following structures:

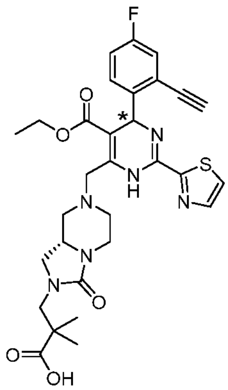
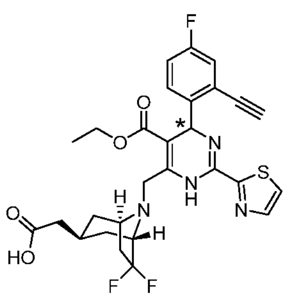
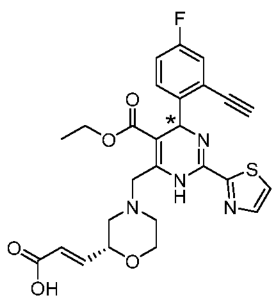
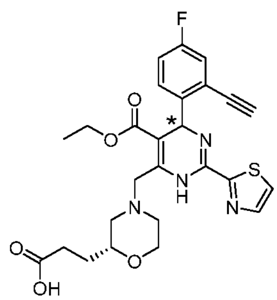

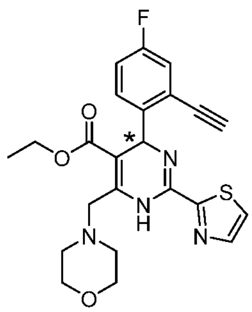
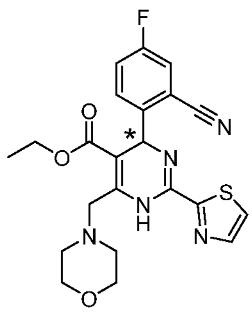
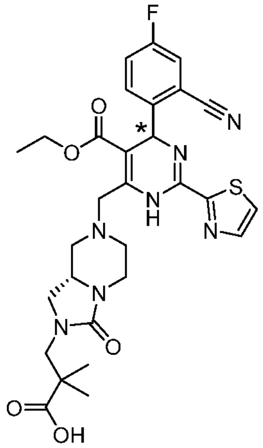
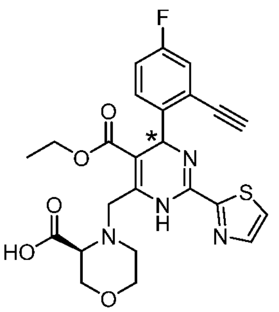

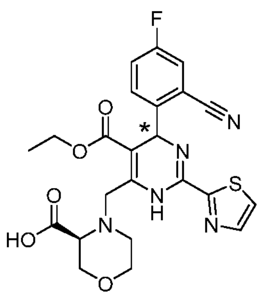
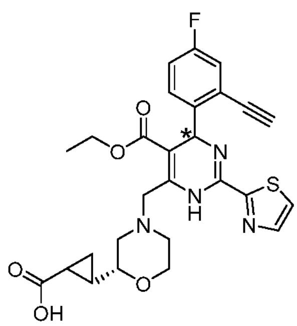
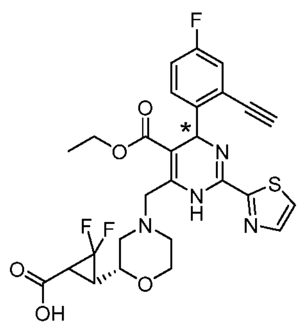
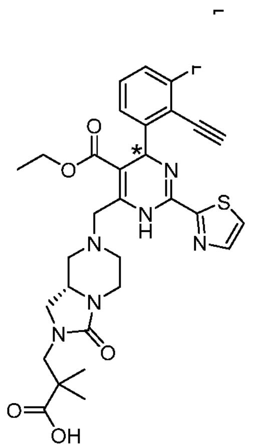

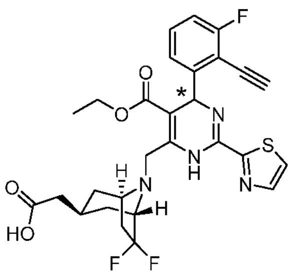
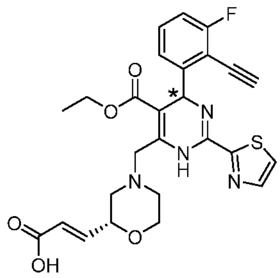
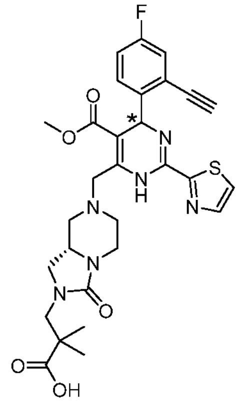
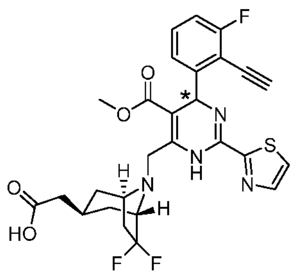

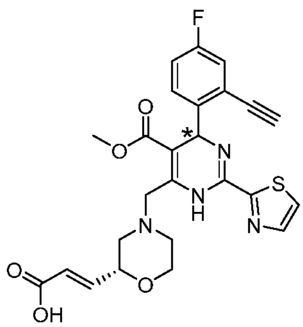
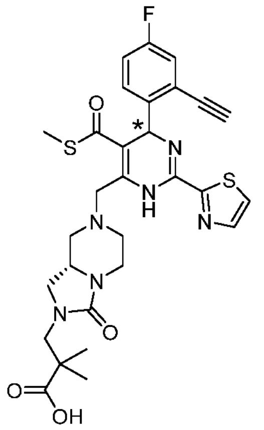
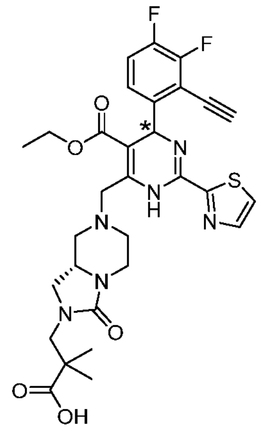
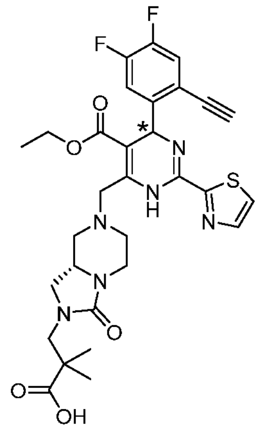

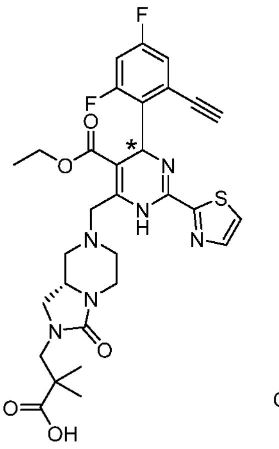
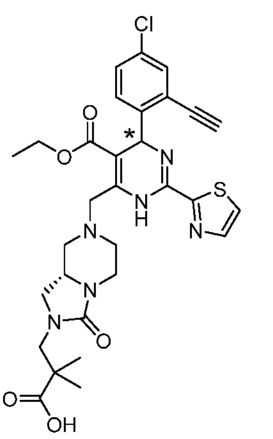
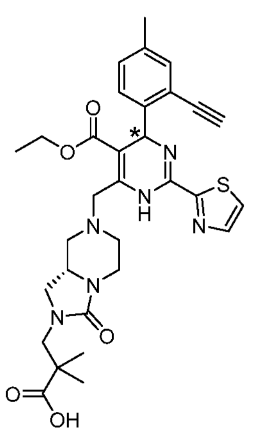
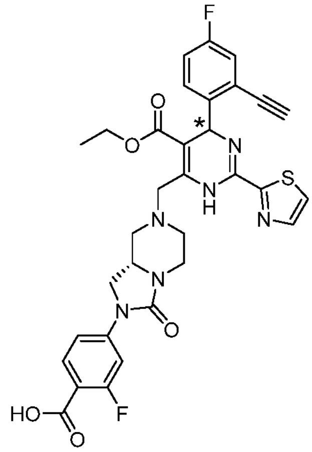

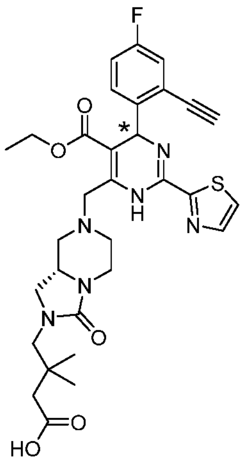
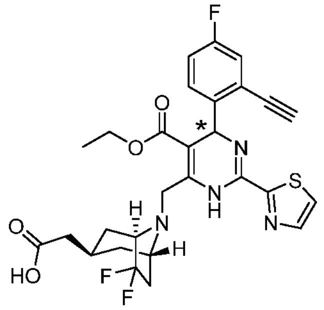
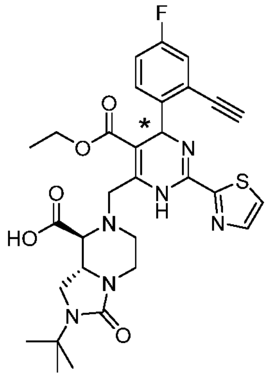
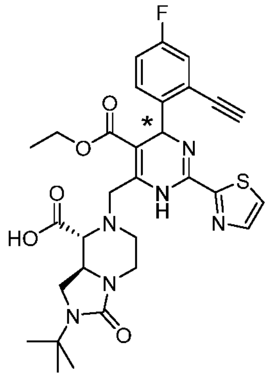

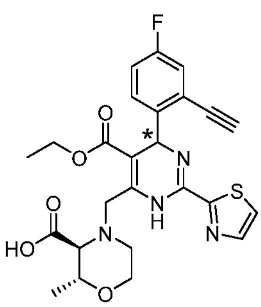
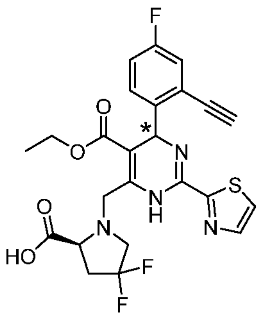
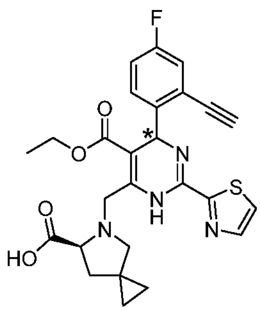
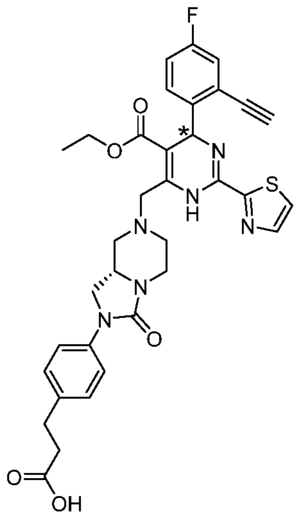

a carbon atom with "*" indicates that when it is a chiral carbon atom, it is in S configuration, R configuration or a mixture thereof.

**11.** The phenyldihydropyrimidine compound as shown in Formula I, a tautomer or a pharmaceutically acceptable salt thereof according to claim 1, **characterized in that**,
said phenyldihydropyrimidine compound as shown in Formula I is selected from the following structures:

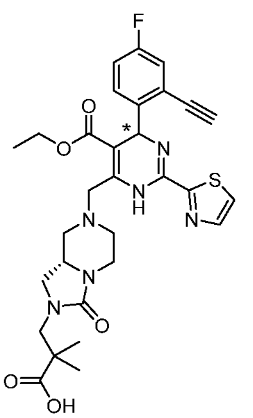
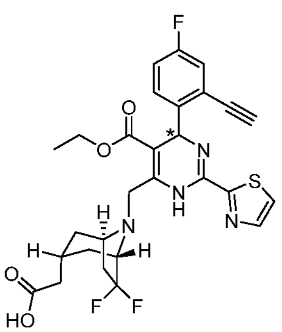
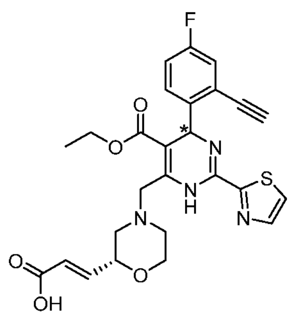
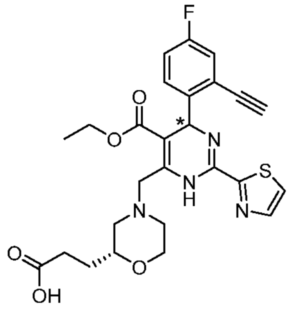

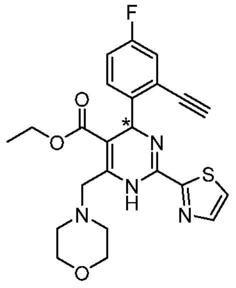
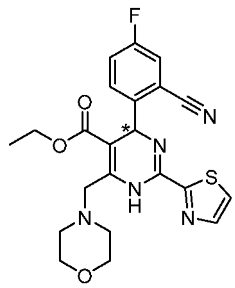
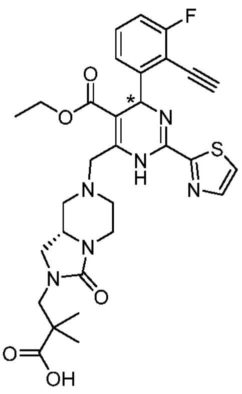
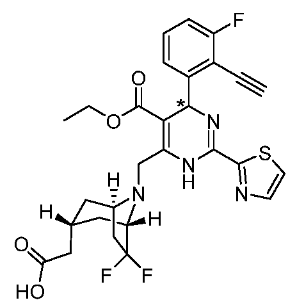

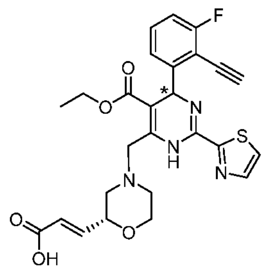
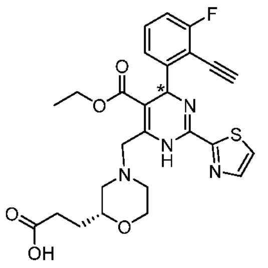
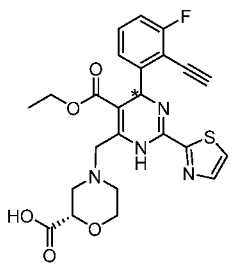
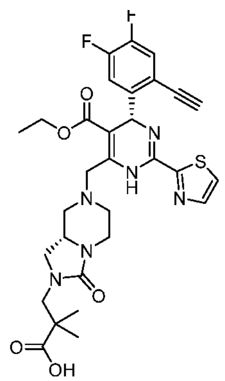

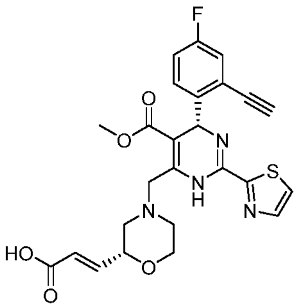
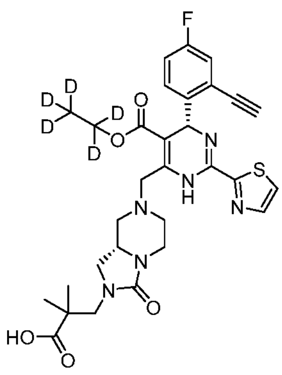
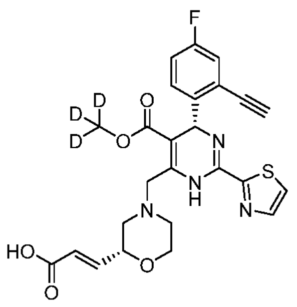
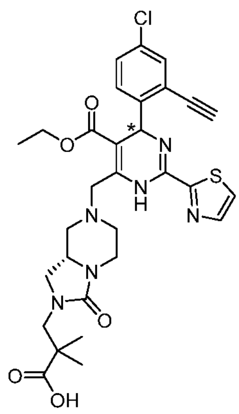

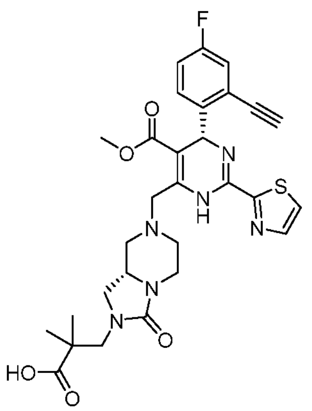
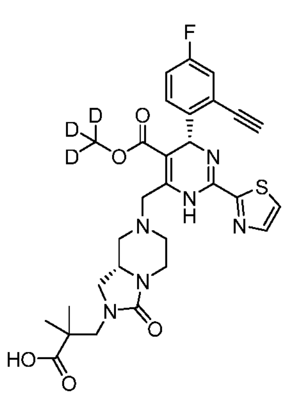
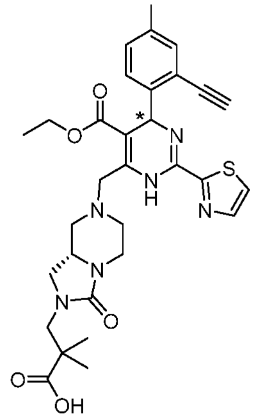
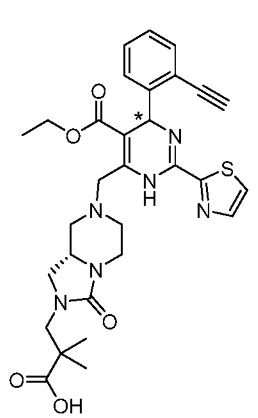

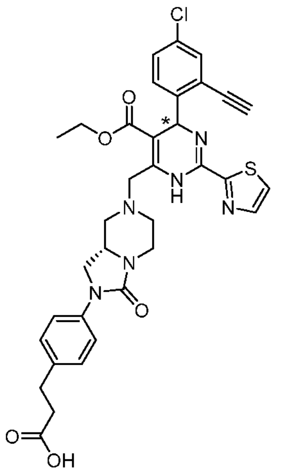
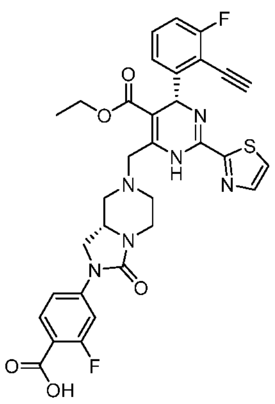
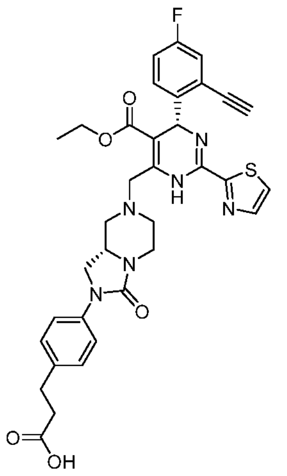
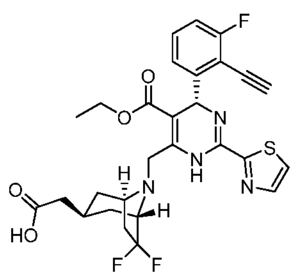

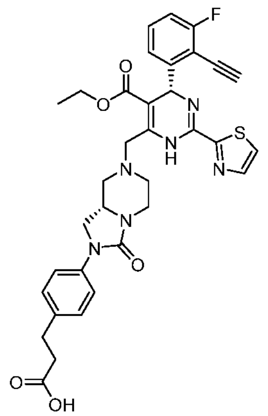
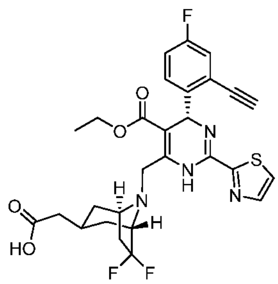
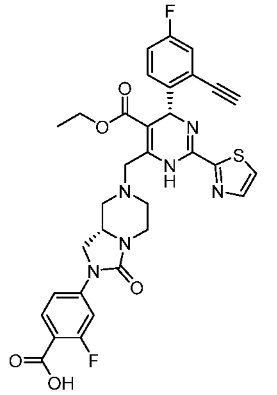
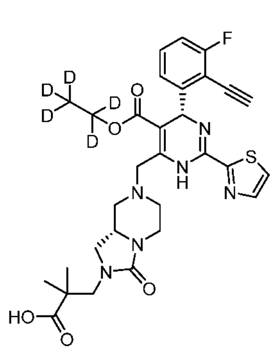

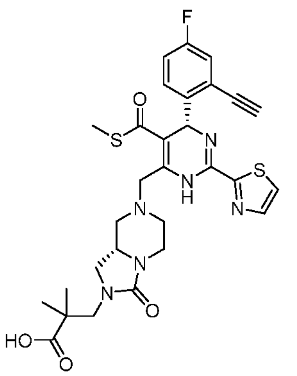
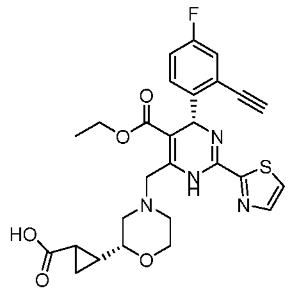
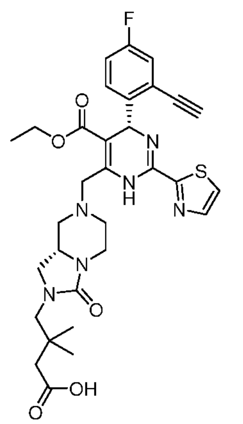
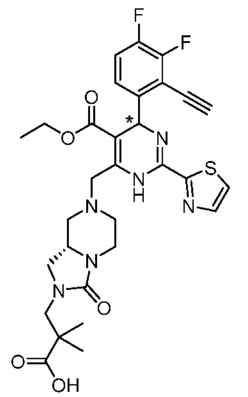

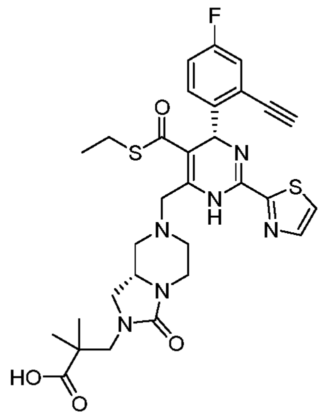

a carbon atom with "*" indicates that when it is a chiral carbon atom, it is in S configuration, R configuration or a mixture thereof.

**12.** The phenyldihydropyrimidine compound as shown in Formula I, a tautomer or a pharmaceutically acceptable salt thereof according to claim 1, **characterized in that**, said phenyldihydropyrimidine compound as shown in Formula I is selected from the following structures:

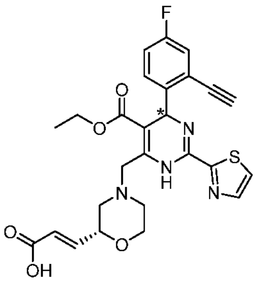
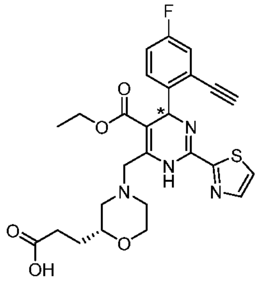
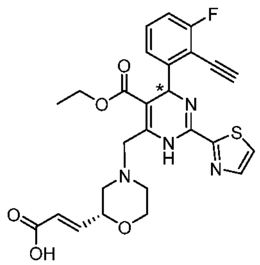
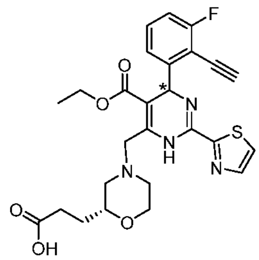

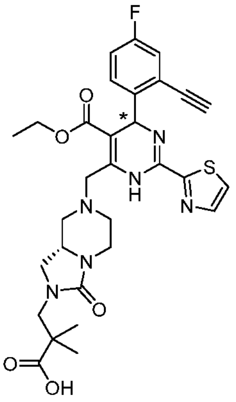
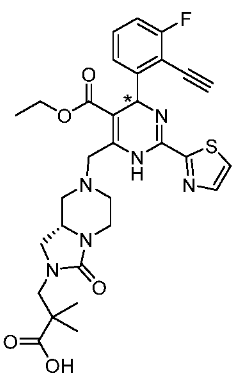
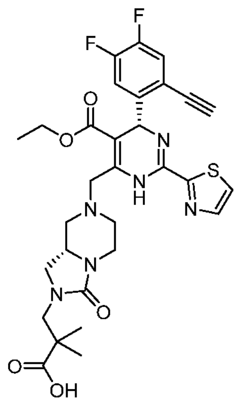
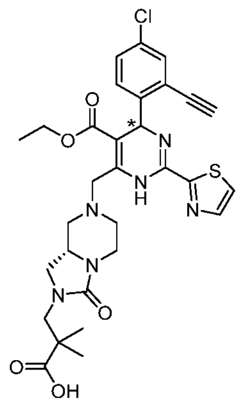

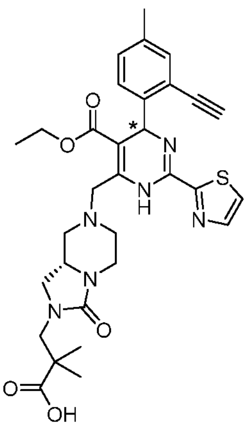 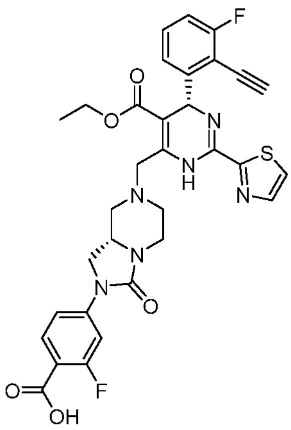 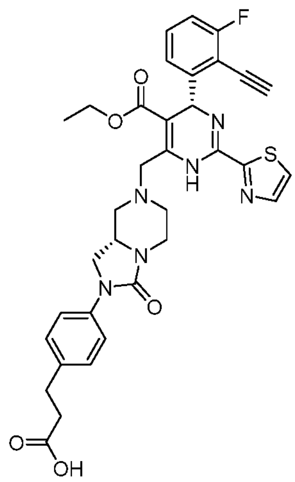

a carbon atom with "*" indicates that when it is a chiral carbon atom, it is in S configuration, R configuration or a mixture thereof.

**13.** A pharmaceutical composition comprising a phenyldihydropyrimidine compound as shown in Formula I, a tautomer or a pharmaceutically acceptable salt thereof according to any of claims 1-12, and a pharmaceutical excipient.

**14.** Use of a phenyldihydropyrimidine compound as shown in Formula I, a tautomer or a pharmaceutically acceptable salt thereof according to any of claims 1-12 in preparation of an HBV inhibitor.

**15.** Use of a phenyldihydropyrimidine compound as shown in Formula I, a tautomer or a pharmaceutically acceptable salt thereof according to any of claims 1-12 in preparation of a medicament, the medicament is a medicament for prevention and/or treatment of HBV infection.

**16.** A method of preventing and/or treating HBV infection comprising administering to a patient a therapeutically effective amount of a phenyldihydropyrimidine compound as shown in Formula I, a tautomer or a pharmaceutically acceptable salt thereof according to any of claims 1-12.

**17.** A phenyldihydropyrimidine compound as shown in Formula I, a tautomer or a pharmaceutically acceptable salt thereof according to any of claims 1-12 for use in prevention and/or treatment of HBV infection.

**INTERNATIONAL SEARCH REPORT**

International application No. **PCT/CN2022/075185**

**A. CLASSIFICATION OF SUBJECT MATTER**

C07D 417/14(2006.01)i; C07D 413/14(2006.01)i; C07D 403/14(2006.01)i; C07D 487/04(2006.01)i; A61K 31/5377(2006.01)i; A61K 31/506(2006.01)i; A61K 31/4985(2006.01)i; A61K 31/4025(2006.01)i; A61P 31/20(2006.01)i; A61P 31/12(2006.01)i; A61P 1/16(2006.01)i

According to International Patent Classification (IPC) or to both national classification and IPC

**B. FIELDS SEARCHED**

Minimum documentation searched (classification system followed by classification symbols)

C07D417/-; C07D413/-; C07D403/-; C07D487/-; A61K31/-; A61P31/-; A61P1/-

Documentation searched other than minimum documentation to the extent that such documents are included in the fields searched

Electronic data base consulted during the international search (name of data base and, where practicable, search terms used)

WPI, EPODOC, CNPAT, CNKI, STN(caplus, registry): 二氢嘧啶, 乙肝, 乙型肝炎, 抑制剂, dihydropyrimidine, 1,4-dihydropyrimidin-6-yl, HBV, hepatitis B, inhibitor, 结构检索, structure search

**C. DOCUMENTS CONSIDERED TO BE RELEVANT**

| Category* | Citation of document, with indication, where appropriate, of the relevant passages | Relevant to claim No. |
|---|---|---|
| X | CN 103626752 A (GUANGDONG HEC PHARMACEUTICAL CO., LTD.) 12 March 2014 (2014-03-12) description, paragraphs [0006]-[1888] | 1-17 |
| Y | CN 103626752 A (GUANGDONG HEC PHARMACEUTICAL CO., LTD.) 12 March 2014 (2014-03-12) description, paragraphs [0006]-[1888] | 1-17 |
| X | CN 104603125 A (HOFFMANN LA ROCHE) 06 May 2015 (2015-05-06) description, paragraphs [0010]-[1003] | 1-17 |
| Y | CN 104603125 A (HOFFMANN LA ROCHE) 06 May 2015 (2015-05-06) description, paragraphs [0010]-[1003] | 1-17 |
| Y | CN 105209470 A (F. HOFFMANN-LA ROCHE AG) 30 December 2015 (2015-12-30) description, paragraphs [0010]-[1859] | 1-17 |
| Y | CN 106061978 A (F. HOFFMANN-LA ROCHE AG) 26 October 2016 (2016-10-26) description, paragraphs [0014]-[1812] | 1-17 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 April 2022** | **28 April 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2022/075185**

**C. DOCUMENTS CONSIDERED TO BE RELEVANT**

| Category* | Citation of document, with indication, where appropriate, of the relevant passages | Relevant to claim No. |
|---|---|---|
| Y | WO 2017064156 A1 (F. HOFFMANN-LA ROCHE AG. et al.) 20 April 2017 (2017-04-20) description, p. 1, line 6 to p. 42, line 6 | 1-17 |

Form PCT/ISA/210 (second sheet) (January 2015)

**INTERNATIONAL SEARCH REPORT**

International application No.
**PCT/CN2022/075185**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **16**
because they relate to subject matter not required to be searched by this Authority, namely:

[1] Claim 16 sets forth a method for preventing and/or treating HBV infections. Hence, claim 16 relates to a method for treating a human or animal body, and therefore, said claim does not comply with PCT Rule 39.1(iv). Nevertheless, a search is still performed on the basis of the claimed effect of the compound.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/075185**

| Patent document cited in search report | Publication date (day/month/year) | Patent family member(s) | Publication date (day/month/year) |
|---|---|---|---|
| CN 103626752 A | 12 March 2014 | TW 201408662 A | 01 March 2014 |
| | | WO 2014029193 A1 | 27 February 2014 |
| | | US 2015152096 A1 | 04 June 2015 |
| | | DK 2888241 T3 | 23 October 2017 |
| | | PL 2888241 T3 | 29 December 2017 |
| | | AU 2013305390 A1 | 15 January 2015 |
| | | ZA 201500451 B | 26 October 2016 |
| | | CA 2876690 A1 | 27 February 2014 |
| | | EP 2888241 A1 | 01 July 2015 |
| | | BR 112015002858 A2 | 08 August 2017 |
| | | ES 2640049 T3 | 31 October 2017 |
| | | HK 1206027 A1 | 31 December 2015 |
| | | PT 2888241 T | 14 September 2017 |
| | | HU E034919 T2 | 28 March 2018 |
| | | KR 20150044859 A | 27 April 2015 |
| | | JP 2015526448 A | 10 September 2015 |
| | | MX 2015002511 A | 08 March 2016 |
| | | CN 104926808 A | 23 September 2015 |
| | | SG 11201408791 T A | 29 January 2015 |
| | | RU 2015110082 A | 10 October 2016 |
| CN 104603125 A | 06 May 2015 | ES 2617906 T3 | 20 June 2017 |
| | | AU 2013311705 A1 | 05 February 2015 |
| | | US 2015031687 A1 | 29 January 2015 |
| | | CO 7170160 A2 | 28 January 2015 |
| | | PE 20150776 A1 | 21 May 2015 |
| | | PH 12015500347 A1 | 20 April 2015 |
| | | EA 201590384 A1 | 30 October 2015 |
| | | CA 2881322 A1 | 13 March 2014 |
| | | TW 201416360 A | 01 May 2014 |
| | | CL 2015000482 A1 | 10 July 2015 |
| | | ZA 201500580 B | 27 September 2017 |
| | | MA 37942 A2 | 30 December 2016 |
| | | HK 1210152 A1 | 15 April 2016 |
| | | WO 2014037480 A1 | 13 March 2014 |
| | | SG CN 11201500377 U A | 27 February 2015 |
| | | BR 112015004113 A2 | 04 July 2017 |
| | | MX 2015002954 A | 05 June 2015 |
| | | IL 236691 D0 | 26 February 2015 |
| | | AR 092503 A1 | 22 April 2015 |
| | | JP 2015527382 A | 17 September 2015 |
| | | CR 20150085 A | 06 April 2015 |
| | | KR 20150054795 A | 20 May 2015 |
| | | EP 2892893 A1 | 15 July 2015 |
| CN 105209470 A | 30 December 2015 | TW 201512193 A | 01 April 2015 |
| | | EA 201592126 A1 | 31 May 2016 |
| | | EP 2997032 A1 | 23 March 2016 |
| | | SG 11201509463 Y A | 30 December 2015 |
| | | BR 112015028873 A2 | 25 July 2017 |
| | | CA 2911214 A1 | 20 November 2014 |

Form PCT/ISA/210 (patent family annex) (January 2015)

**INTERNATIONAL SEARCH REPORT**
**Information on patent family members**

International application No.
**PCT/CN2022/075185**

| Patent document cited in search report | Publication date (day/month/year) | Patent family member(s) | Publication date (day/month/year) |
|---|---|---|---|
| | | US 2016122363 A1 | 05 May 2016 |
| | | CR 11 June 2015 A | 07 December 2015 |
| | | AU 2014267198 A1 | 05 November 2015 |
| | | JP 2016518432 A | 23 June 2016 |
| | | HK 1219480 A1 | 07 April 2017 |
| | | MX 2015015692 A | 04 March 2016 |
| | | PH 12015502443 A1 | 22 February 2016 |
| | | CL 2015003298 A1 | 17 June 2016 |
| | | PE 20151951 A1 | 26 December 2015 |
| | | ZA 201507840 B | 29 November 2017 |
| | | US 2014343032 A1 | 20 November 2014 |
| | | WO 2014184328 A1 | 20 November 2014 |
| CN 106061978 A | 26 October 2016 | US 2016083383 A1 | 24 March 2016 |
| | | TW 201726682 A | 01 August 2017 |
| | | EP 3114128 A1 | 11 January 2017 |
| | | PL 3114128 T3 | 28 June 2019 |
| | | DK 3114128 T3 | 25 March 2019 |
| | | HU E041734 T2 | 28 May 2019 |
| | | KR 20160105978 A | 08 September 2016 |
| | | RS 58384 B1 | 30 April 2019 |
| | | MX 2016011515 A | 20 December 2016 |
| | | AU 2015226206 A1 | 07 July 2016 |
| | | KR 20180130016 A | 05 December 2018 |
| | | US 2020062753 A1 | 27 February 2020 |
| | | IL 246449 D0 | 31 August 2016 |
| | | CA 2935811 A1 | 11 September 2015 |
| | | PE 20161338 A1 | 12 December 2016 |
| | | PT 3114128 T | 27 February 2019 |
| | | EA 201691726 A1 | 30 December 2016 |
| | | CN 107513073 A | 26 December 2017 |
| | | LT 3114128 T | 25 March 2019 |
| | | HR P20190352 T1 | 05 April 2019 |
| | | UA 117518 C2 | 10 August 2018 |
| | | US 2015252057 A1 | 10 September 2015 |
| | | US 2018370969 A1 | 27 December 2018 |
| | | SG CN 11201607332 U A | 28 October 2016 |
| | | WO 2015132276 A1 | 11 September 2015 |
| | | JP 2017507186 A | 16 March 2017 |
| | | TW 201546075 A | 16 December 2015 |
| | | MA 39721 A | 11 January 2017 |
| | | SI 3114128 T1 | 30 April 2019 |
| | | CL 2016002179 A1 | 17 February 2017 |
| | | HK 1245784 A1 | 31 August 2018 |
| | | CR 20160337 A | 20 September 2016 |
| | | ES 2714110 T3 | 27 May 2019 |
| | | PH 12016501631 A1 | 06 February 2017 |
| | | CN 109232613 A | 18 January 2019 |
| WO 2017064156 A1 | 20 April 2017 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202110164859
- CN 202110360500
- CN 202110500219
- CN 202110697314
- CN 108718527 A
- CN 105209470 B
- CN 109790145 B
- CN 104945395 B
- CN 102066369 B
- CN 109678859
- CN 111825676


### Non-patent literature cited in the description


- *Inactive Ingredient Guide,* 1996
- **ASH ; ASH.** Hand book of Pharmaceutical Additives. Synapse Information Resources, Inc, 2002
- Handbook of Chemistry and Physics. 1994
- **THOMAS SORRELL.** Organic Chemistry. University Science Books, 1999
- **MICHAEL B. SMITH ; JERRY MARCH.** March's Advanced Organic Chemistry. John Wiley & Sons, 2007